(19) Europäisches Patentamt
European Patent Office
Office européen des brevets

(11) EP 4 327 822 A1

(12) # EUROPEAN PATENT APPLICATION
published in accordance with Art. 153(4) EPC

(43) Date of publication:
**28.02.2024 Bulletin 2024/09**

(21) Application number: **22791112.0**

(22) Date of filing: **21.04.2022**

(51) International Patent Classification (IPC):
***A61K 39/395** (2006.01)* ***A61K 31/00** (2006.01)*
***A61P 35/00** (2006.01)*

(52) Cooperative Patent Classification (CPC):
**A61K 31/00; A61K 31/337; A61K 31/519; A61K 31/555; A61K 33/243; A61K 39/395; A61K 45/06; A61P 35/00**

(86) International application number:
**PCT/CN2022/088257**

(87) International publication number:
**WO 2022/223006 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.04.2021 CN 202110436365**

(71) Applicant: **Shanghai Junshi Biosciences Co., Ltd.**
**Pilot Free Trade Zone**
**Shanghai**
**201210 (CN)**

(72) Inventors:
- **YAO, Sheng**
  **Shanghai 201210 (CN)**
- **FENG, Hui**
  **Shanghai 201210 (CN)**

(74) Representative: **V.O.**
**P.O. Box 87930**
**2508 DH Den Haag (NL)**

Remarks:
The complete document including Reference Table(s) and the Sequence Listing(s) can be downloaded from the EPO website

(54) **USE OF ANTI-PD-1 ANTIBODY IN COMBINATION WITH FIRST-LINE CHEMOTHERAPY FOR TREATING ADVANCED NON-SMALL CELL LUNG CANCER**

(57) The present invention relates to use of an anti-PD-1 antibody in combination with first-line chemotherapy in the treatment of advanced non-small cell lung cancer. Specifically, the present invention relates to use of a combination of an anti-PD-1 antibody or an antigen-binding fragment thereof and a first-line chemotherapeutic agent in the preparation of a drug for treating non-small cell lung cancer (NSCLC). The present invention also relates to a related drug combination and a kit.

EP 4 327 822 A1

## Description

### TECHNICAL FIELD

The present invention relates to a combination of an anti-PD-1 antibody or an antigen-binding fragment thereof and standard first-line chemotherapy, and use thereof in the preparation of a drug for treating advanced non-small cell lung cancer (NSCLC). Specifically, the present invention relates to a combination of an anti-PD-1 antibody or an antigen-binding fragment thereof, albumin-bound paclitaxel, and carboplatin, or a combination of an anti-PD-1 antibody or an antigen-binding fragment thereof, pemetrexed, and cisplatin/carboplatin, and use thereof in the preparation of a drug for treating advanced NSCLC.

### BACKGROUND

Non-small cell lung cancer (NSCLC) is the main subtype of lung cancer, accounting for about 85% of all cases. NSCLC is divided into two main histological types: adenocarcinoma and squamous cell carcinoma. The adenocarcinoma histological type accounts for more than half of all NSCLCs, while the squamous cell carcinoma histological type accounts for about 25%. Other NSCLC types include large cell carcinoma, neuroendocrine neoplasm, sarcomatoid carcinoma, and poorly differentiated carcinoma.

Genetic changes that have a significant prognostic and/or predictive effect in NSCLCs include epidermal growth factor receptor (EGFR) mutations, anaplastic lymphoma kinase (ALK) gene rearrangements, and GTPase-Kras (KRAS) gene mutations. The incidence rates of these mutations differ in squamous cell carcinoma and adenocarcinoma. For example, EGFR kinase domain mutations are reported in 10%-40% of NSCLC adenocarcinoma patients, but rarely in squamous cell NSCLC patients. Likewise, ALK fusion oncogenes (identified as promoters of lung tumorigenesis) are observed in about 7% of adenocarcinoma patients, but are quite rare in squamous histology. In addition, KRAS mutations are quite rare in squamous cell NSCLC, but can be observed in about 30% of NSCLC adenocarcinoma cases.

Programmed death receptor 1 (PD-1) plays an important role in immune regulation and maintenance of peripheral tolerance. PD-1 is mainly expressed in activated T and B cells and functions to inhibit the activation of lymphocytes, which is a normal peripheral tissue tolerance mechanism of the immune system that prevents over-reactive immunity. However, the activated T cells infiltrated in the tumor microenvironment highly express PD-1 molecules, and inflammatory factors secreted by the activated leukocytes can induce the tumor cells to highly express ligands PD-L1 and PD-L2 of PD-1, resulting in the continuous activation of the PD-1 pathway of the activated T cells in the tumor microenvironment, and the suppression of T cell function to kill tumor cells. Therapeutic PD-1 antibodies can block this pathway, partially restore the function of T cells, and enable the activated T cells to continuously kill tumor cells.

Currently, immunotherapy has revolutionized the treatment of patients with advanced non-small cell lung cancer. Immune checkpoint inhibitors (ICIs) directed to the PD-1/PD-L1 pathway, such as pembrolizumab, were approved by the U.S. Food and Drug Administration (FDA) in August 2018 based on the confirmatory results of KEYNOTE-189, for use in combination with chemotherapy for first-line treatment of metastatic non-squamous NSCLC patients without EGFR or ALK gene mutations. This study demonstrates that immunotherapy in combination with chemotherapy can be an effective first-line treatment option for advanced NSCLC patients. For advanced NSCLC patients, there is still a need for better replacement strategies.

### SUMMARY

The present invention provides use of a combination of an anti-PD-1 antibody or an antigen-binding fragment thereof and a first-line chemotherapeutic agent in the preparation of a drug or a kit for treating non-small cell lung cancer (NSCLC).

In one or more embodiments, the non-small cell lung cancer described herein is advanced non-small cell lung cancer.

In one or more embodiments, the non-small cell lung cancer described herein is untreated advanced non-small cell lung cancer.

In one or more embodiments, the non-small cell lung cancer described herein is advanced non-small cell lung cancer not accompanied by EGFR-sensitive mutations and ALK fusions.

In one or more embodiments, the non-small cell lung cancer described herein is non-small cell lung cancer with a tumor mutation burden of $\geq$ 10 mutations/Mbp.

In one or more embodiments, the non-small cell lung cancer described herein is non-small cell lung cancer accompanied by gene mutations of an FA/PI3K/Akt signaling pathway (e.g., mutations in one or more of genes COL3A1, COL6A3, FLT1, FLNC, HGF, IRS1, IRS2, ITGA4, ITGA8, and KDR), gene mutations of an IL-7 signaling pathway (e.g., mutations in one or more of genes HGF, IRS1, IRS2, and SMARCA4), or gene mutations of an SWI/SNF chromatin

remodeling complex (e.g., mutations in one or more of genes SMARCA4, SMARCA2, and PBRM1).

In one or more embodiments, the non-small cell lung cancer described herein is non-small cell lung cancer accompanied by mutations in one or more of genes RB1, KEAP1, and SMARCA4.

In one or more embodiments, the non-small cell lung cancer described herein is squamous cell carcinoma or non-squamous cell carcinoma.

In one or more embodiments, the non-small cell lung cancer described herein is non-small cell lung cancer with PD-L1 expression of ≥ 1% in immunohistochemical staining analysis of a tumor tissue section; preferably non-small cell lung cancer with PD-L1 expression of ≥ 50% in immunohistochemical staining analysis of a tumor tissue section.

In one or more embodiments, the non-small cell lung cancer described herein is non-small cell lung cancer with PD-L1 expression of < 1% in immunohistochemical staining analysis of a tumor tissue section. In one or more embodiments, the anti-PD-1 antibody or the antigen-binding fragment thereof described herein comprises a light chain complementarity determining region with amino acid sequences set forth in SEQ ID NOs: 1, 2 and 3, respectively, and a heavy chain complementarity determining region with amino acid sequences set forth in SEQ ID NOs: 4, 5 and 6, respectively.

In one or more embodiments, the anti-PD-1 antibody or the antigen-binding fragment thereof described herein comprises a light chain variable region with an amino acid sequence set forth in SEQ ID NO: 7 and a heavy chain variable region with an amino acid sequence set forth in SEQ ID NO: 8.

In one or more embodiments, the anti-PD-1 antibody described herein comprises a light chain with an amino acid sequence set forth in SEQ ID NO: 9 and a heavy chain with an amino acid sequence set forth in SEQ ID NO: 10.

In one or more embodiments, the anti-PD-1 antibody described herein is selected from one or more of nivolumab, pembrolizumab, toripalimab, sintilimab, camrelizumab, tislelizumab, and cemiplimab, preferably toripallimab.

In one or more embodiments, the first-line chemotherapeutic agent described herein is selected from one or more of carboplatin, cisplatin, pemetrexed, and albumin-bound paclitaxel.

In one or more embodiments, the chemotherapeutic agent described herein is selected from a combination of carboplatin and albumin-bound paclitaxel and a combination of pemetrexed and carboplatin or cisplatin.

In one or more embodiments, the non-small cell lung cancer described herein is squamous cell carcinoma, and the combination is a combination of the anti-PD-1 antibody or the antigen-binding fragment thereof, carboplatin, and albumin-bound paclitaxel; or the NSCLC is non-squamous cell carcinoma, and the combination is a combination of the anti-PD-1 antibody or the antigen-binding fragment thereof, pemetrexed, and carboplatin or cisplatin.

In one or more embodiments, the non-small cell lung cancer described herein is squamous cell carcinoma, and the combination is a combination of toripalimab, carboplatin, and albumin-bound paclitaxel; or the NSCLC is non-squamous cell carcinoma, and the combination is a combination of toripalimab, pemetrexed, and carboplatin or cisplatin.

In one or more embodiments, the non-small cell lung cancer described herein is squamous cell carcinoma, and in the induction stage, the combination is a combination of toripalimab, carboplatin, and albumin-bound paclitaxel.

In one or more embodiments, the non-small cell lung cancer described herein is squamous cell carcinoma, and in the maintenance stage, the combination is toripalimab administered alone.

In one or more embodiments, the non-small cell lung cancer described herein is non-squamous cell carcinoma, and in the induction stage, the combination is a combination of toripalimab, pemetrexed, and carboplatin or a combination of toripalimab, pemetrexed, and cisplatin.

In one or more embodiments, the non-small cell lung cancer described herein is non-squamous cell carcinoma, and in the maintenance stage, the combination is toripalimab and pemetrexed.

In one or more embodiments, the drug or kit is used for treating a patient with non-small cell lung cancer that is squamous cell carcinoma, which comprises one or more groups of combinations of toripalimab, carboplatin, and albumin-bound paclitaxel administered in the induction stage, and one or more groups of toripalimab administered alone in the maintenance stage. Preferably, one group of combinations is at a dose for one treatment cycle, such as three weeks.

In one or more embodiments, the drug or kit is used for treating a patient with non-small cell lung cancer that is non-squamous cell carcinoma, which comprises one or more groups of combinations of toripalimab, pemetrexed, and carboplatin administered in the induction stage, or a combination of toripalimab, pemetrexed, and cisplatin, and one or more groups of combinations of toripalimab and pemetrexed administered in the maintenance stage. Preferably, one group of combinations is at a dose for one treatment cycle, such as three weeks.

In one or more embodiments, the induction stage is 4-6 cycles, and the maintenance stage is achieved after the induction stage is over. In one or more embodiments, the maintenance stage continues until progressive disease.

In one or more embodiments, in the use described herein, the non-small cell lung cancer is squamous cell carcinoma, and the drug or kit is used for providing the anti-PD-1 antibody or the antigen-binding fragment thereof, albumin-bound paclitaxel, and carboplatin at doses described as follows:

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg

body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;

the albumin-bound paclitaxel is administered at a single dose of about 60 mg/$m^2$ body surface area to about 140 mg/$m^2$ body surface area, e.g., about 60 mg/$m^2$ body surface area, 80 mg/$m^2$ body surface area, 100 mg/$m^2$ body surface area, 120 mg/$m^2$ body surface area, or 140 mg/$m^2$ body surface area, preferably about 100 mg/$m^2$ body surface area; and

the carboplatin is administered at a single dose of about AUC 5 to AUC 7, e.g., about AUC 5, AUC 6, or AUC 7, preferably about AUC 5.

In one or more embodiments, in the use described herein, the non-small cell lung cancer is squamous cell carcinoma, and the drug or kit is used for providing the anti-PD-1 antibody or the antigen-binding fragment thereof at doses described as follows, for administration in the maintenance stage:
the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg.

In one or more embodiments, in the use described herein, the NSCLC is non-squamous cell carcinoma, and the drug or kit is used for providing the anti-PD-1 antibody or the antigen-binding fragment thereof, pemetrexed, and cisplatin at doses described as follows:

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;

the pemetrexed is administered at a single dose of about 200 mg/$m^2$ body surface area to about 800 mg/$m^2$ body surface area, e.g., about 400 mg/$m^2$ body surface area, 500 mg/$m^2$ body surface area, or 600 mg/$m^2$ body surface area, preferably about 500 mg/$m^2$ body surface area; and

the cisplatin is administered at a single dose of about 60 mg/$m^2$ body surface area to about 90 mg/$m^2$ body surface area, e.g., about 70 mg/$m^2$ body surface area, 75 mg/$m^2$ body surface area, or 80 mg/$m^2$ body surface area, preferably about 75 mg/$m^2$ body surface area.

In one or more embodiments, in the use described herein, the NSCLC is non-squamous cell carcinoma, and the drug or kit is used for providing the anti-PD-1 antibody or the antigen-binding fragment thereof, pemetrexed, and carboplatin at doses described as follows:

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;

the pemetrexed is administered at a single dose of about 200 mg/$m^2$ body surface area to about 800 mg/$m^2$ body surface area, e.g., about 400 mg/$m^2$ body surface area, 500 mg/$m^2$ body surface area, or 600 mg/$m^2$ body surface area, preferably about 500 mg/$m^2$ body surface area; and

the carboplatin is administered at a single dose of about AUC 5, AUC 6, or AUC 7, preferably about AUC 5.

In one or more embodiments, in the use described herein, the NSCLC is non-squamous cell carcinoma, and the drug or kit is used for providing the anti-PD-1 antibody or the antigen-binding fragment thereof, and pemetrexed at doses described as follows, for administration in the maintenance stage:

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;

the pemetrexed is administered at a single dose of about 200 mg/$m^2$ body surface area to about 800 mg/$m^2$ body

surface area, e.g., about 400 mg/$m^2$ body surface area, 500 mg/$m^2$ body surface area, or 600 mg/$m^2$ body surface area, preferably about 500 mg/$m^2$ body surface area.

In one or more embodiments, in the use described herein, the non-small cell lung cancer is squamous cell carcinoma, and the drug or kit is used for providing the anti-PD-1 antibody or the antigen-binding fragment thereof, albumin-bound paclitaxel, and carboplatin at dosing frequencies described as follows:

the anti-PD-1 antibody or the antigen-binding fragment thereof is administered at a frequency of about once every week, once every two weeks, once every three weeks, once every four weeks, or once a month, preferably once every three weeks;
the albumin-bound paclitaxel is administered at a frequency of about once every week, twice every three weeks, once every two weeks, once every three weeks, once every four weeks, or once a month, preferably once every week or twice every three weeks; and
the carboplatin is administered at a frequency of about once every week, once every two weeks, once every three weeks, once every four weeks, or once a month, preferably once every three weeks.

In one or more embodiments, in the use described herein, the non-small cell lung cancer is squamous cell carcinoma, and the drug or kit is used for providing the anti-PD-1 antibody or the antigen-binding fragment thereof at a dosing frequency described as follows, for treatment in the maintenance stage:
the anti-PD-1 antibody or the antigen-binding fragment thereof is administered at a frequency of about once every week, once every two weeks, once every three weeks, once every four weeks, or once a month, preferably once every three weeks.
In one or more embodiments, in the use described herein, the non-small cell lung cancer is non-squamous cell carcinoma, and the drug or kit is used for providing the anti-PD-1 antibody or the antigen-binding fragment thereof, pemetrexed, and cisplatin or carboplatin at dosing frequencies described as follows:

the anti-PD-1 antibody or the antigen-binding fragment thereof is administered at a frequency of about once every week, once every two weeks, once every three weeks, once every four weeks, or once a month, preferably once every three weeks;
the pemetrexed is administered at a frequency of about once every week, once every two weeks, once every three weeks, once every four weeks, or once a month, preferably once every three weeks; and
the cisplatin or carboplatin is administered at a frequency of about once every week, twice every three weeks, once every two weeks, once every three weeks, once every four weeks, or once a month, preferably once every three weeks.

In one or more embodiments, in the use described herein, the non-small cell lung cancer is non-squamous cell carcinoma, and the drug or kit is used for providing the anti-PD-1 antibody or the antigen-binding fragment thereof and pemetrexed at dosing frequencies described as follows, for treatment in the maintenance stage:

the anti-PD-1 antibody or the antigen-binding fragment thereof is administered at a frequency of about once every week, once every two weeks, once every three weeks, once every four weeks, or once a month, preferably once every three weeks; and
the pemetrexed is administered at a frequency of about once every week, once every two weeks, once every three weeks, once every four weeks, or once a month, preferably once every three weeks.

In one or more embodiments, in the use described herein, the non-small cell lung cancer is squamous cell carcinoma, and the drug or kit is used for providing the anti-PD-1 antibody or the antigen-binding fragment thereof, albumin-bound paclitaxel, and carboplatin at administration doses and dosing frequencies described as follows: the anti-PD-1 antibody or the antigen-binding fragment thereof is administered at a fixed dose of 240 mg or 360 mg once every three weeks; the albumin-bound paclitaxel is administered at a single dose of about 100 mg/$m^2$ body surface area once every week or twice every three weeks; and the carboplatin is administered at a single dose of about AUC 5 once every three weeks. In one or more embodiments, the pharmaceutical combination described above is administered in 4-6 cycles.
In one or more embodiments, in the use described herein, the non-small cell lung cancer is squamous cell carcinoma, and the drug or kit is used for providing the anti-PD-1 antibody or the antigen-binding fragment thereof at an administration dose and dosing frequency described as follows: a fixed dose of 240 mg or 360 mg, once every three weeks, for treatment in the maintenance stage.
In one or more embodiments, in the use described herein, the non-small cell lung cancer is non-squamous cell carcinoma, and the drug or kit is used for providing the anti-PD-1 antibody or the antigen-binding fragment thereof,

pemetrexed, and cisplatin at administration doses and dosing frequencies described as follows: the anti-PD-1 antibody or the antigen-binding fragment thereof is administered at a fixed dose of 240 mg or 360 mg once every three weeks; the pemetrexed is administered at a single dose of about 500 mg/$m^2$ body surface area once every three weeks; and the cisplatin is administered at a single dose of about 75 mg/$m^2$ body surface area once every three weeks. In one or more embodiments, the pharmaceutical combination described above is administered in 4-6 cycles.

In one or more embodiments, in the use described herein, the non-small cell lung cancer is non-squamous cell carcinoma, and the drug or kit is used for providing the anti-PD-1 antibody or the antigen-binding fragment thereof, pemetrexed, and carboplatin at administration doses and dosing frequencies described as follows: the anti-PD-1 antibody or the antigen-binding fragment thereof is administered at a fixed dose of 240 mg or 360 mg once every three weeks; the pemetrexed is administered at a single dose of about 500 mg/$m^2$ body surface area once every three weeks; and the carboplatin is administered at a single dose of about AUC 5 once every three weeks. In one or more embodiments, the pharmaceutical combination described above is administered in 4-6 cycles.

In one or more embodiments, in the use described herein, the non-small cell lung cancer is non-squamous cell carcinoma, and the drug or kit is used for providing the anti-PD-1 antibody or the antigen-binding fragment thereof and pemetrexed at administration doses and dosing frequencies described as follows, for treatment in the maintenance stage: the anti-PD-1 antibody or the antigen-binding fragment thereof is administered at a fixed dose of 240 mg or 360 mg once every three weeks; and the pemetrexed is administered at a single dose of about 500 mg/$m^2$ body surface area once every three weeks.

In one or more embodiments, in the use described herein, the anti-PD-1 antibody or the antigen-binding fragment thereof, the albumin-bound paclitaxel, the carboplatin, the cisplatin, and the pemetrexed are administered in cycles of one week, two weeks, three weeks, one month, two months, three months, four months, five months, half a year or longer; optionally, the cycles are identical or different and at identical or different intervals. In one or more embodiments, in the use described herein, the anti-PD-1 antibody or the antigen-binding fragment thereof, the albumin-bound paclitaxel, the carboplatin, the cisplatin, and the pemetrexed are administered parenterally, e.g., by intravenous infusion, in a liquid dosage form, e.g., an injection.

In one or more embodiments, in the use described herein, the anti-PD-1 antibody or the antigen-binding fragment thereof and first-line chemotherapeutic agents (e.g., the albumin-bound paclitaxel, the carboplatin, the cisplatin, and the pemetrexed) in the drug or kit are each independently in a liquid dosage form, e.g., an injection, for parenteral administration, e.g., by intravenous infusion.

In one or more embodiments, in the use described herein, the drug or the kit comprises the anti-PD-1 antibody or the antigen-binding fragment thereof, the albumin-bound paclitaxel, and the carboplatin, wherein the anti-PD-1 antibody or the antigen-binding fragment thereof is in a liquid dosage form, and the albumin-bound paclitaxel and the carboplatin are both in a sterile aqueous injection dosage form.

In one or more embodiments, in the use described herein, the drug or the kit comprises the anti-PD-1 antibody or the antigen-binding fragment thereof, the pemetrexed, and the cisplatin, wherein the anti-PD-1 antibody or the antigen-binding fragment thereof is in a liquid dosage form, the pemetrexed is in a sterile lyophilized powder injection dosage form, and the cisplatin is in a sterile aqueous injection dosage form.

In one or more embodiments, in the use described herein, the drug or the kit comprises the anti-PD-1 antibody or the antigen-binding fragment thereof, the pemetrexed, and the carboplatin, wherein the anti-PD-1 antibody or the antigen-binding fragment thereof is in a liquid dosage form, the pemetrexed is in a sterile lyophilized powder injection dosage form, and the carboplatin is in a sterile aqueous injection dosage form.

In one or more embodiments, in the use described herein, the drug or the kit is the pharmaceutical combination or the kit according to any one of the embodiments of the present invention.

In yet another aspect, the present invention provides a pharmaceutical combination, which comprises an anti-PD-1 antibody or an antigen-binding fragment thereof, albumin-bound paclitaxel, and carboplatin, or comprises an anti-PD-1 antibody or an antigen-binding fragment thereof, pemetrexed, and cisplatin or carboplatin.

In one or more embodiments, provided is the pharmaceutical combination described herein, wherein,

the anti-PD-1 antibody or the antigen-binding fragment thereof comprises a light chain complementarity determining region with amino acid sequences set forth in SEQ ID NOs: 1, 2, and 3, respectively, and a heavy chain complementarity determining region with amino acid sequences set forth in SEQ ID NOs: 4, 5, and 6, respectively; preferably, the anti-PD-1 antibody or the antigen-binding fragment thereof comprises a light chain variable region with an amino acid sequence set forth in SEQ ID NO: 7 and a heavy chain variable region with an amino acid sequence set forth in SEQ ID NO: 8; preferably, the anti-PD-1 antibody comprises a light chain with an amino acid sequence set forth in SEQ ID NO: 9 and a heavy chain with an amino acid sequence set forth in SEQ ID NO: 10; and more preferably, the anti-PD-1 antibody is toripalimab.

In yet another aspect, the present invention provides a method for preventing or treating non-small cell lung cancer, which comprises: administering to an individual in need thereof a therapeutically effective amount of the anti-PD-1 antibody or the antigen-binding fragment thereof described herein or the pharmaceutical combination according

to the present invention in combination.

In yet another aspect, the present invention provides a kit, which comprises:

one or more single dosage units of an anti-PD-1 antibody or an antigen-binding fragment thereof, one or more single dosage units of albumin-bound paclitaxel, and one or more single dosage units of carboplatin, wherein the anti-PD-1 antibody or the antigen-binding fragment thereof is according to any one of the embodiments of the present invention; or

one or more single dosage units of an anti-PD-1 antibody or an antigen-binding fragment thereof, one or more single dosage units of pemetrexed, and one or more single dosage units of carboplatin or cisplatin, wherein the anti-PD-1 antibody or the antigen-binding fragment thereof is according to any one of the embodiments of the present invention; or

one or more single dosage units of the pharmaceutical combination described herein.

In one or more embodiments, the pharmaceutical combination or the kit described herein comprises the anti-PD-1 antibody or the antigen-binding fragment thereof, the albumin-bound paclitaxel, and the carboplatin, wherein the anti-PD-1 antibody or the antigen-binding fragment thereof is in a liquid dosage form, and the albumin-bound paclitaxel and the carboplatin are in a sterile aqueous injection dosage form.

In one or more embodiments, the pharmaceutical combination or the kit described herein comprises the anti-PD-1 antibody or the antigen-binding fragment thereof, the pemetrexed, and the cisplatin, wherein the anti-PD-1 antibody or the antigen-binding fragment thereof is in a liquid dosage form, the pemetrexed is in a sterile lyophilized powder dosage form, and the cisplatin is in a sterile aqueous injection dosage form.

In one or more embodiments, the pharmaceutical combination or the kit described herein comprises the anti-PD-1 antibody or the antigen-binding fragment thereof, the pemetrexed, and the carboplatin, wherein the anti-PD-1 antibody or the antigen-binding fragment thereof is in a liquid dosage form, the pemetrexed is in a sterile lyophilized powder injection dosage form, and the carboplatin is in a sterile aqueous injection dosage form.

In one or more embodiments, the kit is used for treating a patient with non-small cell lung cancer that is squamous cell carcinoma, which comprises one or more groups of combinations of toripalimab, carboplatin, and albumin-bound paclitaxel administered in the induction stage, and one or more groups of toripalimab administered alone in the maintenance stage. Preferably, one group of combinations is at a dose for one treatment cycle, such as three weeks.

In one or more embodiments, the kit is used for treating a patient with non-small cell lung cancer that is non-squamous cell carcinoma, which comprises one or more groups of combinations of toripalimab, pemetrexed, and carboplatin administered in the induction stage, or a combination of toripalimab, pemetrexed, and cisplatin, and one or more groups of combinations of toripalimab and pemetrexed administered in the maintenance stage. Preferably, one group of combinations is at a dose for one treatment cycle, such as three weeks.

In one or more embodiments, the induction stage is 4-6 cycles, and the maintenance stage is achieved after the induction stage is over. In one or more embodiments, the maintenance stage may continue until progressive disease.

## BRIEF DESCRIPTION OF THE DRAWINGS

FIG. 1: Kaplan-Meier curves for PFS assessed by the investigator according to RECIST 1.1 criteria, 1a: intent-to-treat (ITT) analysis set; 1b: patients with non-squamous cell carcinoma; 1c: patients with squamous cell carcinoma.

FIG. 2: Kaplan-Meier curves for PFS assessed by the blind independent review committee (BIRC) according to RECIST 1.1 criteria. 2a: intent-to-treat (ITT) analysis set; 2b: patients with non-squamous cell carcinoma; 2c: patients with squamous cell carcinoma.

FIG. 3: a forest plot of subgroup analysis for PFS assessed by the investigator according to RECIST 1.1 criteria - intent-to-treat (ITT) analysis set.

FIG. 4: Kaplan-Meier curves for overall survival (OS) - intent-to-treat (ITT) analysis set.

FIG. 5: gene mutation spectrum; 5a: patients with non-squamous cell carcinoma; 5b: patients with squamous cell carcinoma.

FIG. 6: tumor mutation burden; 6a: correlation with PD-L1; 6b: response to treatment.

FIG. 7: Kaplan-Meier curves for PFS assessed by the investigator according to RECIST 1.1 criteria; 7a: high TMB; 7b: low TMB.

FIG. 8: Kaplan-Meier curves for overall survival (OS); 8a: high TMB; 8b: low TMB.

FIG. 9: correlation of gene mutations with the therapeutic effect; 9a: progression-free survival; 9b: overall survival.

FIG. 10: correlation of RB1/SMARCA4 deletion with the therapeutic effect.

FIG. 11: correlation of changes in the FA-PI3K-AKT signaling pathway with the therapeutic effect.

FIG. 12: correlation of changes in the IL-7 signaling pathway with the therapeutic effect.
FIG. 13: correlation of changes in the SWI/SNF pathway with the therapeutic effect.

## DETAILED DESCRIPTION

The present invention relates to a method for treating non-small cell lung cancer (NSCLC). The method of the present invention comprises administering to a patient in need thereof an anti-PD-1 antibody or an antigen-binding fragment thereof in combination with a first-line chemotherapeutic agent.

## Terms

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

"Administering", "giving", and "treating" refer to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

"Adverse event" (AE) described herein is any adverse and often unintended or undesirable sign, symptom, or disease associated with the use of medical treatment. For example, an adverse event may be associated with the activation of the immune system or the amplification of immune system cells in response to treatment. The medical treatment may have one or more related AEs, and the AEs each may have identical or different severity levels.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is in the body.

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is in the body.

The terms "subject", "individual", and "object" include any organism, preferably an animal, more preferably a mammal (such as a rat, mouse, dog, cat, and rabbit), and most preferably a human. The terms "subject" and "patient" are used interchangeably herein.

"Antibody" described herein refers to any form of antibody capable of achieving a desired biological or binding activity. Therefore, it is used in the broadest sense, but is not limited to, monoclonal antibodies, polyclonal antibodies, multispecific antibodies, humanized full-length human antibodies, chimeric antibodies, and camelid single-domain antibodies. The "antibody" specifically binds to an antigen and comprises at least two heavy (H) chains and two light (L) chains interconnected by disulfide bonds. Each heavy chain comprises a heavy chain variable region (VH) and a heavy chain constant region comprising three constant domains CH1, CH2 and CH3. Each light chain comprises a light chain variable region (VL) and a light chain constant region comprising one constant domain CL. The VH and VL regions can be further divided into hypervariable regions termed complementarity determining regions (CDRs), which are scattered over more conserved regions termed framework regions (FRs). Generally, both light and heavy chain variable domains comprise FR1, CDR1, FR2, CDR2, FR3, CDR3, and FR4 from N-terminus to C-terminus. Amino acids are typically assigned to each domain according to the following definitions: Sequences of Proteins of Immunological Interest, Kabat et al.; National Institutes of Health, Bethesda, Md.; 5th edition; NIH publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 341:878-883.

The carboxyl-terminal portion of the heavy chain can define a constant region primarily responsible for effector function. Human light chains are generally classified as $\kappa$ and $\lambda$ chains. Human heavy chains are generally classified as $\mu$, $\delta$, $\gamma$, $\alpha$, or $\varepsilon$ chains, and isotypes of the antibody are defined as IgM, IgD, IgG, IgA, and IgE, respectively. IgG subclass is well known to those skilled in the art and includes, but is not limited to, IgG1, IgG2, IgG3, and IgG4.

The term "antibody" includes: naturally occurring and non-naturally occurring Abs; monoclonal and polyclonal Abs; chimeric and humanized Abs; human or non-human Abs; fully synthetic Abs; and single-chain Abs. Non-human

Abs can be humanized by recombinant methods to reduce their immunogenicity in humans.

Unless otherwise specifically indicated, the "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDR regions. Examples of an antigen-binding fragment include, but are not limited to, Fab, Fab', $F(ab')_2$, and Fv fragments; a diabody; a linear antibody; a single-chain antibody molecule; and a nanoantibody and a multispecific antibody formed from fragments of the antibody.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences.

"Humanized antibody" refers to an antibody form containing sequences from both non-human (e.g., murine) and human antibodies. Such antibodies contain minimal sequences derived from non-human immunoglobulins. Generally, a humanized antibody will comprise substantially all of at least one and generally two variable domains, in which all or substantially all of the hypervariable loops correspond to those of a non-human immunoglobulin and all or substantially all of the FR regions are those of a human immunoglobulin. The humanized antibody optionally also comprises at least one portion of an immunoglobulin constant region (Fc), generally a human immunoglobulin constant region.

The term "cancer" or "malignant tumor" used herein refers to a wide variety of diseases characterized by the uncontrolled growth of abnormal cells in the body. Unregulated cell division, growth division and growth lead to the formation of malignant tumors that invade adj acent tissues and can also metastasize to distal parts of the body through the lymphatic system or the blood flow. Examples of cancers suitable for treatment or prevention using the method, the drug, and the kit of the present invention include, but are not limited to, carcinoma, lymphoma, leukemia, blastoma and sarcoma. More specific examples of cancer include squamous cell carcinoma, myeloma, small-cell lung cancer, non-small cell lung cancer, glioma, Hodgkin's lymphoma, non-Hodgkin's lymphoma, acute myeloid leukemia, multiple myeloma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, nasopharyngeal cancer, cervical cancer, brain cancer, gastric cancer, bladder cancer, hepatoma, breast cancer, colon cancer, and head and neck cancer.

The term "tumor mutation burden (TMB)" used herein refers to the total number of somatic gene coding errors, base substitutions, and gene insertion or deletion errors detected per million bases. In some embodiments of the present invention, tumor mutation burden (TMB) is estimated by analysis of somatic mutations, including coding base substitutions and the megabase insertions of the panel sequences studied.

The term "non-small cell lung cancer" refers to non-small cell lung cancer (NSCLC) divided into three categories based on the appearance and other characteristics of the cancer cells: squamous cell carcinoma (SCC, squamous carcinoma), adenocarcinoma, and large cell carcinoma (LCC). SCC accounts for about 25%-30% of all lung cancer cases. SCC is closely associated with smoking and usually develops in the central region of the lung; adenocarcinoma accounts for about 40% of all lung cancer cases and usually develops in the outer region of the lung; LCC accounts for about 10%-15% of all lung cancer cases, and LCC patients usually have rapid tumor growth and poor prognosis. Other less common types of lung cancer include a carcinoid tumor, adenoid cystic carcinoma, hamartoma, lymphoma, and sarcoma.

The term "immunotherapy" refers to the treatment of a subject with a disease or at risk of infection or disease recurrence by a method that includes inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing or preventing the onset, progression, severity, or recurrence of symptoms, complications or conditions, or biochemical indicators associated with the disease.

A "programmed death receptor-1 (PD-1)" refers to an immunosuppressive receptor belonging to the CD28 family. PD-1 is expressed primarily on previously activated T cells *in vivo* and binds to two ligands PD-L1 and PD-L2. The term "PD-1" used herein includes human PD-1 (hPD-1), variants, isotypes, and species homologs of hPD-1, and analogs having at least one common epitope with hPD-1.

A "therapeutically effective amount" or "therapeutically effective dose" of a drug or therapeutic agent is any amount of the drug that, when used alone or in combination with an additional therapeutic agent, protects a subject from the onset of a disease or promotes the regression of a disease as evidenced by a decrease in the severity of disease symptoms, an increase in the frequency and duration of disease symptom-free stage, or the prevention of injury or

disability resulting from the affliction of the disease. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a drug includes a "prophylactically effective amount" which is any amount of a drug that, when administered alone or in combination with an antineoplastic agent, inhibits the development or recurrence of cancer to a subject at risk of developing cancer or a subject having cancer recurrence.

A "biotherapeutic agent" refers to a biomolecule, such as an antibody or fusion protein, that blocks ligand/receptor signaling in any biological pathway that supports tumor maintenance and/or growth or inhibits an anti-tumor immune response.

Unless otherwise specifically indicated, "CDR" used herein refers to a complementarity determining region of the immunoglobulin variable region defined using the Kabat numbering system.

"Therapeutic anti-PD-1 monoclonal antibody" refers to an antibody that specifically binds to the mature form of a specific PD-1 expressed on the surface of certain mammalian cells. Mature PD-1 does not have a secretory leader sequence, or leader peptide. The terms "PD-1" and "mature PD-1" are used interchangeably herein and are to be understood as the same molecule unless otherwise specifically defined, or clearly seen from the context.

The therapeutic anti-human PD-1 antibody or anti-hPD-1 antibody described herein refers to a monoclonal antibody that specifically binds to mature human PD-1.

The "framework region" or "FR" described herein refers to the immunoglobulin variable region excluding CDR regions.

"Isolated antibody or an antigen-binding fragment thereof' refers to a molecule that is in a purified state, and in this case, is substantially free of other biomolecules, such as nucleic acids, proteins, lipids, carbohydrates, or other materials (such as cell debris or growth medium).

"Patient" or "subject" refers to any single subject in need of a medical procedure or participating in a clinical trial, epidemiological study, or serving as a control, and is generally a mammal, including a human and other mammals, such as horses, cows, dogs or cats.

The "RECIST 1.1 efficacy criteria" described herein refers to the definition of target injury and non-target injury described in Eisenhauver E.A. et al., Eur. J Cancer 45:228-247(2009) in the context of the measured response. RECIST 1.1 efficacy criteria is the most commonly used criteria for efficacy assessment of solid tumors prior to immunotherapy. However, with the advent of the immunotherapy, many difficulties that had not previously appeared in tumor evaluation have emerged. Therefore, based on the newly emerging phenomenon brought by immunotherapy itself, the RECIST working group revised the existing "RECIST V.1.1" and proposed a new judgment criterion in 2016, namely the "irRECIST criteria" described herein, aiming at better assessing the efficacy of immunotherapeutic drugs.

The term "ECOG" score standard is an indicator of general health status and tolerance to treatment of patients from their physical strength. ECOG score standard for the physical condition include 0 points, 1 point, 2 points, 3 points, 4 points, and 5 points. A score of 0 means that the motility is completely normal and has no difference from the motility before onset of the disease. A score of 1 means that the person is free to walk and can engage in light physical activities, including general housework or office work, but not in heavy physical activities.

"Sustained response" refers to a sustained therapeutic effect following cessation of treatment with a therapeutic agent or combination therapy described herein. In some embodiments, the sustained response has a duration that is at least the same as the duration of treatment or at least 1.5, 2.0, 2.5 or 3 times the duration of the treatment.

"Tissue section" refers to a single portion or piece of a tissue sample, such as a tissue slice cut from a sample of normal tissue or a tumor.

"Treating" cancer described herein refers to administering to a subject with or diagnosed with cancer the treatment regimen described herein (e.g., administration of an anti-PD-1 antibody) to achieve at least one positive therapeutic effect (e.g., a decrease in cancer cell number, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C $\leq$ 42% for tumor growth inhibition is the minimum level of anti-tumor activity according to the NCI criteria. It is considered that T/C (%) = median volume of treated tumor/median volume of control tumor $\times$ 100. In some embodiments, the therapeutic effect achieved by the combination of the present invention is any one of PR, CR, OR, PFS, DFS, and OS. PFS (also called "time to tumor progression") refers to the length of time during and after treatment for which cancer does not grow, and includes the amount of time a patient experiences CR or PR and the amount of time a patient experiences SD. DFS refers to the length of time during and after treatment for which a patient remains disease-free. OS refers to an extension of life expectancy compared to an initial or untreated individual or a patient. In some embodiments, the response to the combination of the present invention is any one of PR, CR, PFS, DFS, OR, and OS, assessed using RECIST 1.1 efficacy criteria. The treatment regimen of the combination of the present invention effective in treating a patient with cancer may vary depending upon a variety of factors such as the disease state, age, weight of the patient

and the ability of the therapy to elicit an anti-cancer response in the subject. Embodiments of the present invention may not achieve an effective positive therapeutic effect in each subject, but should be effective and achieve a positive therapeutic effect in a statistically significant number of subjects.

The terms "mode of administration" and "dosing regimen" are used interchangeably and refer to the dosage and time of use of each therapeutic agent in the combination of the present invention.

The term "immunohistochemistry (IHC)" refers to a method for determining antigens (polypeptides and proteins) in tissue cells by developing chromogenic agents (fluoresceins, enzymes, metal ions, isotopes) that label antibodies through chemical reactions based on the principle that antigens specifically bind to antibodies, and performing localized, qualitative and relatively quantitative studies on those antigens. In some embodiments of the present invention, a tumor tissue sample from a subject is tested for PD-L1 prior to treatment with an anti-PD-1 antibody by staining the anti-human PD-L1 antibody SP142 from Roche (Cat No: M4422). In some embodiments, the presence of tumor cells with membrane staining ≥ 1% is defined as PD-L1 positive.

In the following paragraphs, various aspects of the present invention are further described in detail.

Anti-PD-1 Antibody

Herein, "PD-1 antibody" refers to any chemical compound or biological molecule that binds to the PD-1 receptor, blocks the binding of PD-L1 expressed on cancer cells to PD-1 expressed on immune cells (T, B, and NK cells), and preferably also blocks the binding of PD-L2 expressed on cancer cells to PD-1 expressed on immune cells. Alternative nouns or synonyms for PD-1 and its ligands include: PDCD1, PD1, CD279, and SLEB2 for PD-1; PDCD1L1, PDL1, B7-H1, B7H1, B7-4, CD274, and B7-H for PD-L1; and PDCD1L2, PDL2, B7-DC, and CD273 for PD-L2. In any of the therapy, the drug, and the use described herein for treating a human subject, the PD-1 antibody blocks the binding of human PD-L1 to human PD-1, and preferably blocks the binding of both human PD-L1 and PD-L2 to human PD1. The amino acid sequence of human PD-1 can be found at NCBI locus number: NP_005009. The amino acid sequences of human PD-L1 and PD-L2 can be found at NCBI locus numbers: NP_054862 and NP_079515.

As used herein, unless otherwise indicated or described, when referring to the term "anti-PD-1 antibody", it includes antigen-binding fragments of the antibody.

The anti-PD-1 antibody suitable for any of the use, the therapy, the pharmaceutical combination, and the kit described herein has an immunosuppressive effect achieved by binding to PD-1 with high specificity and high affinity, blocking the binding of PD-L1/2 to PD-1 and inhibiting PD-1 signal transduction. In any of the use, the therapy, the pharmaceutical combination, and the kit disclosed herein, the anti-PD-1 antibody includes the full-length antibody itself, as well as an antigen-binding portion or fragment that binds to the PD-1 receptor and exhibits functional properties similar to an intact Ab in inhibiting ligand binding and up-regulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized, or human Ab or an antigen-binding fragment thereof. In certain embodiments for treating a human individual, the Ab is humanized Ab.

In some embodiments, the anti-PD-1 antibody for use in any of the use, the therapy, the pharmaceutical combination, and the kit described herein includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from human IgG1, IgG2, IgG3, and IgG4 constant regions; preferably, the anti-PD-1 antibody or the antigen-binding fragment thereof suitable for use in any of the use, the therapy, the pharmaceutical combination, and the kit described herein comprises a heavy chain constant region of human IgG1 or IgG4 isotype, more preferably a human IgG4 constant region. In some embodiments, the sequence of the IgG4 heavy chain constant region of the anti-PD-1 antibody or the antigen-binding fragment thereof comprises the S228P mutation that replaces a serine residue in the hinge region with a proline residue that is typically present at the corresponding position of an antibody of IgG1 isotype.

Preferably, in any one of the embodiments of the use, the therapy, the pharmaceutical combination, and the kit described herein, the PD-1 antibody is a monoclonal antibody or an antigen-binding fragment thereof, which comprises light chain CDRs with amino acid sequences set forth in SEQ ID NOs: 1, 2, and 3, respectively, and heavy chain CDRs having amino acid sequences set forth in SEQ ID NOs: 4, 5, and 6, respectively.

Preferably, in any one of the embodiments of the use, the therapy, the pharmaceutical combination, and the kit described herein, the PD-1 antibody is a monoclonal antibody that specifically binds to human PD-1 and comprises: (a) a light chain variable region set forth in SEQ ID NO: 7, and (b) a heavy chain variable region set forth in SEQ ID NO: 8.

Further preferably, in any one of the embodiments of the use, the therapy, the pharmaceutical combination, and the kit described herein, the PD-1 antibody is a monoclonal antibody that specifically binds to human PD-1 and comprises: (a) a light chain set forth in SEQ ID NO: 9, and (b) a heavy chain set forth in SEQ ID NO: 10.

Table A below provides amino acid sequence numbers of the light chain CDRs and heavy chain CDRs of an exemplary anti-PD-1 antibody mAb for use in the use, the therapy, the pharmaceutical combination, and the kit described herein:

Table A: light and heavy chain CDRs of an exemplary anti-human PD-1 antibody

| | |
|---|---|
| LCDR1 | SEQ ID NO: 1 |
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

An example of an anti-PD-1 antibody that binds to human PD-1 and can be used for the use, the therapy, the pharmaceutical combination, and the kit described herein is described in WO2014206107. Human PD-1 mAb that can be used as an anti-PD-1 antibody in the use, the therapy, the pharmaceutical combination, and the kit described herein includes any one of the anti-PD-1 antibodies described in WO2014206107, including: toripalimab, a humanized IgG4 mAb that has the structure described in WHO Drug Information (Vol. 32, 2nd, pp.372-373 (2018)) and comprises light and heavy chain amino acid sequences set forth in sequences SEQ ID NOs: 9 and 10. In a preferred embodiment, the anti-PD-1 antibody that can be used for any one of the use, the therapy, the pharmaceutical combination, and the kit described herein is selected from humanized antibodies 38, 39, 41, and 48 described in WO2014206107. In a particularly preferred embodiment, the anti-PD-1 antibody that can be used for any one of the use, the therapy, the pharmaceutical combination, and the kit described herein is toripalimab.

The anti-PD-1 antibody that can be used for any one of the use, the therapy, the pharmaceutical combination, and the kit described herein also includes nivolumab and pembrolizumab that have been approved by FDA.

In certain embodiments, the anti-PD-1 antibody that can be used for any one of the use, the therapy, the pharmaceutical combination, and the kit described herein also includes an anti-PD-L1 monoclonal antibody that specifically binds to PD-L1 to block the binding of PD-L1 to PD-1, such as nivolumab, pembrolizumab, toripalimab, sintilimab, camrelizumab, tislelizumab, and cemiplimab.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of a tumor tissue section or by flow cytometry using diagnostic PD-L antibodies. Alternatively, PD-L protein expression of tumor cells can be detected by PET imaging using a binding agent that specifically binds to a desired PD-L target (such as PD-L1 or PD-L2).

Methods for quantifying PD-L1 protein expression in IHC analysis of a tumor tissue section are found in, but are not limited to, Thompson, R. H. et al., PNAS 101(49): 17174-17179 (2004); Taube, J. M. et al., Sci Transl Med 4, 127ra37 (2012); Topalian, S. L. et al., New Eng. J. Med. 366(26): 2443-2454 (2012), and the like.

In one method, a simple binary endpoint of positive or negative PD-L1 expression is adopted, where the positive expression is defined by the percentage of tumor cells showing histological evidence of cell surface membrane staining. The case where tumor cells on a tumor tissue section account for at least 1% of the total tumor cells is defined as positive PD-L1 expression.

In another method, PD-L1 expression in a tumor tissue section is quantified in tumor cells as well as in infiltrating immune cells. The percentage of tumor cells and infiltrating immune cells exhibiting membrane staining are quantified individually as < 1%, 1% to 50%, and subsequent 50% to 100%. For tumor cells, the PD-L1 expression is counted as negative if the score is < 1%, and positive if the score is ≥ 1%.

In some embodiments, the expression level of PD-L1 by malignant cells and/or by infiltrating immune cells within the tumor is determined to be "overexpressed" or "elevated" based on comparison with the expression level of PD-L1 by an appropriate control. For example, the protein or mRNA expression level of control PD-L1 can be a quantified level in non-malignant cells of the same type or in sections from matched normal tissue.

Albumin-Bound Paclitaxel

The active pharmaceutical ingredient of the albumin-bound paclitaxel is paclitaxel, and the human serum albumin is used as an auxiliary material to play the roles of dispersing and stabilizing particles and carrying a main drug. Paclitaxel is a natural secondary metabolite separated and purified from the bark of the gymnosperm yew, which has been clinically

verified to have a good anti-tumor effect. The chemical name of paclitaxel is 5β,20-epoxy-1,2α,4,7β,10β,13α-hexahydroxytax-11-en-9-one-4,10-diacetate-2-benzoate-13-(2*R*,3*S*)-*N*-benzoyl-3-phenylisoserine, which has a structure shown in the formula below.

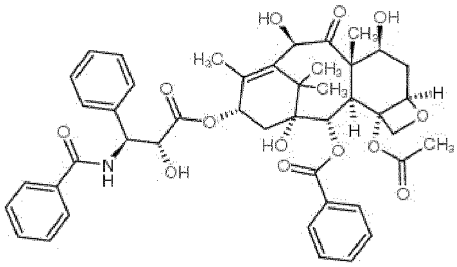

In some embodiments of the present invention, paclitaxel may also refer to a composition comprising a therapeutically effective amount of a compound represented by the structural formula described above or a pharmaceutically acceptable salt thereof, and a pharmaceutically acceptable excipient. In some embodiments, the albumin-bound paclitaxel is in a sterile aqueous injection dosage form.

Pemetrexed

Pemetrexed is an anti-folate formulation structurally containing a pyrrolopyrimidine group as a core, which inhibits cell replication by destroying normal folate-dependent metabolic processes within the cell, thereby inhibiting tumor growth. Pemetrexed is a compound having a structure shown in the formula below.

In some embodiments of the present invention, the pemetrexed may also refer to a composition comprising a therapeutically effective amount of a compound represented by the structural formula described above or a pharmaceutically acceptable salt thereof, and a pharmaceutically acceptable excipient. In some embodiments, the pemetrexed is a pemetrexed disodium salt. In some embodiments, the pemetrexed is in a sterile lyophilized powder injection dosage form.

Carboplatin

Carboplatin, discovered by Clear et al. in 1980, was first marketed in the UK in 1986, and approved for marketing in 1989 by FDA in the United States, with applications gradually spreading. In 1990, the production of carboplatin powder and injection was approved in China. Carboplatin, as a second-generation platinum compound, has the biochemical characteristics similar to those of cisplatin, is a new drug that has received widespread attention in recent years, and is a cell cycle non-specific drug. It acts mainly on N7 and O6 atoms of guanine in DNA, causing inter- and intra-strand cross-linking of DNA, destroying DNA molecules, preventing its helical melting, interfering with DNA synthesis, and thus producing a cytotoxic effect. Carboplatin is a compound having a structure shown in the formula below:

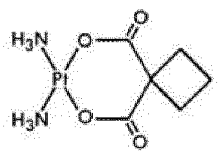

In some embodiments of the present invention, the carboplatin may also refer to a composition comprising a therapeutically effective amount of a compound represented by the structural formula described above or a pharmaceutically acceptable salt thereof, and a pharmaceutically acceptable excipient. In some embodiments, the carboplatin comprises cis-1,1-cyclopropanedicarboxylic acid diammineplatinum and edetate disodium. In some embodiments, the carboplatin is in a sterile aqueous injection dosage form.

Cisplatin

Cisplatin, the first metal complex with anti-cancer activity, was first discovered in 1965 by American scientists B.Rosenborg et al. Cisplatin, also known as cis-dichlorodiammineplatinum, is a platinum-containing anti-cancer drug in the form of orange-yellow or yellow crystalline powder, which is slightly soluble in water and readily soluble in dimethylformamide and can be gradually converted into trans and hydrolyzed in aqueous solution. Cisplatin can clinically show efficacy on various solid tumors. Carboplatin is a compound having a structure shown in the formula below:

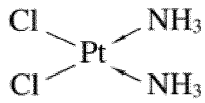

.

In some embodiments of the present invention, the cisplatin may also refer to a composition comprising a therapeutically effective amount of a compound represented by the structural formula described above or a pharmaceutically acceptable salt thereof, and a pharmaceutically acceptable excipient. In some embodiments, the cisplatin comprises (cis)-diamminedichloroplatinum, sodium chloride, and polyethylene glycol 400. In some embodiments, the carboplatin is in a sterile aqueous injection dosage form.

Pharmaceutical combination

The present invention provides a pharmaceutical combination, which comprises the anti-PD-1 antibody or the antigen-binding fragment thereof described herein, albumin-bound paclitaxel, and carboplatin; or comprises an anti-PD-1 antibody or an antigen-binding fragment thereof, pemetrexed, and cisplatin or carboplatin; or an anti-PD-1 antibody or an antigen-binding fragment thereof and pemetrexed. In the drug composition, the anti-PD-1 antibody or the antigen-binding fragment thereof, the albumin-bound paclitaxel, and the carboplatin may be provided as a mixture of the three (i.e., as a pharmaceutical composition), or as a mixture of any two and another separate formulation, or as separate formulations. In the pharmaceutical combination, the anti-PD-1 antibody or the antigen-binding fragment thereof, the pemetrexed, and the cisplatin or carboplatin may be provided as a mixture of the three (i.e., as a pharmaceutical composition), or as a mixture of any two and another separate formulation, or as separate formulations. In some embodiments, the pharmaceutical combination is administered at doses for three weeks, comprising 1 dose of the anti-PD-1 antibody or the antigen-binding fragment thereof described herein, 2 or 3 doses of albumin-bound paclitaxel, and 1 dose of carboplatin. In some embodiments, the pharmaceutical combination is administered at doses for three weeks, comprising 1 dose of the anti-PD-1 antibody or the antigen-binding fragment thereof described herein, 1 dose of pemetrexed, and 1 dose of cisplatin or carboplatin. When present as separate formulations, each formulation may also comprise a pharmaceutically acceptable carrier in addition to the active ingredient. Preferably, the anti-PD-1 antibody or the antigen-binding fragment thereof is in a liquid dosage form, the albumin-bound paclitaxel, the cisplatin, and the carboplatin are all in a sterile aqueous injection dosage form, and the pemetrexed is in a sterile lyophilized powder injection dosage form.

In some embodiments, the anti-PD-1 antibody or the antigen-binding fragment thereof described herein may be preferably according to any one of the embodiments herein, more preferably an antibody comprising light chain CDRs with amino acid sequences set forth in SEQ ID NOs: 1, 2, and 3, respectively, and heavy chain CDRs with amino acid sequences set forth in SEQ ID NOs: 4, 5, and 6, respectively, more preferably a monoclonal antibody comprising a light chain variable region set forth in SEQ ID NO: 7 and a heavy chain variable region set forth in SEQ ID NO: 8, more preferably a monoclonal antibody comprising a light chain set forth in SEQ ID NO: 9 and a heavy chain set forth in SEQ ID NO: 10, more preferably humanized antibodies 38, 39, 41, and 48 described in Patent No. WO2014206107, and most preferably toripalimab.

As described herein, "pharmaceutically acceptable carrier" includes any and all solvents, dispersion media, coatings, antibacterial and antifungal agents, isotonic and absorption delaying agents, and the like that are physiologically compatible. Preferably, the carrier suitable for the composition comprising the anti-PD-1 antibody is suitable for intravenous, intramuscular, subcutaneous, parenteral, spinal, or epidermal administration, such as by injection or infusion, while the carrier suitable for the composition comprising an additional anti-cancer agent is suitable for enteral administration, such as oral administration. The pharmaceutical composition of the present invention can contain one or more pharmaceutically acceptable salts, antioxidants, water, non-aqueous carriers, and/or adjuvants such as preserving agent, wetting agent, emulsifying agent, and dispersing agent.

The pharmaceutical combination of the present invention may also comprise one or more additional therapeutic agents. The additional therapeutic agent can be, for example, a chemotherapeutic agent, a biotherapeutic agent, an immunogenic agent (e.g., an attenuated cancerous cell, a tumor antigen, an antigen-presenting cell (such as a dendritic cell pulsed with a tumor-derived antigen or nucleic acid), an immunostimulatory cytokine (e.g., IL-2, IFN-tumor, GM-CSF), and a cell transfected with a gene encoding an immunostimulatory cytokine (such as, but not limited to, GM-CSF)).

Preferably, each pharmaceutical combination comprises a dose of the drug corresponding to the amount required for 1 administration cycle.

Dose and Dosing Regimen

The selection of a dosing regimen (also referred to herein as an administration regimen) for the pharmaceutical combination of the present invention depends on several factors, including the solid serum or tissue turnover rate of the treated individual, the level of symptoms, the overall immunogenicity, and the degree of accessibility of the target cell, tissue or organ. Preferably, the dosing regimen maximizes the amount of each therapeutic agent delivered to the patient, consistent with acceptable level of side effects. Thus, the dose and dosing frequency of each of the biotherapeutic and chemotherapeutic agents will depend, in part, on the particular therapeutic agent, the severity of cancer being treated, and the characterization of the patient. Guidance in selecting appropriate doses of antibodies, cytokines and small molecules may be obtained. Determination of an appropriate dosage regimen may be made by a clinician, for example, with reference to parameters or factors known or suspected to affect treatment or expected to affect treatment in the art, and will depend on, for example, the clinical history of the patients (e.g., previous treatments), the type and stage of cancer being treated, and biomarkers in response to one or more therapeutic agents in the combination therapy.

Each therapeutic agent of the pharmaceutical combination of the present invention may be administered simultaneously (i.e., in the same pharmaceutical composition), concurrently (i.e., in separate pharmaceutical formulations, one after the other in any order), or sequentially in any order. Sequential administration is particularly useful where the therapeutic agents in the pharmaceutical combination can be in different dosage forms (one drug is a tablet or capsule and the other drug is a sterile liquid formulation) and/or on different dosing schedules (e.g., the chemotherapeutic agent is administered at least daily and the biotherapeutic agent is administered less frequently (e.g., once every week, once every two weeks or once every three weeks)).

In some embodiments, the therapeutic agent in at least one of the pharmaceutical combinations is administered using the same dosage regimen (therapeutic dose, frequency and duration) as that generally used when the agent is used as a monotherapy for treating the same tumor. In other embodiments, the patient receives at least one of the therapeutic agents in the combination therapy at a lower total amount than that when the agent is used as a monotherapy, e.g., a lower dose, a lower dosing frequency, and/or a shorter duration of treatment.

Each therapeutic agent in the pharmaceutical combination of the present invention can be administered orally or parenterally, including intravenous, intramuscular, intraperitoneal, subcutaneous, rectal, topical and transdermal routes of administration.

The anti-PD-1 antibody or the antigen-binding fragment thereof of the present invention can be administered by continuous infusion or by intermittent dosing. The single administration dose may be in a range of about 0.01 mg/kg body weight to about 20 mg/kg body weight or about 0.1 mg/kg body weight to about 10 mg/kg body weight, or may be a fixed dose of about 120 mg to about 480 mg. For example, the dose may be about 0.1 mg/kg body weight, about 0.3 mg/kg body weight, about 1 mg/kg body weight, about 2 mg/kg body weight, about 3 mg/kg body weight, about 4 mg/kg body weight, about 5 mg/kg body weight, about 6 mg/kg body weight, about 7 mg/kg body weight, about 8 mg/kg body weight, about 9 mg/kg body weight, or about 10 mg/kg body weight. Alternatively, the dose may be a fixed dose of about 120 mg, 240 mg, 360 mg, or 480 mg. The dosing regimen is generally designed to achieve an exposure that results in sustained receptor occupancy (RO) based on the typical pharmacokinetics of Ab. A representative dosing regimen may be given about once a week, about once every two weeks, about once every three weeks, about once every four weeks, about once a month, or longer. In some embodiments, the anti-PD-1 antibody is administered to the individual about once every three weeks.

In some embodiments, the anti-PD-1 antibody of the present invention is toripalimab administered at a single dose selected from about 1 mg/kg body weight to about 5 mg/kg body weight. In some embodiments, the toripalimab is administered intravenously at a single dose selected from about 1 mg/kg body weight, 2 mg/kg body weight, 3 mg/kg body weight, 4 mg/kg body weight, and 5 mg/kg body weight, or fixed doses of about 120 mg, 240 mg, and 360 mg. In some preferred embodiments, the toripalimab is administered as a liquid drug at a selected dose administered by intravenous infusion over a period of 30-60 min. In some embodiments, the toripalimab is administered at about 3 mg/kg body weight or a fixed dose of about 240 mg by intravenous infusion over a period of 30 min once every three weeks (Q3W). In some embodiments, the toripalimab is administered at about 4.5 mg/kg body weight or a fixed dose of about 360mg by intravenous infusion over a period of 30 min once every three weeks (Q3W).

In some embodiments, the albumin-bound paclitaxel of the present invention is administered at an approved or recommended dose, with treatment continued until a clinical effect is observed or until unacceptable toxicity or progressive disease occurs. In some embodiments, the albumin-bound paclitaxel of the present invention is administered at a single dose selected from about 60 $mg/m^2$ body surface area to about 140 $mg/m^2$ body surface area. In some embodiments, the albumin-bound paclitaxel of the present invention is administered at a single dose selected from about 60 $mg/m^2$ body surface area, 80 $mg/m^2$ body surface area, 100 $mg/m^2$ body surface area, 120 $mg/m^2$ body surface area, and 140 $mg/m^2$ body surface area. A representative dosing regimen may be given about once a week, about once every two weeks, about once every three weeks, about once every four weeks or about once a month. In some embodiments, the albumin-bound paclitaxel is administered to the individual once every week. In some embodiments, the

albumin-bound paclitaxel is administered to the individual twice every three weeks. In some embodiments, the albumin-bound paclitaxel is administered at about 100 mg/$m^2$ body surface area once every week (Q1W). In some embodiments, the albumin-bound paclitaxel is administered at about 100 mg/$m^2$ body surface area twice every three weeks.

As used herein, the body surface area (BSA) is defined by the Dubois formula: BSA ($m^2$) = 0.20247 × height $(m)^{0.725}$ × weight $(kg)^{0.425}$.

In some embodiments, the carboplatin of the present invention is administered at an approved or recommended dose, with treatment continued until a disease maintenance stage is achieved, or until unacceptable toxicity or progressive disease occurs. In some embodiments, the carboplatin of the present invention is administered at a single dose selected from about AUC 5, AUC 6, and AUC 7. In some embodiments, the carboplatin is administered at a single dose of about AUC 5. A representative dosing regimen may be given about once a week, about once every two weeks, about once every three weeks, about once every four weeks or about once a month. In some embodiments, the carboplatin is administered to the individual once every three weeks. In some embodiments, the carboplatin is administered at about AUC 5 once every three weeks.

The AUC 5 dose of carboplatin can be calculated by using the Calvert formula (Calvert et al. 1989):
Calvert formula

$$\text{Total dose (mg)} = (\text{target AUC}) \times (\text{glomerular filtration rate [GFR]} + 25)$$

Note: GFR used in the Calvert formula to calculate AUC-based dose should not exceed 125 mL/min. Therefore, maximum dose of carboplatin = 5 × (125 + 25) = 750 mg.

In this scheme, GFR is considered to correspond to creatinine clearance (CRCL). CRCL was calculated by using the following formula according to institutional guidelines or the method of Cockcroft and Gault (1976):

$$\text{CRCL} = \frac{(140\text{-age})\ (\text{wt})}{72 \times \text{Scr}}\ (\times\ 0.85,\ \text{if female})$$

wherein:

CRCL = creatinine clearance, in mL/min;
age = patient age, in years;
wt = patient body weight, in kg; and
Scr = serum creatinine, in mg/dL.

Or:

$$\text{CRCL} = \frac{(140\text{-age})\ (\text{wt})}{0.814 \times \text{Scr}}\ (\times\ 0.85,\ \text{if female})$$

wherein:

CRCL = creatinine clearance, in mL/min;
age = patient age, in years;
wt = patient body weight, in kg; and
Scr = serum creatinine, in μmol/L.

In some embodiments, the pemetrexed of the present invention is administered at an approved or recommended dose, with treatment continued until a clinical effect is observed or until unacceptable toxicity or progressive disease occurs. In some embodiments, the pemetrexed of the present invention is administered at a single dose selected from about 200 mg/$m^2$ body surface area to about 800 mg/$m^2$ body surface area. In some embodiments, the pemetrexed of the present invention is administered at a single dose selected from about 200 mg/$m^2$ body surface area, 400 mg/$m^2$ body surface area, 500 mg/$m^2$ body surface area, 600 mg/$m^2$ body surface area, and 800 mg/$m^2$ body surface area. A representative dosing regimen may be given about once a week, about once every two weeks, about once every three weeks, about once every four weeks or about once a month. In some embodiments, the pemetrexed is administered to the individual once every three weeks. In some embodiments, the pemetrexed is administered at about 500 mg/$m^2$ body surface area once every three weeks (Q3W).

In some embodiments, the cisplatin of the present invention is administered at an approved or recommended dose, with treatment continued until a clinical effect is observed or until unacceptable toxicity or progressive disease occurs. In some embodiments, the cisplatin of the present invention is administered at a single dose selected from about 60 $mg/m^2$ body surface area to about 90 $mg/m^2$ body surface area. In some embodiments, the cisplatin of the present invention is administered at a single dose selected from about 60 $mg/m^2$ body surface area, 70 $mg/m^2$ body surface area, 75 $mg/m^2$ body surface area, 80 $mg/m^2$ body surface area, and 90 $mg/m^2$ body surface area. A representative dosing regimen may be given about once a week, about once every two weeks, about once every three weeks, about once every four weeks or about once a month. In some embodiments, the cisplatin is administered to the individual once every three weeks. In some embodiments, the cisplatin is administered at about 75 $mg/m^2$ body surface area once every three weeks (Q3W).

In some embodiments, the toripalimab is administered at a fixed dose of about 240 mg, Q3W, the albumin-bound paclitaxel is administered at about 500 $mg/m^2$ body surface area once a week, and the carboplatin is administered at about AUC 5, Q3W. In some embodiments, the toripalimab is administered at a fixed dose of about 240 mg, Q3W, the albumin-bound paclitaxel is administered at about 500 $mg/m^2$ body surface area twice every three weeks, and the carboplatin is administered at about AUC 5, Q3W.

In some embodiments, the toripalimab is administered at a fixed dose of about 240 mg, Q3W, the pemetrexed is administered at about 500 $mg/m^2$ body surface area, Q3W, and the cisplatin is administered at about 75 $mg/m^2$ body surface area, Q3W. In some embodiments, the toripalimab is administered at a fixed dose of about 240 mg, Q3W, the pemetrexed is administered at about 500 $mg/m^2$ body surface area, Q3W, and the carboplatin is administered at about AUC 5, Q3W. In some embodiments, the toripalimab is administered at a fixed dose of about 240 mg, Q3W, and the pemetrexed is administered at about 500 $mg/m^2$ body surface area, Q3W.

In some embodiments, the albumin-bound paclitaxel may be administered before or after toripalimab administration and the carboplatin may be administered before or after toripalimab administration on the day of toripalimab administration. In some embodiments, the pemetrexed may be administered before or after toripalimab administration and the carboplatin/cisplatin may be administered before or after toripalimab administration on the day of toripalimab administration.

The anti-PD-1 antibody or the antigen-binding fragment thereof, the albumin-bound paclitaxel, the carboplatin, the cisplatin, and the pemetrexed of the present invention may be administered in identical or different cycles of one week, two weeks, three weeks, one month, two months, three months, four months, five months, half a year or longer; optionally, the cycles may be identical or different and at identical or different intervals. In some embodiments, one administration/treatment cycle is three weeks. In some embodiments, in one administration cycle, the toripalimab is administered at a fixed dose of about 240 mg once every three weeks, the pemetrexed is administered at about 500 $mg/m^2$ body surface area once every three weeks, and the cisplatin is administered at about 75 $mg/m^2$ body surface area once every three weeks. All three are administered in 4-6 cycles, i.e., 12-18 weeks. In some embodiments, in one administration cycle, the toripalimab is administered at a fixed dose of about 240 mg once every three weeks, the pemetrexed is administered at about 500 $mg/m^2$ body surface area once every three weeks, and the carboplatin is administered at about AUC 5 once every three weeks. All three are administered in 4-6 cycles, i.e., 12-18 weeks. In some embodiments, in one administration cycle, the toripalimab is administered at a fixed dose of about 240 mg once every three weeks, the albumin-bound paclitaxel is administered at about 100 $mg/m^2$ body surface area once a week or twice every three weeks, and the carboplatin is administered at about AUC 5 once every three weeks. All three are administered in 4-6 cycles, i.e., 12-18 weeks.

Treatment Method and Use

The present invention provides use of a combination of the anti-PD-1 antibody or the antigen-binding fragment thereof according to any one of the embodiments of the present invention, and a first-line chemotherapeutic agent, in the preparation of a drug or kit for treating non-small cell lung cancer. Preferably, the drug and kit are according to any one of the embodiments herein. In one or more embodiments, the present invention provides use of the pharmaceutical combination according to any one of the embodiments herein in the preparation of a drug or kit for treating non-small cell lung cancer.

The present invention also provides a method for preventing or treating non-small cell lung cancer, which comprises: administering to an individual in need thereof an effective amount of the anti-PD-1 antibody or the antigen-binding fragment thereof in combination with a first-line chemotherapeutic agent, or the pharmaceutical combination described herein. The effective amount includes a prophylactically effective amount and a therapeutically effective amount. In preferred embodiments, the dosing regimen of the prevention or treatment method is according to any one of the embodiments herein.

The present invention also provides a pharmaceutical combination of the anti-PD-1 antibody or the antigen-binding fragment thereof according to any one of the embodiments of the present invention and a first-line chemotherapeutic agent, for use in preventing or treating non-small cell lung cancer.

The non-small cell lung cancer described herein may be according to any one of the preceding embodiments; preferably, the non-small cell lung cancer of the present invention is an untreated advanced non-small cell lung cancer.

Preferably, the method, the use, the anti-PD-1 antibody, and the pharmaceutical combination according to any one of the embodiments of the present invention are particularly suitable for squamous cell NSCLC or non-squamous cell NSCLC, preferably non-squamous cell NSCLC.

Preferably, the method, the use, and the pharmaceutical composition according to any one of the embodiments of the present invention are particularly suitable for non-small cell lung cancer positive for PD-L1 expression in immunohistochemical staining analysis of a tumor tissue section; preferably non-small cell lung cancer with PD-L1 $\geq$ 50% in immunohistochemical staining analysis of a tumor tissue section.

Preferably, the method, the use, and the pharmaceutical composition according to any one of the embodiments of the present invention are particularly suitable for advanced non-small cell lung cancer not accompanied by EGFR-sensitive mutations and ALK fusions.

Preferably, the method, the use, and the pharmaceutical composition according to any one of the embodiments of the present invention are particularly suitable for non-small cell lung cancers with a tumor mutation burden of $\geq$ 10 mutations/Mbp, and accompanied by gene mutations of an FA/PI3K/Akt signaling pathway (e.g., mutations in one or more genes of COL3A1, COL6A3, FLT1, FLNC, HGF, IRS1, IRS2, ITGA4, ITGA8, and KDR), gene mutations of an IL-7 signaling pathway (e.g., mutations in one or more genes of HGF, IRS1, IRS2, and SMARCA4), or gene mutations of an SWI/SNF chromatin remodeling complex (e.g., mutations in one or more genes of SMARCA4, SMARCA2, and PBRM1).

Preferably, the method, the use, and the pharmaceutical composition according to any one of the embodiments of the present invention are particularly suitable for non-small cell lung cancer with a tumor mutation burden of $\geq$ 10 mutations/Mbp, and accompanied by mutations in one or more genes of RB1, KEAP1, and SMARCA4 (especially in the non-squamous cell subgroup).

The anti-PD-1 antibody for non-small cell lung cancer may be preferably according to any one of the embodiments herein, more preferably an antibody comprising light chain CDRs with amino acid sequences set forth in SEQ ID NOs: 1, 2, and 3, respectively, and heavy chain CDRs with amino acid sequences set forth in SEQ ID NOs: 4, 5, and 6, respectively, more preferably a monoclonal antibody comprising a light chain variable region set forth in SEQ ID NO: 7 and a heavy chain variable region set forth in SEQ ID NO: 8, more preferably a monoclonal antibody comprising a light chain set forth in SEQ ID NO: 9 and a heavy chain set forth in SEQ ID NO: 10, more preferably humanized antibodies 38, 39, 41, and 48 described in Patent No. WO2014206107, and most preferably toripalimab.

Preferred first-line chemotherapeutic agents for squamous cell NSCLC may be according to any one of the embodiments herein, and are preferably carboplatin and albumin-bound paclitaxel. Preferred first-line chemotherapeutic agents for non-squamous cell NSCLC may be according to any one of the embodiments herein, and are preferably pemetrexed and carboplatin or cisplatin.

In a more preferred embodiment, the present invention provides a method for treating squamous cell NSCLC, which comprises administering to a patient with squamous cell NSCLC a therapeutically effective amount of toripalimab, carboplatin, and albumin-bound paclitaxel; preferably, the patient is positive for PD-L1 expression. Preferably, the dosing regimen of the treatment method is according to any one of the embodiments herein. In a more preferred embodiment, the present invention provides a method for treating non-squamous cell NSCLC, which comprises administering to a patient with squamous cell NSCLC a therapeutically effective amount of toripalimab, pemetrexed, and carboplatin or cisplatin; preferably, the patient is positive for PD-L1 expression. Preferably, the dosing regimen of the treatment method is according to any one of the embodiments herein.

In a more preferred embodiment, the present invention provides use of the anti-PD-1 antibody or the antigen-binding fragment thereof, the carboplatin, and the albumin-bound paclitaxel in the preparation of a drug or kit for preventing or treating squamous cell NSCLC. Preferably, the squamous cell NSCLC is positive for PD-L1 expression in an immunohistochemical staining analysis of a tumor tissue section. In a more preferred embodiment, the present invention provides use of the anti-PD-1 antibody or the antigen-binding fragment thereof, the pemetrexed, and the carboplatin or cisplatin in the preparation of a drug or kit for preventing or treating non-squamous cell NSCLC. Preferably, the non-squamous cell NSCLC is positive for PD-L1 expression in an immunohistochemical staining analysis of a tumor tissue section. Preferably, the drug and kit are according to any one of the embodiments herein.

Kit

The present invention also provides a kit comprising one or more single dosage units of the anti-PD-1 antibody or the antigen-binding fragment thereof according to any one of the embodiments herein, one or more single dosage units of albumin-bound paclitaxel, and one or more single dosage units of carboplatin; or one or more single dosage units of the anti-PD-1 antibody or the antigen-binding fragment thereof, one or more single dosage units of pemetrexed,

and one or more single dosage units of carboplatin/cisplatin, wherein the anti-PD-1 antibody or the antigen-binding fragment thereof is according to any one of the embodiments of the present invention; or one or more single dosage units of the pharmaceutical combination described herein.

In some embodiments, the amount and dose of each drug comprised in the kit is sufficient to enable administration of the drug according to the dosing regimen according to any one of the embodiments herein.

In some embodiments, the kit comprises one or more single dosage units of the pharmaceutical combination according to any one of the embodiments herein. In some embodiments, the kit comprises one or more groups of pharmaceutical formulations, each group of pharmaceutical formulations being administered at doses for three weeks, comprising 1 dose of toripalimab, 2 or 3 doses of albumin-bound paclitaxel, and 1 dose of carboplatin. Preferably, toripalimab is at a fixed dose of about 240 mg, the 2 or 3 doses of albumin-bound paclitaxel are sufficient to be administered twice or three times at about 100 mg/$m^2$ body surface area, and the 1 dose of carboplatin is sufficient to be administered once at about AUC 5.

In some embodiments, the kit comprises one or more groups of pharmaceutical formulations, each group of pharmaceutical formulations being administered at doses for 3 weeks, comprising 1 dose of toripalimab, 1 dose of pemetrexed, and 1 dose of cisplatin or carboplatin. Preferably, toripalimab is at a fixed dose of about 240 mg, the 1 dose of pemetrexed is sufficient to be administered once at about 500 mg/$m^2$ body surface area, the 1 dose of carboplatin is sufficient to be administered once at about AUC 5, and the 1 dose of cisplatin is sufficient to be administered once at about 75 mg/$m^2$ body surface area. When present as separate formulations, each formulation may also comprise a pharmaceutically acceptable carrier in addition to the active ingredient.

Preferably, in the kit, one group of pharmaceutical formulations corresponds to 1 administration cycle. In some embodiments, the kit may comprise 1-6 groups of pharmaceutical formulations for administration in 1-6 treatment cycles. In some embodiments, the kit may comprise 4-6 groups of pharmaceutical formulations for administration in 4-6 treatment cycles. In a preferred embodiment, 1 administration cycle is 3 weeks.

The kit of the present invention can be used for treating non-small cell lung cancer (NSCLC).

Abbreviation

The following abbreviations are used throughout the description and examples of the present invention:

| | |
|---|---|
| BID | One dose, twice a day |
| CDR | Complementarity determining region |
| DFS | Disease-free survival |
| FR | Framework region |
| IgG | Immunoglobulin G |
| IHC | Immunohistochemistry |
| OR | Overall response |
| ORR | Objective response rate |
| DCR | Disease control rate |
| OS | Overall survival |
| mOS | Mean overall survival |
| PD | Progressive disease |
| PFS | Progression-free survival |
| mPFS | Mean progression-free survival |
| PR | Partial response |
| CR | Complete response |
| SD | Stable disease |
| DLT | Dose-limiting toxicity |
| MTD | Maximum tolerated dose |
| Q2W | One dose every 2 weeks |
| QD | One dose everyday |
| CSD | Chronic sun-damaged |
| non-CSD | Non-chronic sun-damaged |
| IRC | Independent review committee |
| AE | Adverse event |
| TRAE | Treatment-related adverse event |
| SAE | Serious adverse event |
| RO | Receptor occupancy |
| UC | Urothelial carcinoma |

| | |
|---|---|
| RCC | Renal cell carcinoma |
| MM | Metastatic melanoma |
| RECIST | Response Evaluation Criteria in Solid Tumor |
| irRECIST | Immune-Related Response Evaluation Criteria in Solid Tumor |
| DOR | Duration of response |
| TTR | Time to response |
| BIRC | Blinded Individual Review Committee |
| NE | Not evaluable |
| TMB | Tumor mutation burden |

The present invention is further illustrated by the following examples, which should not be construed as limiting the present invention. The contents of all references cited throughout the present application are explicitly incorporated herein by reference.

## EXAMPLES

Example 1: Clinical study of an anti-PD-1 antibody in combination with standard first-line chemotherapy for the treatment of untreated advanced non-small cell lung cancer

### 1.1 Clinical design

This is a randomized, double-blind, placebo-controlled, multicenter, phase III clinical study for assessing the efficacy and safety of toripalimab (JS001) or placebo in combination with first-line chemotherapy in patients with untreated advanced NSCLC and exploring the population with optimal biomarker prediction.

About 450 patients with advanced NSCLC not accompanied by EGFR-sensitive mutations and ALK fusions were randomly divided into two groups according to a 2:1 ratio, with the test group receiving toripalimab in combination with first-line standard chemotherapy and the control group receiving placebo in combination with first-line standard chemotherapy. Stratification was performed according to PD-L1 expression (TC $\geq$ 1% vs TC < 1%), smoking status (frequent smoking vs no smoking or little smoking), and pathological type (squamous cell carcinoma vs non-squamous cell carcinoma).

After randomization, the patients were subjected to treatment in the induction stage for 4-6 cycles, during which the patients received toripalimab/placebo + first-line standard chemotherapy. After the induction stage was over, the patients were subjected to treatment in the maintenance stage, during which patients with squamous cell carcinoma continued to receive toripalimab/placebo treatment until progressive disease, and patients with non-squamous cell carcinoma continued to receive toripalimab/placebo + pemetrexed treatment until progressive disease.

Toripalimab 240 mg/placebo was administered by intravenous infusion once every three weeks (Q3W) in cycles of 21 days, with treatment continued until the patients met the discontinuation criteria, i.e., recorded progressive disease, unacceptable AEs, investigator-assessed unsuitability for continued treatment, withdrawal of informed consent, 2 years of JS001 treatment, or other reasons as specified in the protocol.

The patients with squamous cell carcinoma received albumin-bound paclitaxel + carboplatin treatment in the induction stage: intravenous drip of albumin-bound paclitaxel at 100 mg/m$^2$ on days 1, 8, and 15 of each cycle (whether albumin-bound paclitaxel was administered on day 15 was at the discretion of the investigator), and administration of carboplatin at AUC 5 on day 1 of each cycle.

The patients with non-squamous cell carcinoma received pemetrexed + cisplatin/carboplatin treatment in the induction stage: intravenous drip of pemetrexed at 500 mg/m$^2$ and cisplatin at 75 mg/m$^2$ on day 1 of each cycle, or intravenous drip of pemetrexed at 500 mg/m$^2$ and carboplatin at AUC 5 on day 1 of each cycle. The selection of cisplatin or carboplatin was at the discretion of the investigator.

Tumor assessments were performed once every 6 weeks for the first 12 months of the study stage according to RECIST 1.1 and iRECIST; after 12 months, tumor assessments were performed every 9 weeks until progressive disease, initiation of a new anti-tumor therapy, intolerable toxicity, withdrawal of informed consent or death, 2 years of treatment with toripalimab, or other reasons as specified in the protocol, whichever occurred first.

### 1.2 Primary inclusion criteria

Eligible subjects must (1) be patients with histologically and/or cytologically confirmed locally advanced (stage IIIB or IIIC) and stage IV non-small cell lung cancer that is inoperable and cannot receive radical concurrent chemoradiotherapy; (2) have at least one measurable lesion (according to RECIST 1.1); (3) have not received any systemic treatment (patients with recurrence more than 6 months after completion of prior neoadjuvant/adjuvant therapy may also

be enrolled); (4) be aged between 18 and 75 years; (5) have ECOG of 0-1; (6) have an expected survival of ≥ 3 months; and (7) be available to provide tissue specimens for PD-L1 testing.

**1.3 Subjects**

From April 2019 to November 2020, patients were screened and enrolled at 60 sites in China, with approximately 450 patients planned to be enrolled (approximately 300 in the test group and 150 in the control group) and 465 actually enrolled (309 in the test group and 156 in the control group). Demographics of the enrolled subjects are shown in Table 1.

Table 1: Demographics of the enrolled subjects

| | JS001 + standard first-line chemotherapy N (%) (N = 309) | Placebo + standard first-line chemotherapy N (%) (N = 156) | Total N (%) (N = 465) |
|---|---|---|---|
| **Age** | | | |
| Median age (years) | 63 (36 - 75) | 61 (29 - 75) | 62.0 (29 - 75) |
| < 65 years | 179 (57.9) | 101 (64.7) | 280 (60.2) |
| **Gender** | | | |
| Male | 247 (79.9) | 130 (83.3) | 377 (81.1) |
| Female | 62 (20.1) | 26 (16.7) | 88 (18.9) |
| **Ethnicity** | | | |
| Han | 300 (97.1) | 153 (98.1) | 453 (97.4) |
| Others | 9 (2.9) | 3 (1.9) | 12 (26) |
| **ECOG** | | | |
| 0 | 66 (21.4) | 36 (23.1) | 102 (21.9) |
| 1 | 243 (78.6) | 120 (769) | 363 (78.1) |
| **Pathological type (randomization)** | | | |
| Squamous cell carcinoma | 147 (47.6) | 73 (46.8) | 220 (47.3) |
| Non-squamous cell carcinoma | 162 (52.4) | 83 (53.2) | 245 (52.7) |
| **PD-L1 expression (randomization)** | | | |
| TC≥1% | 201 (65.0) | 103 (66.0) | 304 (65.4) |
| TC<1% | 108 (35.0) | 53 (34.0) | 161 (34.6) |
| **Smoking status (randomization)** | | | |
| Frequent smoking | 213 (68.9) | 107 (68.6) | 320 (688) |
| No smoking or little smoking | 96 (31.1) | 49 (31.4) | 145 (31.2) |
| **Clinical stage** | | | |
| IIIB/IIIC | 49 (159) | 23 (14.7) | 72 (155) |
| IVA | 141 (456) | 82 (52.6) | 223 (48.0) |
| IVB | 119 (38.5) | 51 (32.7) | 170(366) |
| **Site of metastasis** | | | |
| Brain metastasis | 5 (1.6) | 0 | 5 (1.1) |
| Liver metastasis | 26 (8.4) | 14 (9.0) | 40 (8.6) |

(continued)

| Site of metastasis | | | |
|---|---|---|---|
| Bone metastasis | 95 (30.7) | 43 (27.6) | 138 (297) |
| Number of organs of metastasis ≥ 3 | 53 (17.2) | 24 (15.4) | 77 (16.6) |
| **Previous therapy** | | | |
| Has received neoadjuvant/adjuvant therapy | 13(4.2) | 9(5.8) | 22(47) |
| Has received surgical treatment | 27(8.7) | 18(11.5) | 45(97) |
| Has received radiotherapy | 11(3.6) | 1(0.6) | 12(2.6) |

**1.4 Specification, batch number, and dosage and administration of test drugs**

The specification, batch number, and dosage and administration of test drugs are shown in the following Table 2.

Table 2: Specification, batch number, and dosage and administration of test drugs

| | Study drug | Standard chemotherapy | Standard chemotherapy | Standard chemotherapy | Standard chemotherapy | Control drug |
|---|---|---|---|---|---|---|
| Name: | Toripalimab injection | Pemetrexed disodium lyophilized powder | Albumin-bound paclitaxel inj ection | Carboplati n injection | Cisplatin injection | Placebo |
| Specificat ion: | 240 mg/6 mL/vial | 500 mg/vial | 100 mg/vial | 100 mg/injectio n | 20 mg/injection | 6 mL/vial |
| Dosage: | 240 mg | 500 mg/ $m^2$ | 100mg/$m^2$ | AUC 5 | 75mg/$m^2$ | Not applicable |
| Administr ation: | Intravenous drip, Q3W, administered on the first day of each cycle. | Dose calculated based on body surface area, intravenous drip, Q3W, administered on the first day of each cycle. | Dose calculated based on body surface area, intravenous drip, once every week (Q1W), administered on days 1, 8, and 15. | Intravenou s drip, Q3W, administere d on day 1 of each cycle. | Dose calculated based on body surface area, intravenous drip, Q3W, administered on day 1 of each cycle. | Intravenous drip, Q3W, administered on day 1 of each cycle. |

### 1.5 Duration of treatment

Toripalimab injection 240 mg/placebo was administered by intravenous infusion (Q3W) in cycles of 21 days, with treatment continued until the patients met the discontinuation criteria, i.e., recorded progressive disease, unacceptable AEs, investigator-assessed unsuitability for continued treatment, withdrawal of informed consent, 2 years of JS001 treatment, or other reasons as specified in the protocol.

The patients received JS001/placebo + first-line standard chemotherapy in the induction stage (4-6 cycles). After the induction stage was over, the patients were subjected to treatment in the maintenance stage, during which patients with squamous cell carcinoma continued to receive JS001/placebo treatment until disease progression, and patients with non-squamous cell carcinoma continued to receive JS001/placebo + pemetrexed treatment until disease progression.

### 1.6 Statistics

Analysis set

Intent-to-treat (ITT) set: including all randomized patients during the primary study stage. This analysis set was used as the primary analysis set for efficacy analysis. Per protocol set (PPS): including all ITT populations having valid baseline information without major violation to the protocol that may affect the efficacy analysis. The PPS population was determined based on actual protocol violations before database lock of the study. The PPS population was used for sensitivity analysis of the primary efficacy endpoints as well as part of the secondary efficacy endpoints. Safety analysis set (SS): including all patients who received at least one dose of the study drug (JS001/placebo/chemotherapeutic agent). Crossover treatment analysis set: including all patients who were randomized to the group of placebo in combination with standard first-line chemotherapy to receive treatment, and who crossed over after progressive disease and received treatment with at least one dose of JS001.

Efficacy analysis

Unless otherwise specified, the efficacy analysis was based on the ITT set, which was analyzed by groups according to the treatment regimen to which patients were assigned at randomization. The PPS population was used for sensitivity analysis of the primary efficacy endpoints as well as part of the secondary efficacy endpoints.

The progression-free survival rate and median progression-free survival of the JS001 + first-line standard treatment group and the placebo + first-line standard treatment group at different time points after the start of treatment were estimated by the Kaplan-Meier method, and Kaplan-Meier curves were plotted. The 95% confidence interval for progression-free survival rate at different time points (6 months and 1 year) was estimated using the Greenwood formula. The 95% confidence interval for median progression-free survival was estimated using the Brookmeyer-Crowley method where the log-log function conversion was used to reach normal approximation. Statistical test was performed on the inter-group difference by a stratified log-rank method for the total population. Statistical test was performed on the inter-group difference by a non-stratified log-rank method for the subpopulations. A stratified COX proportional hazards model was used to estimate the inter-group hazard ratio (HR) and the corresponding 95% confidence interval, and the tied events were processed by an exact method. Stratification analysis was performed using 3 stratification factors in the randomization process, including: (1) PD-L1 expression (TC $\geq$ 1% vs TC < 1%); (2) smoking status (frequent smoking vs no smoking or little smoking); and (3) pathological type (squamous cell carcinoma vs non-squamous cell carcinoma).

For analysis of secondary efficacy endpoints OS, DOR, and TTR, the method described above as applied to the primary efficacy endpoint PFS was used. For ORR and DCR, the percentage of patients in each treatment group was calculated, and their 95% confidence intervals were calculated using the Clopper-Pearson method. The nominal P values for inter-group comparison were calculated using the Cochran-Mantel-Haenszel method considering randomization stratification factors that were the same as the primary efficacy analysis, and the 95% CI for the inter-group rate differences was estimated.

Safety analysis

Safety analysis was performed in SS and crossover treatment analysis sets, where safety data from the double-blind stage of the primary study and the crossover treatment stage were analyzed separately. Safety analyses include drug exposure, compliance, adverse events, laboratory examinations, vital signs, physical examinations, electrocardiographic examinations, ECOG scores, etc.

## 2 Study results

A total of 835 patients were screened in this study and 465 patients were randomly enrolled (309 patients in the test group [162 patients with non-squamous cell carcinoma and 147 patients with squamous cell carcinoma], and 156 patients in the control group [83 patients with non-squamous cell carcinoma and 73 patients with squamous cell carcinoma]). All patients were included in the ITT population; one patient with squamous cell carcinoma in the test group did not receive the test drug, and a total of 464 patients were included in the SS population; 47 patients in the control group (22 patients with non-squamous cell carcinoma and 25 patients with squamous cell carcinoma) with progressive disease received JS001 monotherapy after unblinding and were included in the crossover treatment analysis set; 9 patients in the test group (4 patients with non-squamous cell carcinoma and 5 patients with squamous cell carcinoma) with progressive disease received JS001 monotherapy after unblinding. Median follow-up time was 7.10 months for all patients.

Three stratification factors between the two treatment groups were shown to be balanced, and the remaining demographic characteristics and baseline clinical disease characteristics were also substantially balanced, where patients with brain metastasis were not balanced between groups due to the small total number, 5 patients with brain metastasis were randomized to the test group, and there were no significant differences in the values of the remaining proportions. The median age was 62.0 years for all patients enrolled, with the majority of patients being male (81.1%) with an ECOG score of 1 (78.1%) and PD-L1-expressing TC ≥ 1% (65.2% according to CRF records). Of all patients enrolled, 220 (47.3%) were patients with squamous cell carcinoma and 245 (52.7%) were patients with non-squamous cell carcinoma. At enrollment, 72 (15.5%) patients were in stage IIIb/IIIc; 394 (84.7%) patients were patients with metastatic non-small cell lung cancer, of which 223 (48.0%) were patients in stage IVa (141 [45.6%] and 82 [52.6%] for the test and control groups, respectively) and 170 (36.6%) were patients in stage IVb (119 [38.5%] and 51 [32.7%] for the test and control groups, respectively). The most common metastases were contralateral lung metastases (35.7%), pleural/pleural cavity metastases (33.5%), and bone metastases (29.7%). In summary, in the ITT population, the baseline characteristics of the patients enrolled were balanced and not significantly different between the test and control groups.

### Efficacy results

The primary endpoint of this study was PFS assessed by the investigator according to RECIST 1.1. As of November 17, 2020, a total of 218 PFS events have been collected for interim analysis as planned, with a median follow-up time of 7.1 months. The results for this interim analysis showed that the primary endpoint satisfied the primary efficacy endpoint boundary conditions (two-sided significance level cutoff $p \leq 0.03593$) preset for interim analysis, HR = 0.58 (95% CI: 0.442, 0.769; p = 0.0001), and a consistent benefit was observed in both the non-squamous cell carcinoma subgroup and the squamous cell carcinoma subgroup. The efficacy results as assessed by the BICR showed good consistency with those as assessed by the investigator.

The results for the primary and secondary efficacy endpoints in the ITT analysis set are as follows:

### 2.1 PFS assessed by investigator according to RECIST 1.1 (primary endpoint)

As shown in FIG. 1a, in the ITT population, median PFS was 8.3 (95% CI: 6.9, 8.7) months for the patients in the test group and 5.6 (95% CI: 5.4, 6.4) months for the patients in the control group (HR 0.58; 95% CI: 0.442, 0.769; two-sided p = 0.0001), which satisfied the primary efficacy endpoint boundary conditions (two-sided significance level cutoff ≤ 0.03593) preset for interim analysis. Toripalimab in combination with chemotherapy significantly prolonged PFS by 2.7 months and reduced the risk of progression or death by 42% as compared to standard chemotherapy for patients with first-line non-small cell lung cancer. As shown in FIG. 1b, in patients with non-squamous cell carcinoma, median PFS was 8.4 (95% CI: 7.4, 9.9) months for the patients in the test group and 5.5 (95% CI: 4.2, 8.3) months for the patients in the control group (HR 0.59; 95% CI: 0.403, 0.867; two-sided p = 0.0060); as shown in FIG. 1c, in patients with squamous cell carcinoma, median PFS was 7.1 (95% CI: 5.7, 8.3) months for the patients in the test group and 5.6 (95% CI: 5.3, 5.7) months for the patients in the control group (HR 0.55; 95% CI: 0.376, 0.825; two-sided p = 0.0030). A significant benefit in PFS was observed in both patients with non-squamous cell carcinoma and patients with squamous cell carcinoma, and the benefit trend was similar to that in the overall population. Meanwhile, in the populations of the PD-L1 positive and negative subgroups (TC ≥ 1% vs TC < 1), various clinical stage subgroups, liver metastasis and bone metastasis subgroups, and the like, PFS subgroup analysis showed that the result supported the superiority in PFS of the test group to that of the control group (see FIG. 3).

For the test group and the control group, the PFS rates at 6 months were 62.3% (95% CI: 55.59%, 68.30%) and 41.5% (95% CI: 32.06%, 50.68%), respectively; the PFS rates at 12 months were 32.6% (95% CI: 24.94%, 40.43%) and 13.1% (95% CI: 5.69%, 23.58%), respectively. The PFS rate at 6 months for the test group was increased by 20.8% and the PFS rate at 12 months for the test group was increased by 19.5% as compared to those for the control group,

respectively, demonstrating a stable benefit trend.

### 2.2 PFS assessed by BIRC according to RECIST 1.1

As shown in FIG. 2a, in the ITT population, median PFS was 8.3 (95% CI: 6.9, 10.1) months for the patients in the test group and 5.8 (95% CI: 5.6, 7.2) months for the patients in the control group (HR 0.61; 95% CI: 0.450, 0.826; two-sided p = 0.0012). Toripalimab in combination with chemotherapy significantly prolonged PFS by 2.5 months and reduced the risk of progression or death by 39% as compared to standard chemotherapy for patients with first-line non-small cell lung cancer. As shown in FIG. 2b, in patients with non-squamous cell carcinoma, median PFS was 10.3 (95% CI: 8.3, NE) months for the patients in the test group and 6.9 (95% CI: 5.1, 10.0) months for the patients in the control group (HR 0.55; 95% CI: 0.362, 0.854; two-sided p = 0.0061); as shown in FIG. 2c, in patients with squamous cell carcinoma, median PFS was 6.9 (95% CI: 5.8, 8.3) months for the patients in the test group and 5.7 (95% CI: 5.4, 6.9) months for the patients in the control group (HR 0.63; 95% CI: 0.417, 0.970; two-sided p = 0.0314). A significant benefit in PFS was observed in both patients with non-squamous cell carcinoma and patients with squamous cell carcinoma, and the benefit trend was similar to that in the overall population.

For the test group and the control group, the PFS rates at 6 months were 65.3% (95% CI: 58.51%, 71.21%) and 49.3% (95% CI: 38.72%, 58.96%), respectively; the PFS rates at 12 months were 38.9% (95% CI: 30.28%, 47.32%) and 16.2% (95% CI: 6.90%, 28.93%), respectively. The PFS rate at 6 months for the test group was increased by 16% and the PFS rate at 12 months for the test group was increased by 22.7% as compared to those for the control group, respectively, demonstrating a stable benefit trend.

### 2.3 Objective response rate (ORR) and disease control rate (DCR)

The ORR was 63.4% for the test group and 41.7% for the control group as assessed by the investigator according to RECIST v1.1. The test group showed a 21.7% increase in ORR as compared to the control group. The DCR was 90.9% for the test group and 90.4% for the control group, both being greater than 90%, showing good tumor control.

As shown in Table 3, in patients with non-squamous cell carcinoma, the ORR was 58.6% for the patients in the test group and 26.5% for the patients in the control group, and the ORR for the test group was increased by 32.1% as compared to that of the control group; in patients with squamous cell carcinoma, the ORR was 68.7% for the patients in the test group and 58.9% for the patients in the control group, and the ORR for the test group was increased by 9.8% as compared to that of the control group. The ORR for the test group was observed to be superior to that of the control group in both patients with non-squamous cell carcinoma and patients with squamous cell carcinoma.

The ORR was 65.7% for the test group and 44.9% for the control group as assessed by BIRC according to RECIST v1.1. The test group showed a 20.8% increase in ORR as compared to the control group. The DCR was 90.9% for the test group and 91.0% for the control group, both being greater than 90%, showing good tumor control.

As shown in Table 4, in patients with non-squamous cell carcinoma, the ORR was 59.9% for the patients in the test group and 32.5% for the patients in the control group, and the ORR for the test group was increased by 27.4% as compared to that of the control group; in patients with squamous cell carcinoma, the ORR was 72.1% for the patients in the test group and 58.9% for the patients in the control group, and the ORR for the test group was increased by 13.3% as compared to that of the control group. The ORR for the test group was observed to be superior to that of the control group in both patients with non-squamous cell carcinoma and patients with squamous cell carcinoma.

### 2.4 Duration of response (DOR)

In 261 patients with BOR reaching CR or PR (196 in the test group and 65 in the control group), the median DOR was 8.3 months for the patients in the test group and 4.2 months for the patients in the control group (HR 0.56; 95% CI: 0.370, 0.862) as assessed by the investigator according to RECIST1.1. The test group showed a significantly prolonged DOR by 4.1 months as compared to the control group. As shown in Table 3, in patients with non-squamous cell carcinoma, the median DOR was 8.6 months for the patients in the test group and 5.1 months for the patients in the control group (HR 0.63; 95% CI: 0.315, 1.353); in patients with squamous cell carcinoma, the median DOR was 6.9 months for the patients in the test group and 4.2 months for the patients in the control groups (HR 0.46; 95% CI: 0.278, 0.758).

In patients with BOR reaching CR or PR in the overall population, the patients with non-squamous cell carcinoma, and the patients with squamous cell carcinoma, a significant prolongation (in terms of numerical values) of the duration of response was observed in both the test group and the control group.

**2.5 Time to response (TTR)**

In 261 patients with BOR reaching CR or PR (196 in the test group and 65 in the control group) as assessed by the investigator, the median TTR was 1.5 (95% CI: 1.4, 1.6) months for the patients in the test group and 1.4 (95% CI: 1.4, 1.5) months for the patients in the control group. As shown in Table 3, in patients with non-squamous cell carcinoma, the median TTR was 1.4 (95% CI: 1.4, 1.9) months for the patients in the test group and 2.1 (95% CI: 1.3, 2.8) months for the patients in the control group; in patients with squamous cell carcinoma, the median TTR was 1.5 (95% CI: 1.4, 1.6) months for the patients in the test group and 1.4 months (95% CI: 1.4, 1.5) for the patients in the control groups.

In patients with BOR reaching CR or PR in the overall population, the patients with non-squamous cell carcinoma, and the patients with squamous cell carcinoma, the time to response was substantially similar between the test group and the control group.

Table 3: Assessment by the investigator according to RECIST 1.1 criteria (subjects with squamous cell carcinoma/non-squamous cell carcinoma)

| | **Non-squamous cell carcinoma N (%)** | | **Squamous cell carcinoma N (%)** | |
|---|---|---|---|---|
| | **JS001 + standard first-line chemotherapy (N = 162)** | **Placebo + standard first-line chemotherapy (N = 83)** | **JS001 + standard first-line chemotherapy (N = 147)** | **Placebo + standard first-line chemotherapy (N = 73)** |
| **Best overall response for tumors** | | | | |
| Complete response (CR) | 0 | 0 | 0 | 0 |
| Partial response (PR) | 95 (58.6) | 22 (26.5) | 101 (68.7) | 43 (58.9) |
| Stable disease (SD) | 54 (33.3) | 50 (60.2) | 31 (21.1) | 26 (35.6) |
| Progressive disease (PD) | 5 (3.1) | 9 (108) | 6 (4.1) | 3 (41) |
| Not evaluable (NE) | 0 | 1 (1.2) | 1 (0.7) | 0 |
| Deletion | 8 (4.9) | 1 (1.2) | 8 (5.4) | 1 (1.4) |
| **Objective response rate (ORR)** | 95 (58.6) | 22 (26.5) | 101 (68.7) | 43 (58.9) |
| 95% confidence interval | (506,663) | (17.4, 37.3) | (60.5, 76.1) | (468,703) |
| **Duration of response (DOR)** | | | | |
| Median (95% confidence interval) | 8.6 (6.9, NE) | 5.1 (2.9, NE) | 6.9 (4.5, NE) | 4.2 (3.5, 5.5) |

(continued)

| Time to response (TTR) | | | | |
|---|---|---|---|---|
| Median (95% confidence interval) | 1.4 (1.4, 1.9) | 2.1 (1.3, 2.8) | 1.5 (1.4, 1.6) | 1.4 (1.4, 1.5) |
| Note: 95% confidence intervals for objective response rate and disease control rate were calculated for the groups by the Clopper-Pearson method. | | | | |

The ORR and DCR for squamous cell carcinoma/non-squamous cell carcinoma subgroups were subjected to non-stratified analysis, and 95% confidence intervals for the inter-group rate differences were estimated based on the normal approximation.

Table 4: Assessment by the Blinded Individual Review Committee (BIRC) according to RECIST 1.1 criteria (subjects with squamous cell carcinoma/non-squamous cell carcinoma)

| | **Non-squamous cell carcinoma N (%)** | | **Squamous cell carcinoma N (%)** | |
|---|---|---|---|---|
| | **JS001 + standard first-line chemotherapy (N = 162)** | **Placebo + standard first-line chemotherapy (N = 83)** | **JS001 + standard first-line chemotherapy (N = 147)** | **Placebo + standard first-line chemotherapy (N = 73)** |
| **Best overall response for tumors** | | | | |
| Complete response (CR) | 3 (1.9) | 0 | 0 | 0 |
| Partial response (PR) | 94 (58.0) | 27 (32.5) | 106 (72.1) | 43 (58.9) |
| Stable disease (SD) | 49 (30.2) | 45 (54.2) | 29 (19.7) | 27 (37.0) |
| Non-complete response/non-progressive disease (Non CR/Non PD) | 0 | 2 (2.4) | 1 (0.7) | 0 |
| Progressive disease (PD) | 8 (4.9) | 8 (9.6) | 3 (20) | 2 (27) |
| Not evaluable (NE) | 0 | 0 | 0 | 0 |
| Deletion | 8 (4.9) | 1 (1.2) | 8 (5.4) | 1 (1.4) |
| **Objective response rate (ORR)** | 97 (59.9) | 27 (32.5) | 106 (72.1) | 43 (58.9) |
| 95% confidence interval | (51.9,67.5) | (22.6,43.7) | (64.1,79.2) | (468,703) |
| **Duration of response (DOR)** | | | | |
| Median (95% confidence interval) | NE (6.9, NE) | 9.8 (4.1, NE) | 5.7 (5.2, 9.5) | 4.2 (3.5, 5.5) |
| Note: 95% confidence intervals for objective response rate and disease control rate were calculated for the groups by the Clopper-Pearson method. | | | | |

The ORR and DCR for squamous cell carcinoma/non-squamous cell carcinoma subgroups were subjected to non-stratified analysis, and 95% confidence intervals for the inter-group rate differences were estimated based on the normal approximation.

### 2.6 Overall survival (OS)

As of March 11, 2021, the median follow-up time of this study was 9.82 months, and a total of 127 (27.3%) deaths were reported, 79 (25.6%) in the test group and 48 (30.8%) in the control group. As shown in FIG. 4, in the ITT population, median OS was 21.0 months for the test group and 16.0 months for the control group (HR 0.81; 95% CI: 0.568, 1.171; two-sided p = 0.2539). In patients with non-squamous cell carcinoma, the median OS was NE for the patients in the test group and 19.0 months for the patients in the control group (HR 0.56; 95% CI: 0.341, 0.917; two-sided p = 0.0182); in patients with squamous cell carcinoma, the median OS was 21.0 months for the patients in the test group and 16.0 months for the patients in the control group (HR 1.23; 95% CI: 0.732, 2.163; two-sided p = 0.4390). In this interim analysis, the OS follow-up time and the number of OS events were relatively limited. The OS data were not mature, but the test group in the overall population showed a significant survival benefit trend as compared to the control group.

Summary of efficacy results: the results of this interim analysis showed that the primary endpoint PFS assessed by the investigator based on RECIST 1.1 reached the pre-set endpoint boundary value for superiority (two-sided significance level cutoff $P \leq 0.03593$), HR = 0.58 (95% CI: 0.442, 0.769; p = 0.0001), and toripalimab in combination with chemotherapy significantly prolonged progression-free survival by 2.7 months and reduced the risk of progression or death by 42% as compared to standard chemotherapy for patients with first-line non-small cell lung cancer. The PFS results as assessed by the BIRC showed good consistency with those as assessed by the investigator; in other secondary efficacy endpoints, the ORR and the DOR were significantly increased in the test group as compared to those in the control group, and the onset of effect was similar in both groups. Meanwhile, in primary and secondary efficacy endpoints, benefits consistent with the overall population were observed in both patients with non-squamous cell carcinoma and patients with squamous cell carcinoma. The overall survival data were not mature, but the test group in the overall population showed a significant survival benefit trend.

### 2.7 Safety results

All safety analyses were based on the SS population (464 patients), including 308 patients in the test group and 156 patients in the control group. Both groups were sufficiently exposed to JS001 + chemotherapy treatment or placebo + chemotherapy treatment. The primary safety analysis results based on the SS population in the double-blind stage of the primary study are as follows:

The proportion of patients who experienced at least 1 TEAE was 99.0% for the patients in the test group and 99.4% for the patients in the control group; the proportion of patients who experienced at least 1 TEAE related to the treatment regimen (JS001/placebo/any one of the chemotherapeutic agents) was 98.1% for the patients in the test group and 98.7% for the patients in the control group; the proportion of TEAEs of grade 3 or higher as per CTCAE was 76.3% for the test group and 80.1% for the control group, wherein the proportion of TEAEs of grade 3 or higher as per CTCAE that were judged by the investigator to be related to the treatment regimen was 71.4% for the test group and 78.8% for the control group. Therefore, combining JS001 with standard first-line chemotherapy did not increase the incidence of adverse events of grade 3 or higher. The proportion of SAEs occurring during treatment was 38.6% for the test group and 30.1% for the control group, and the proportion of SAEs judged by the investigator to be related to the treatment regimen was 31.2% for the test group and 23.1% for the control group, with the overall SAE and incidence rate as expected; wherein, the proportion of SAEs of grade 3 or higher judged by the investigator to be related to the treatment regimen was 22.4% for the test group and 18.6% for the control group, which were close to each other.

Common TEAEs include anemia (88.3% for the test group vs 94.2% for the control group), neutropenia (83.4% vs 87.8%), leukopenia (83.1% vs 84.0%), thrombocytopenia (68.5% vs 72.4%), elevated alanine aminotransferase (35.7% vs 28.8%), fatigue (33.4% vs 35.9%), anorexia (32.1% vs 35.3%), nausea (31.8% vs 35.9%), elevated aspartate aminotransferase (31.8% vs 23.7%), fever (20.8% vs 19.2%), rash (20.1% vs 12.8%), and constipation (18.5% vs 26.3%), and the like. TEAEs judged by the investigator to be related to the drug were essentially the same as common TEAEs, most of which were common adverse events related to common chemotherapy and were at similar incidence rates in the test group and the control group. Therefore, combining JS001 with chemotherapy did not increase chemotherapy-related toxicity. In common TEAEs, higher incidence rates of elevated alanine aminotransferase, elevated aspartate aminotransferase, hypothyroidism (14.9% vs. 3.8%), and rash test groups were observed, consistent with previously observed safety profile characteristics of JS001.

Common SAEs include thrombocytopenia (6.8% for the test group vs 4.5% for the control group), neutropenia (5.5% vs 3.2%), anemia (5.2% vs 5.8%), infectious pneumonia (4.9% vs 5.8%), leukopenia (2.9% vs 2.6%), bone marrow

depression (1.0% vs 4.5%), and the like. SAEs judged by the investigator to be related to JS001 include anemia (2.6%), immune-mediated pneumonia (2.6%), thrombocytopenia (2.3%), neutropenia (1.9%), infectious pneumonia (1.6%), interstitial lung disease (1.3%), liver function abnormality (1.3%), leukopenia (1.0%), immune-mediated hepatitis (1.0%), pulmonary inflammation (1.0%), and the like. The overall incidence rate of SAEs related to JS001 was low.

Summary of safety results: in the SS population in the double-blind stage of the primary study, the proportion of TEAEs, TEAEs related to the treatment scheme, TEAEs of grade 3 or higher as per CTCAE, and SAEs of grade 3 or higher that were judged by the investigator to be related to the treatment regimen were similar in the test group and the control group. Therefore, combining JS001 with chemotherapy did not increase chemotherapy-related toxicity. JS001-related adverse events were similar to those observed in previous studies, and no new safety signs were found.

**2.8 Biomarker analysis**

As of October 31, 2021, whole exome sequencing (WES) was performed on tumor biopsies and matched peripheral blood from 394 patients. As shown in FIGs. 5a and 5b, overlapping but distinct mutation spectra were observed from both histologic subtypes.

Tumor mutation burden (TMB) was determined by analyzing somatic mutations within coding regions of the human genome. The median TMB in each group was 6.6 mutations per million base pairs (Mb). Tumor tissue from 122 (26.2%) patients had more than 10 mutations/Mb, 72.1% of which was also PD-L1 TC $\geq$ 1% (see FIG. 6a). In TMB-H (i.e., high TMB) patients (TMB $\geq$ 10 mutations/Mb), the ORR was 72.7% for the toripalimab group and 46.7% for the placebo group ($P < 0.0001$) (see FIG. 6b), similar to the response rate in the ITT population, 65.7% vs. 46.2% ($P < 0.0001$). However, compared to TMB-L (i.e., low TMB) patients (TMB < 10 mutations/Mb), TMB-H patients had better PFS in the toripalimab group than in the placebo group (interaction P = 0.026) (see FIGs. 7a and 7b). In contrast, similar OS benefits were observed in both TMB subgroups (interaction P = 0.9962) (see FIGs. 8a and 8b).

Further mutation data analysis showed that gene mutations had significant interactions with therapeutic effects (see FIGs. 9a and 9b), with related genes including RB1, KEAP1, and SMARCA4. For patients carrying the SMARCA4 mutation (n = 48), especially in the non-squamous cell subgroup (n = 33), PFS was significantly improved in the toripalimab group as compared to that in the placebo group (median PFS: 9.9 months vs 2.9 months, see FIG. 10).

Gene set enrichment analysis was performed on the relevant genes to identify over-represented biological pathways. It was found that focal adhesions (FAs) passing through the PI3K/Akt signaling axis (COL3A1, COL6A3, FLT1, FLNC, HGF, IRS1, IRS2, ITGA4, ITGA8, and KDR) appeared to be one of the most therapeutically effective routes. Patients carrying FA/PI3K/Akt pathway mutations had significantly better PFS and OS benefits from combination therapy with toripalimab than from chemotherapy alone (median PFS: 8.9 months vs 4.2 months, median OS: NR vs 13.77 months, with an interaction P value < 0.01, see FIG. 11). In addition, patients with alterations in the IL-7 signaling pathway (HGF, IRS1, IRS2, and SMARCA4) or chromatin remodeling SWI/SNF complex (SMARCA4, SMARCA2, and PBRM1) also had better PFS results from combination therapy with toripalimab (see FIGs. 12-13).

**Conclusion:**

The analysis results for this interim analysis show that toripalimab in combination with chemotherapy significantly prolongs PFS and reduces the risk of progressive disease or death as compared to chemotherapy for first-line treatment of patients with non-small cell lung cancer; the results as assessed by the BIRC have good consistency with those as assessed by the investigator; in secondary efficacy endpoints, significant increases (in terms of numerical values) in ORR and DOR are also observed in the toripalimab group, and the above efficacy effect endpoints have consistent benefit trend in the patients with non-squamous cell carcinoma and patients with squamous cell carcinoma. The test group in the overall population has shown a significant survival benefit trend. Meanwhile, the tolerability and safety in the target indication population are generally controllable, and no new safety signal is found.

## Claims

1. Use of a combination of an anti-PD-1 antibody or an antigen-binding fragment thereof and a first-line chemotherapeutic agent in the preparation of a drug or kit for treating non-small cell lung cancer (NSCLC).

2. The use according to claim 1, wherein, the non-small cell lung cancer is advanced non-small cell lung cancer;

   preferably, the non-small cell lung cancer is untreated advanced non-small cell lung cancer;
   preferably, the non-small cell lung cancer is advanced non-small cell lung cancer not accompanied by EGFR-sensitive mutations and ALK fusions;

preferably, the non-small cell lung cancer is non-small cell lung cancer with a tumor mutation burden of ≥ 10 mutations/Mbp;
further preferably, the non-small cell lung cancer is non-small cell lung cancer accompanied by gene mutations of an FA/PI3K/Akt signaling pathway (e.g., mutations in one or more of genes COL3A1, COL6A3, FLT1, FLNC, HGF, IRS1, IRS2, ITGA4, ITGA8, and KDR), gene mutations of an IL-7 signaling pathway (e.g., mutations in one or more of genes HGF, IRS1, IRS2, and SMARCA4), or gene mutations of an SWI/SNF chromatin remodeling complex (e.g., mutations in one or more of genes SMARCA4, SMARCA2, and PBRM1);
further preferably, the non-small cell lung cancer is non-small cell lung cancer accompanied by mutations in one or more of genes RB1, KEAP1, and SMARCA4.

**3.** The use according to claim 2, wherein, the non-small cell lung cancer is squamous cell carcinoma or non-squamous cell carcinoma.

**4.** The use according to claim 2, wherein, the non-small cell lung cancer is non-small cell lung cancer with PD-L1 expression of ≥ 1% or PD-L1 expression of < 1% in immunohistochemical staining analysis of a tumor tissue section, preferably non-small cell lung cancer with PD-L1 expression of ≥ 50% in immunohistochemical staining analysis of a tumor tissue section.

**5.** The use according to any one of claims 1-4, wherein, the anti-PD-1 antibody or the antigen-binding fragment thereof comprises a light chain complementarity determining region with amino acid sequences set forth in SEQ ID NOs: 1, 2, and 3, respectively, and a heavy chain complementarity determining region with amino acid sequences set forth in SEQ ID NOs: 4, 5, and 6, respectively.

**6.** The use according to claim 5, wherein, the anti-PD-1 antibody or the antigen-binding fragment thereof comprises a light chain variable region with an amino acid sequence set forth in SEQ ID NO: 7 and a heavy chain variable region with an amino acid sequence set forth in SEQ ID NO: 8.

**7.** The use according to claim 6, wherein, the anti-PD-1 antibody comprises a light chain with an amino acid sequence set forth in SEQ ID NO: 9 and a heavy chain with an amino acid sequence set forth in SEQ ID NO: 10.

**8.** The use according to any one of claims 1-4, wherein, the anti-PD-1 antibody is selected from one or more of nivolumab, pembrolizumab, toripalimab, sintilimab, camrelizumab, tislelizumab, and cemiplimab, preferably toripalimab.

**9.** The use according to any one of claims 1-4, wherein, the first-line chemotherapeutic agent is selected from one or more of carboplatin, cisplatin, pemetrexed, and albumin-bound paclitaxel.

**10.** The use according to claim 9, wherein, the first-line chemotherapeutic agent is selected from a combination of carboplatin and albumin-bound paclitaxel and a combination of pemetrexed and carboplatin or cisplatin.

**11.** The use according to any one of claims 1-10, wherein, the non-small cell lung cancer is squamous cell carcinoma, and the combination comprises a combination I of the anti-PD-1 antibody or the antigen-binding fragment thereof, carboplatin, and albumin-bound paclitaxel, and optionally the anti-PD-1 antibody or the antigen-binding fragment thereof administered alone after the end of an administration cycle of the combination I; or the non-small cell lung cancer is non-squamous cell carcinoma, and the combination comprises a combination II of the anti-PD-1 antibody or the antigen-binding fragment thereof, pemetrexed, and carboplatin or cisplatin, and optionally a combination III of the anti-PD-1 antibody or the antigen-binding fragment thereof and pemetrexed administered after the end of an administration cycle of the combination II;
preferably, the non-small cell lung cancer is squamous cell carcinoma, and the combination comprises a combination A of toripalimab, carboplatin, and albumin-bound paclitaxel, and optionally toripalimab administered alone after the end of an administration cycle of the combination A; or the non-small cell lung cancer is non-squamous cell carcinoma, and the combination comprises a combination B of toripalimab, pemetrexed, and carboplatin or cisplatin, and optionally a combination C of toripalimab and pemetrexed administered after the end of an administration cycle of the combination B.

**12.** The use according to claim 11, wherein,

(I) the non-small cell lung cancer is squamous cell carcinoma, and in the combination I or the combination A,

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;
the albumin-bound paclitaxel is administered at a single dose of about 60 mg/m$^2$ body surface area to about 140 mg/m$^2$ body surface area, e.g., about 60 mg/m$^2$ body surface area, 80 mg/m$^2$ body surface area, 100 mg/m$^2$ body surface area, 120 mg/m$^2$ body surface area, or 140 mg/m$^2$ body surface area; and
the carboplatin is administered at a single dose of about AUC 5 to AUC 7, e.g., about AUC 5, AUC 6, or AUC 7; or

(II) the non-small cell lung cancer is non-squamous cell carcinoma, and in the combination II or the combination B,

the anti-PD-1 antibody or the antigen-binding fragment thereof is administered at a single dose of about 0.1 mg/kg body weight to about 10.0 mg/kg body weight, e.g., about 0.1 mg/kg body weight, 0.3 mg/kg body weight, 1 mg/kg body weight, 2 mg/kg body weight, 3 mg/kg body weight, 5 mg/kg body weight, or 10 mg/kg body weight, or selected from a fixed dose of about 120 mg to about 480 mg, e.g., a fixed dose of about 120 mg, 240 mg, 360 mg, or 480 mg, preferably a fixed dose of about 240 mg and about 360 mg;
the pemetrexed is administered at a single dose of about 200 mg/m$^2$ body surface area to about 800 mg/m$^2$ body surface area, e.g., about 400 mg/m$^2$ body surface area, 500 mg/m$^2$ body surface area, or 600 mg/m$^2$ body surface area; and
the cisplatin is administered at a single dose of about 60 mg/m$^2$ body surface area to about 90 mg/m$^2$ body surface area, e.g., about 70 mg/m$^2$ body surface area, 75 mg/m$^2$ body surface area, or 80 mg/m$^2$ body surface area; or the carboplatin is administered at a single dose of about AUC 5 to AUC 7, e.g., about AUC 5, AUC 6, or AUC 7.

**13.** The use according to claim 12, wherein,

(I) the non-small cell lung cancer is squamous cell carcinoma, and in the combination I or the combination A,

the anti-PD-1 antibody or the antigen-binding fragment thereof is administered at a frequency of about once every week, once every two weeks, once every three weeks, once every four weeks, or once a month, preferably once every three weeks;
the albumin-bound paclitaxel is administered at a frequency of about once every week, twice every three weeks, once every two weeks, once every three weeks, once every four weeks, or once a month, preferably once every week or twice every three weeks; and
the carboplatin is administered at a frequency of about once every week, once every two weeks, once every three weeks, once every four weeks, or once a month, preferably once every three weeks; or

(II) the non-small cell lung cancer is non-squamous cell carcinoma, and in the combination II or the combination B,

the anti-PD-1 antibody or the antigen-binding fragment thereof is administered at a frequency of about once every week, once every two weeks, once every three weeks, once every four weeks, or once a month, preferably once every three weeks;
the pemetrexed is administered at a frequency of about once every week, once every two weeks, once every three weeks, once every four weeks, or once a month, preferably once every three weeks; and
the cisplatin or carboplatin is administered at a frequency of about once every week, twice every three weeks, once every two weeks, once every three weeks, once every four weeks, or once a month, preferably once every three weeks.

**14.** The use according to claim 13, wherein,

(I) the non-small cell lung cancer is squamous cell carcinoma, and in the combination I or the combination A, the anti-PD-1 antibody or the antigen-binding fragment thereof is administered at a fixed dose of 240 mg or 360 mg once every three weeks; the albumin-bound paclitaxel is administered at a single dose of about 100 mg/m$^2$ body surface area once every week or twice every three weeks; and the carboplatin is administered at a single dose of about AUC 5 once every three weeks; or
(II) the non-small cell lung cancer is non-squamous cell carcinoma, and in the combination II or the combination

B, the anti-PD-1 antibody or the antigen-binding fragment thereof is administered at a fixed dose of 240 mg or 360 mg once every three weeks; the pemetrexed is administered at a single dose of about 500 mg/$m^2$ body surface area once every three weeks; and the cisplatin is administered at a single dose of about 75 mg/$m^2$ body surface area once every three weeks, or the carboplatin is administered at a single dose of about AUC 5 once every three weeks.

**15.** The use according to claim 14, wherein, the anti-PD-1 antibody or the antigen-binding fragment thereof, the albumin-bound paclitaxel, the carboplatin, the cisplatin, and the pemetrexed are administered in an administration cycle of one week, two weeks, three weeks, one month, two months, three months, four months, five months, half a year, or longer; optionally, the cycles are identical or different and at identical or different intervals.

**16.** The use according to claim 14, wherein, the anti-PD-1 antibody or the antigen-binding fragment thereof, the albumin-bound paclitaxel, the carboplatin, the cisplatin, and the pemetrexed are administered parenterally, e.g., by intravenous infusion, in a liquid dosage form, e.g., an injection.

**17.** A pharmaceutical combination comprising an anti-PD-1 antibody or an antigen-binding fragment thereof, albumin-bound paclitaxel, and carboplatin; or comprising an anti-PD-1 antibody or an antigen-binding fragment thereof, pemetrexed, and cisplatin or carboplatin.

**18.** The pharmaceutical combination according to claim 17, wherein,
the anti-PD-1 antibody or the antigen-binding fragment thereof comprises a light chain complementarity determining region with amino acid sequences set forth in SEQ ID NOs: 1, 2, and 3, respectively, and a heavy chain complementarity determining region with amino acid sequences set forth in SEQ ID NOs: 4, 5, and 6, respectively; preferably, the anti-PD-1 antibody or the antigen-binding fragment thereof comprises a light chain variable region with an amino acid sequence set forth in SEQ ID NO: 7 and a heavy chain variable region with an amino acid sequence set forth in SEQ ID NO: 8; preferably, the anti-PD-1 antibody comprises a light chain with an amino acid sequence set forth in SEQ ID NO: 9 and a heavy chain with an amino acid sequence set forth in SEQ ID NO: 10; and more preferably, the anti-PD-1 antibody is toripalimab.

**19.** A method for preventing or treating non-small cell lung cancer, comprising administering to an individual in need thereof a therapeutically effective amount of the anti-PD-1 antibody or the antigen-binding fragment thereof according to any one of claims 5-8, or the pharmaceutical combination according to claim 17 or 18.

**20.** A kit, comprising:

one or more single dosage units of an anti-PD-1 antibody or an antigen-binding fragment thereof, one or more single dosage units of albumin-bound paclitaxel, and one or more single dosage units of carboplatin, wherein the anti-PD-1 antibody or the antigen-binding fragment thereof is according to any one of claims 5-8; or
one or more single dosage units of an anti-PD-1 antibody or an antigen-binding fragment thereof, one or more single dosage units of pemetrexed, and one or more single dosage units of carboplatin or cisplatin, wherein the anti-PD-1 antibody or the antigen-binding fragment thereof is according to any one of claims 5-8; or
one or more single dosage units of the pharmaceutical combination according to claim 17 or 18.

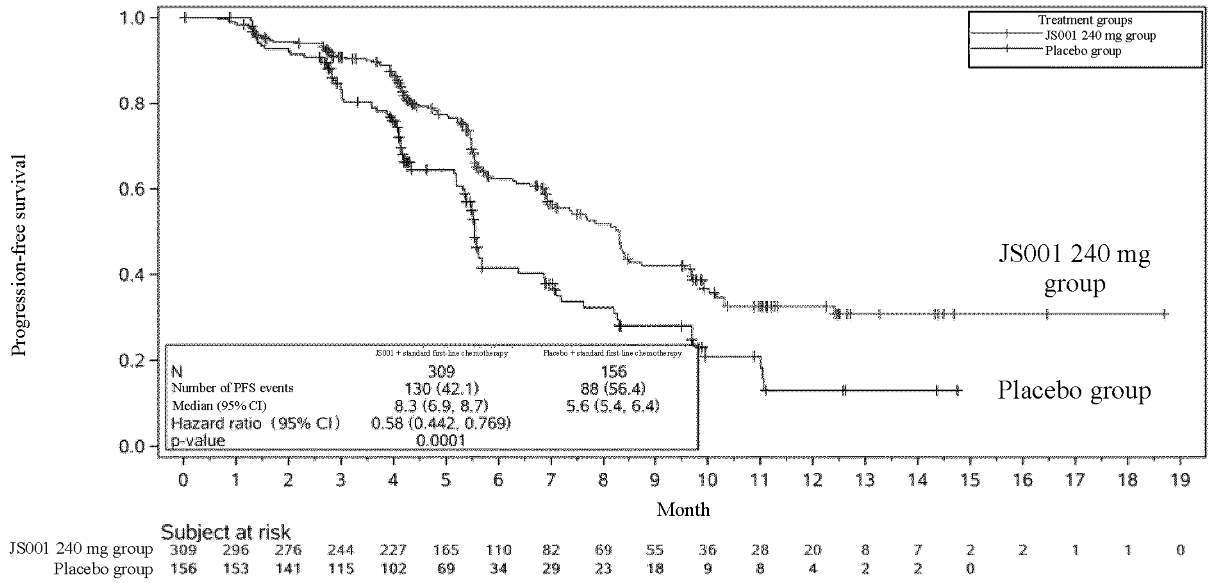
Treatment groups
JS001 240 mg group
Placebo group
Progression-free survival
Month
JS001 240 mg group
Placebo group
JS001 + standard first-line chemotherapy
Placebo + standard first-line chemotherapy
N 309 156
Number of PFS events 130 (42.1) 88 (56.4)
Median (95% CI) 8.3 (6.9, 8.7) 5.6 (5.4, 6.4)
Hazard ratio (95% CI) 0.58 (0.442, 0.769)
p-value 0.0001
Subject at risk
JS001 240 mg group 309 296 276 244 227 165 110 82 69 55 36 28 20 8 7 2 2 1 1 0
Placebo group 156 153 141 115 102 69 34 29 23 18 9 8 4 2 2 0

1a

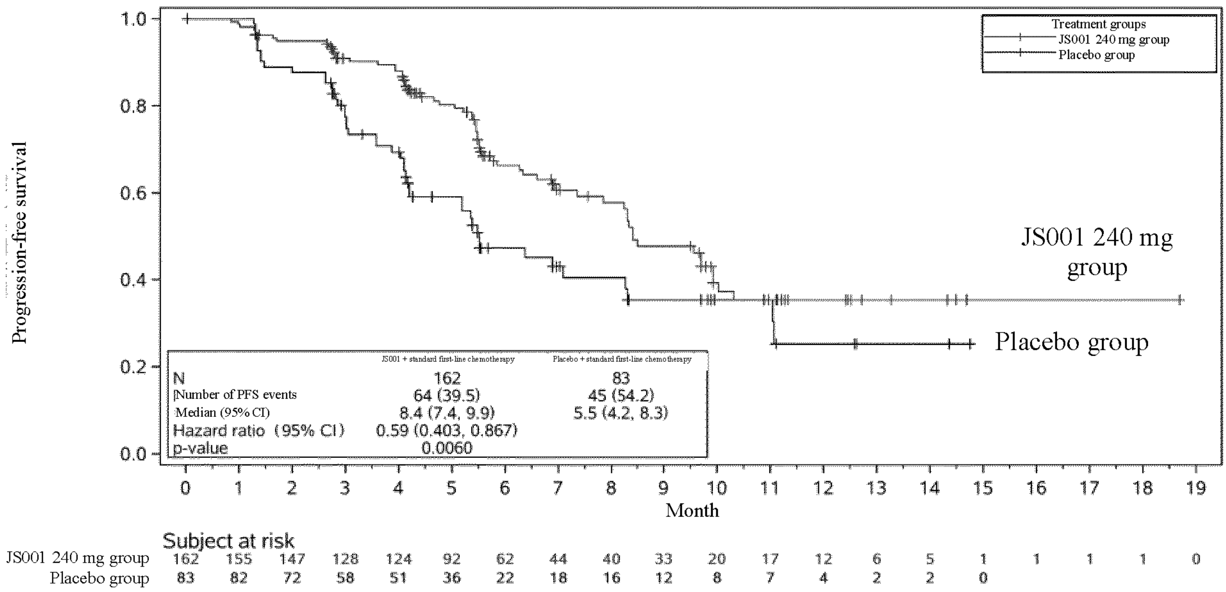
Treatment groups
JS001 240 mg group
Placebo group
Progression-free survival
Month
JS001 240 mg group
Placebo group
JS001 + standard first-line chemotherapy
Placebo + standard first-line chemotherapy
N 162 83
Number of PFS events 64 (39.5) 45 (54.2)
Median (95% CI) 8.4 (7.4, 9.9) 5.5 (4.2, 8.3)
Hazard ratio (95% CI) 0.59 (0.403, 0.867)
p-value 0.0060
Subject at risk
JS001 240 mg group 162 155 147 128 124 92 62 44 40 33 20 17 12 6 5 1 1 1 1 0
Placebo group 83 82 72 58 51 36 22 18 16 12 8 7 4 2 2 0

1b

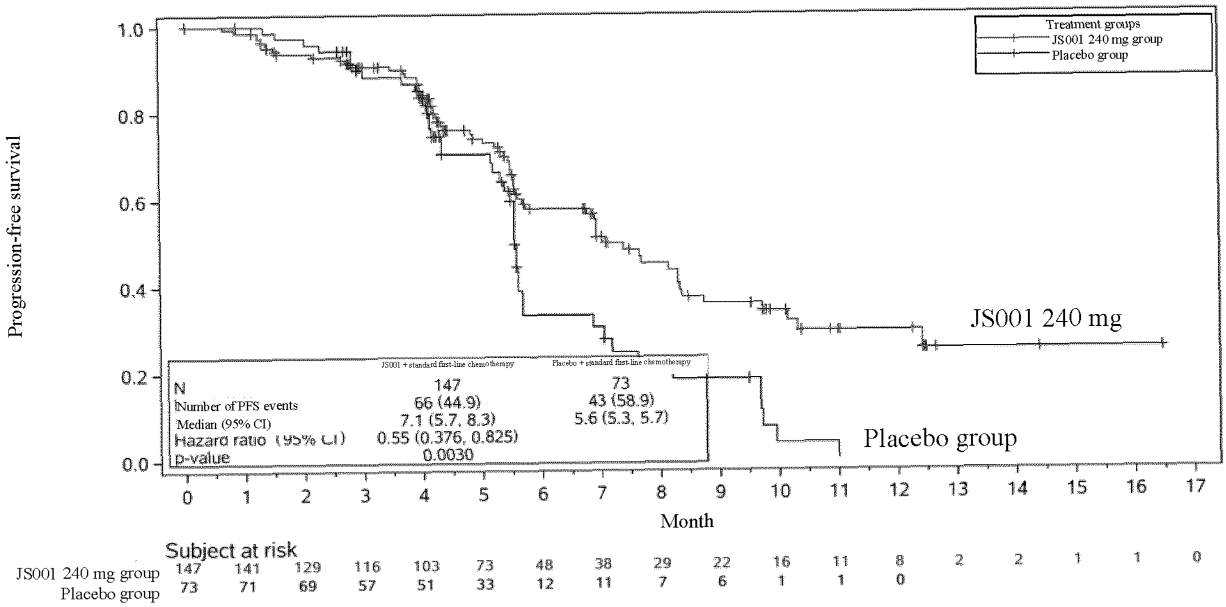
Treatment groups
JS001 240 mg group
Placebo group
Progression-free survival
1.0
0.8
0.6
0.4
0.2
0.0
JS001 240 mg
Placebo group
JS001 + standard first-line chemotherapy
Placebo + standard first-line chemotherapy
N 147 73
Number of PFS events 66 (44.9) 43 (58.9)
Median (95% CI) 7.1 (5.7, 8.3) 5.6 (5.3, 5.7)
Hazard ratio (95% CI) 0.55 (0.376, 0.825)
p-value 0.0030
0 1 2 3 4 5 6 7 8 9 10 11 12 13 14 15 16 17
Month
Subject at risk
JS001 240 mg group 147 141 129 116 103 73 48 38 29 22 16 11 8 2 2 1 1 0
Placebo group 73 71 69 57 51 33 12 11 7 6 1 1 0

1c

FIG. 1

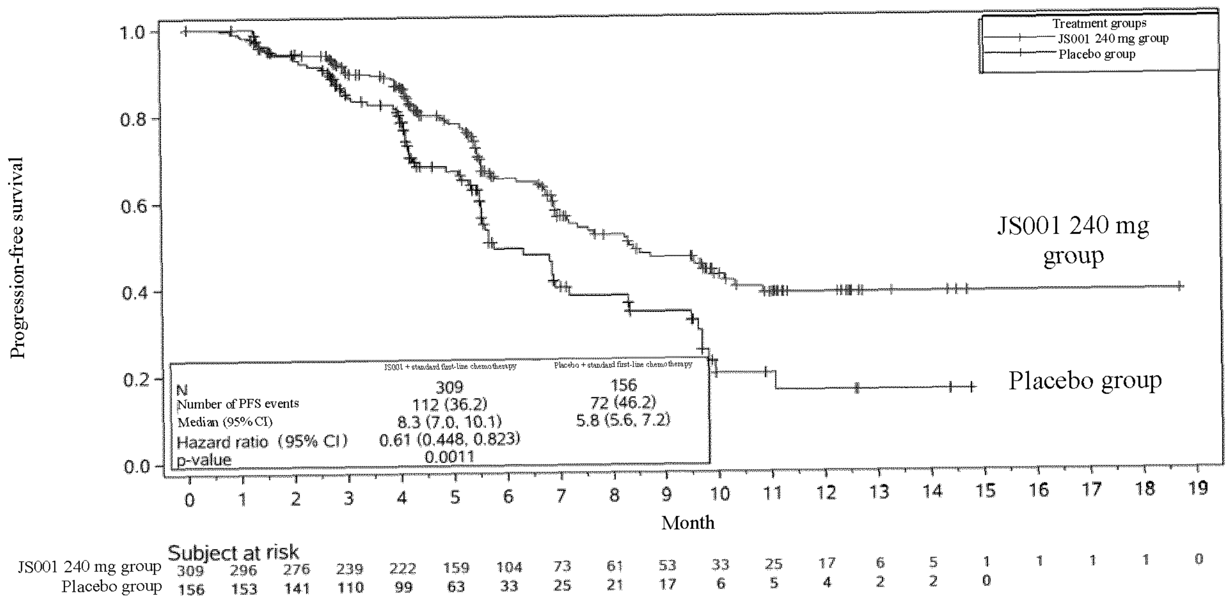
Treatment groups
JS001 240 mg group
Placebo group
Progression-free survival
1.0
0.8
0.6
0.4
0.2
0.0
JS001 240 mg group
Placebo group
JS001 + standard first-line chemotherapy
Placebo + standard first-line chemotherapy
N 309 156
Number of PFS events 112 (36.2) 72 (46.2)
Median (95% CI) 8.3 (7.0, 10.1) 5.8 (5.6, 7.2)
Hazard ratio (95% CI) 0.61 (0.448, 0.823)
p-value 0.0011
0 1 2 3 4 5 6 7 8 9 10 11 12 13 14 15 16 17 18 19
Month
Subject at risk
JS001 240 mg group 309 296 276 239 222 159 104 73 61 53 33 25 17 6 5 1 1 1 1 0
Placebo group 156 153 141 110 99 63 33 25 21 17 6 5 4 2 2 0

2a

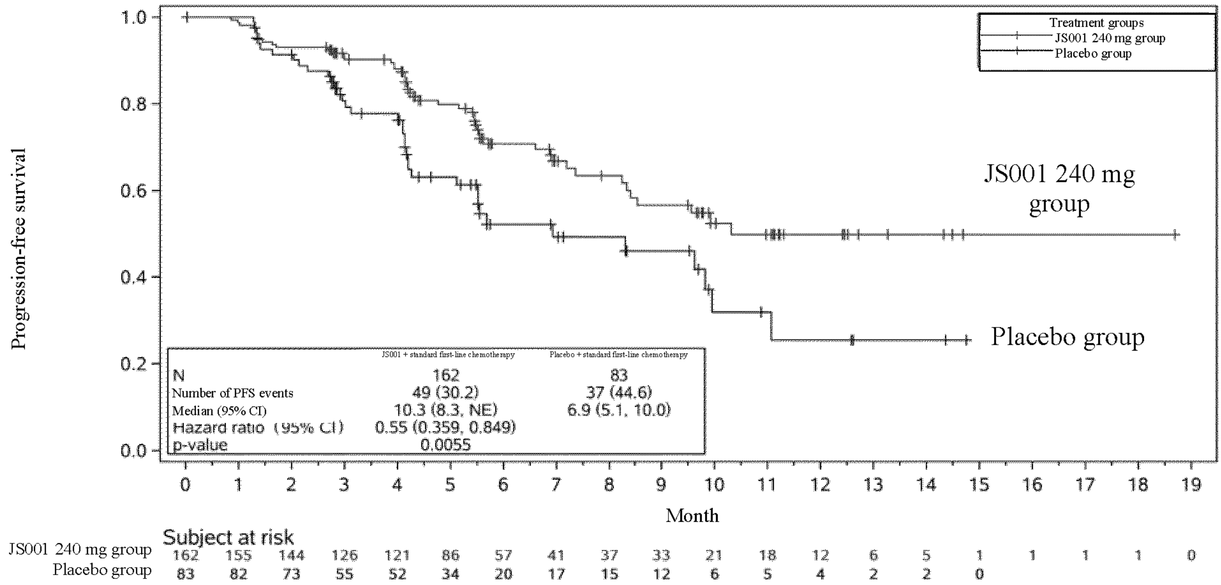
Treatment groups
JS001 240 mg group
Placebo group
JS001 240 mg group
Placebo group
Progression-free survival
1.0
0.8
0.6
0.4
0.2
0.0
JS001 + standard first-line chemotherapy
Placebo + standard first-line chemotherapy
N 162 83
Number of PFS events 49 (30.2) 37 (44.6)
Median (95% CI) 10.3 (8.3, NE) 6.9 (5.1, 10.0)
Hazard ratio (95% CI) 0.55 (0.359, 0.849)
p-value 0.0055
0 1 2 3 4 5 6 7 8 9 10 11 12 13 14 15 16 17 18 19
Month
Subject at risk
JS001 240 mg group 162 155 144 126 121 86 57 41 37 33 21 18 12 6 5 1 1 1 1 0
Placebo group 83 82 73 55 52 34 20 17 15 12 6 5 4 2 2 0

2b

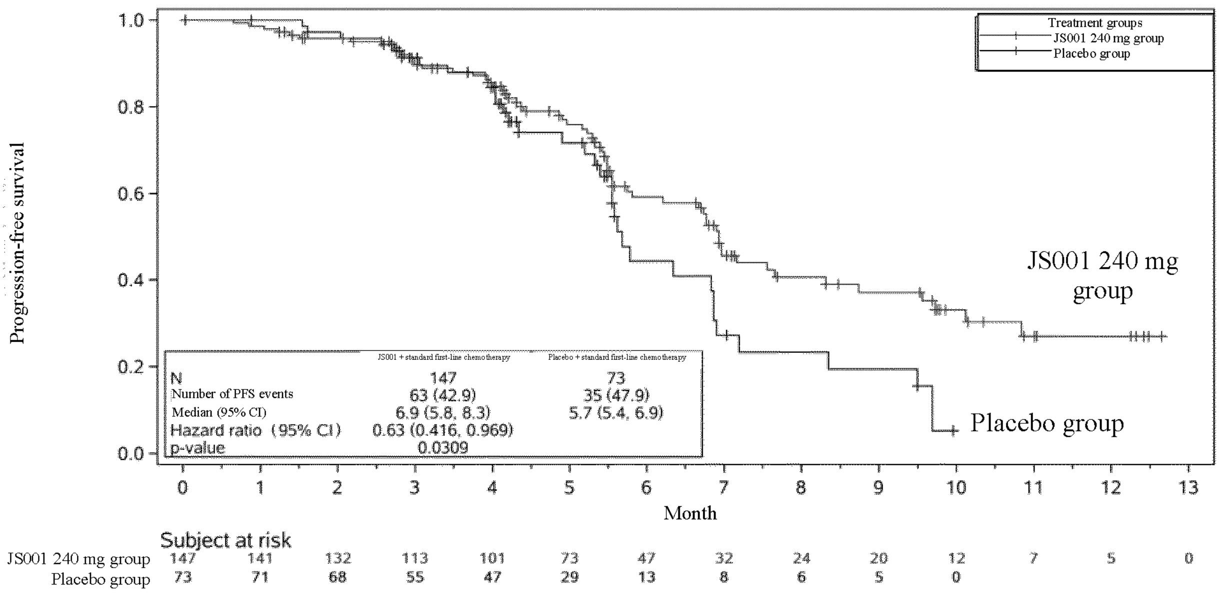
Treatment groups
JS001 240 mg group
Placebo group
JS001 240 mg group
Placebo group
Progression-free survival
1.0
0.8
0.6
0.4
0.2
0.0
JS001 + standard first-line chemotherapy
Placebo + standard first-line chemotherapy
N 147 73
Number of PFS events 63 (42.9) 35 (47.9)
Median (95% CI) 6.9 (5.8, 8.3) 5.7 (5.4, 6.9)
Hazard ratio (95% CI) 0.63 (0.416, 0.969)
p-value 0.0309
0 1 2 3 4 5 6 7 8 9 10 11 12 13
Month
Subject at risk
JS001 240 mg group 147 141 132 113 101 73 47 32 24 20 12 7 5 0
Placebo group 73 71 68 55 47 29 13 8 6 5 0

2c

FIG. 2

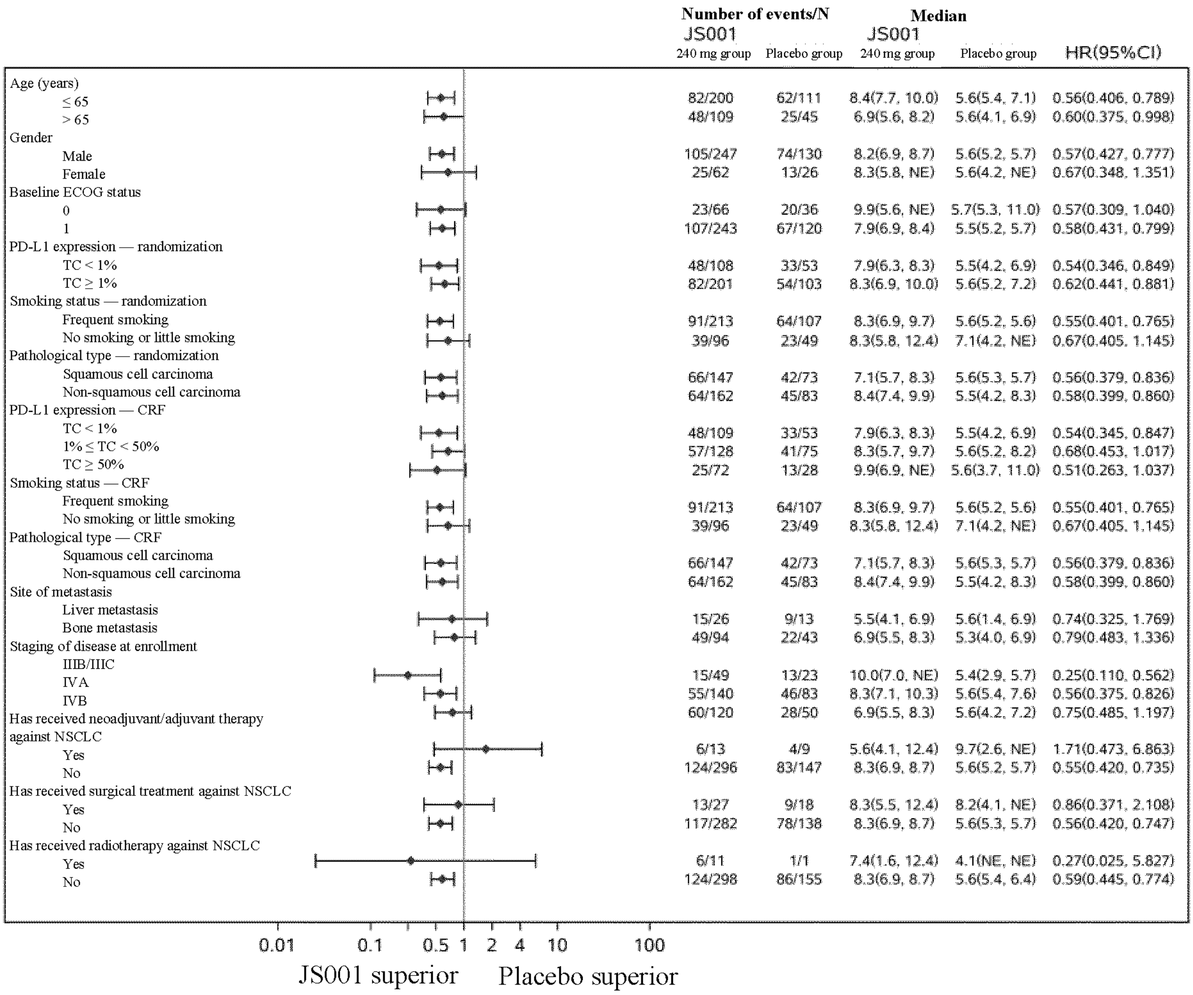
Number of events/N
Median
JS001 240 mg group
Placebo group
JS001 240 mg group
Placebo group
HR(95%CI)
Age (years)
≤ 65 82/200 62/111 8.4(7.7, 10.0) 5.6(5.4, 7.1) 0.56(0.406, 0.789)
> 65 48/109 25/45 6.9(5.6, 8.2) 5.6(4.1, 6.9) 0.60(0.375, 0.998)
Gender
Male 105/247 74/130 8.2(6.9, 8.7) 5.6(5.2, 5.7) 0.57(0.427, 0.777)
Female 25/62 13/26 8.3(5.8, NE) 5.6(4.2, NE) 0.67(0.348, 1.351)
Baseline ECOG status
0 23/66 20/36 9.9(5.6, NE) 5.7(5.3, 11.0) 0.57(0.309, 1.040)
1 107/243 67/120 7.9(6.9, 8.4) 5.5(5.2, 5.7) 0.58(0.431, 0.799)
PD-L1 expression — randomization
TC < 1% 48/108 33/53 7.9(6.3, 8.3) 5.5(4.2, 6.9) 0.54(0.346, 0.849)
TC ≥ 1% 82/201 54/103 8.3(6.9, 10.0) 5.6(5.2, 7.2) 0.62(0.441, 0.881)
Smoking status — randomization
Frequent smoking 91/213 64/107 8.3(6.9, 9.7) 5.6(5.2, 5.6) 0.55(0.401, 0.765)
No smoking or little smoking 39/96 23/49 8.3(5.8, 12.4) 7.1(4.2, NE) 0.67(0.405, 1.145)
Pathological type — randomization
Squamous cell carcinoma 66/147 42/73 7.1(5.7, 8.3) 5.6(5.3, 5.7) 0.56(0.379, 0.836)
Non-squamous cell carcinoma 64/162 45/83 8.4(7.4, 9.9) 5.5(4.2, 8.3) 0.58(0.399, 0.860)
PD-L1 expression — CRF
TC < 1% 48/109 33/53 7.9(6.3, 8.3) 5.5(4.2, 6.9) 0.54(0.345, 0.847)
1% ≤ TC < 50% 57/128 41/75 8.3(5.7, 9.7) 5.6(5.2, 8.2) 0.68(0.453, 1.017)
TC ≥ 50% 25/72 13/28 9.9(6.9, NE) 5.6(3.7, 11.0) 0.51(0.263, 1.037)
Smoking status — CRF
Frequent smoking 91/213 64/107 8.3(6.9, 9.7) 5.6(5.2, 5.6) 0.55(0.401, 0.765)
No smoking or little smoking 39/96 23/49 8.3(5.8, 12.4) 7.1(4.2, NE) 0.67(0.405, 1.145)
Pathological type — CRF
Squamous cell carcinoma 66/147 42/73 7.1(5.7, 8.3) 5.6(5.3, 5.7) 0.56(0.379, 0.836)
Non-squamous cell carcinoma 64/162 45/83 8.4(7.4, 9.9) 5.5(4.2, 8.3) 0.58(0.399, 0.860)
Site of metastasis
Liver metastasis 15/26 9/13 5.5(4.1, 6.9) 5.6(1.4, 6.9) 0.74(0.325, 1.769)
Bone metastasis 49/94 22/43 6.9(5.5, 8.3) 5.3(4.0, 6.9) 0.79(0.483, 1.336)
Staging of disease at enrollment
IIIB/IIIC 15/49 13/23 10.0(7.0, NE) 5.4(2.9, 5.7) 0.25(0.110, 0.562)
IVA 55/140 46/83 8.3(7.1, 10.3) 5.6(5.4, 7.6) 0.56(0.375, 0.826)
IVB 60/120 28/50 6.9(5.5, 8.3) 5.6(4.2, 7.2) 0.75(0.485, 1.197)
Has received neoadjuvant/adjuvant therapy against NSCLC
Yes 6/13 4/9 5.6(4.1, 12.4) 9.7(2.6, NE) 1.71(0.473, 6.863)
No 124/296 83/147 8.3(6.9, 8.7) 5.6(5.2, 5.7) 0.55(0.420, 0.735)
Has received surgical treatment against NSCLC
Yes 13/27 9/18 8.3(5.5, 12.4) 8.2(4.1, NE) 0.86(0.371, 2.108)
No 117/282 78/138 8.3(6.9, 8.7) 5.6(5.3, 5.7) 0.56(0.420, 0.747)
Has received radiotherapy against NSCLC
Yes 6/11 1/1 7.4(1.6, 12.4) 4.1(NE, NE) 0.27(0.025, 5.827)
No 124/298 86/155 8.3(6.9, 8.7) 5.6(5.4, 6.4) 0.59(0.445, 0.774)
0.01 0.1 0.5 1 2 4 10 100
JS001 superior
Placebo superior

FIG. 3

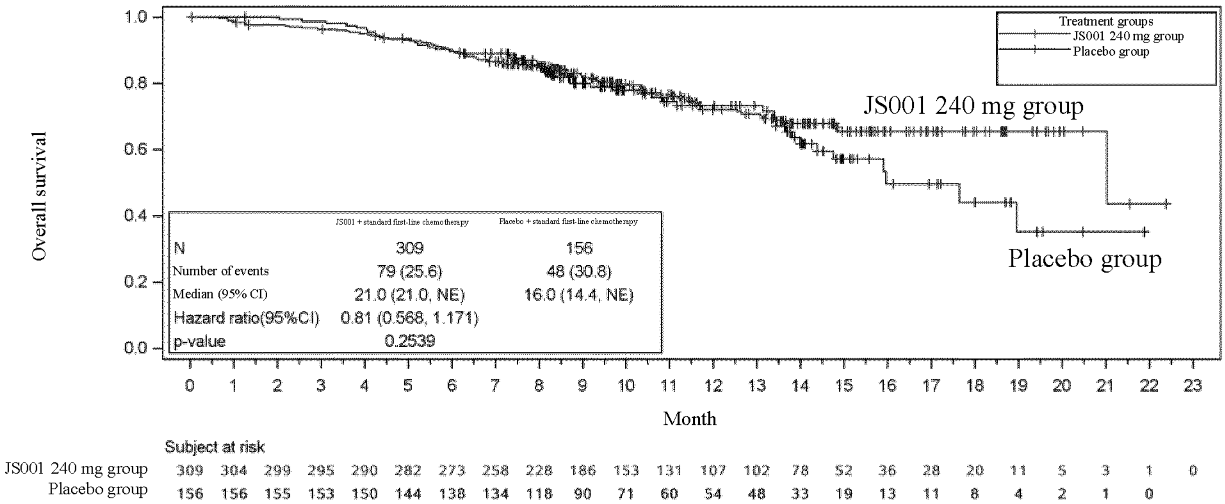
Treatment groups
JS001 240 mg group
Placebo group
JS001 240 mg group
Placebo group
Overall survival
1.0
0.8
0.6
0.4
0.2
0.0
JS001 + standard first-line chemotherapy
Placebo + standard first-line chemotherapy
N 309 156
Number of events 79 (25.6) 48 (30.8)
Median (95% CI) 21.0 (21.0, NE) 16.0 (14.4, NE)
Hazard ratio(95%CI) 0.81 (0.568, 1.171)
p-value 0.2539
0 1 2 3 4 5 6 7 8 9 10 11 12 13 14 15 16 17 18 19 20 21 22 23
Month
Subject at risk
JS001 240 mg group 309 304 299 295 290 282 273 258 228 186 153 131 107 102 78 52 36 28 20 11 5 3 1 0
Placebo group 156 156 155 153 150 144 138 134 118 90 71 60 54 48 33 19 13 11 8 4 2 1 0

FIG. 4

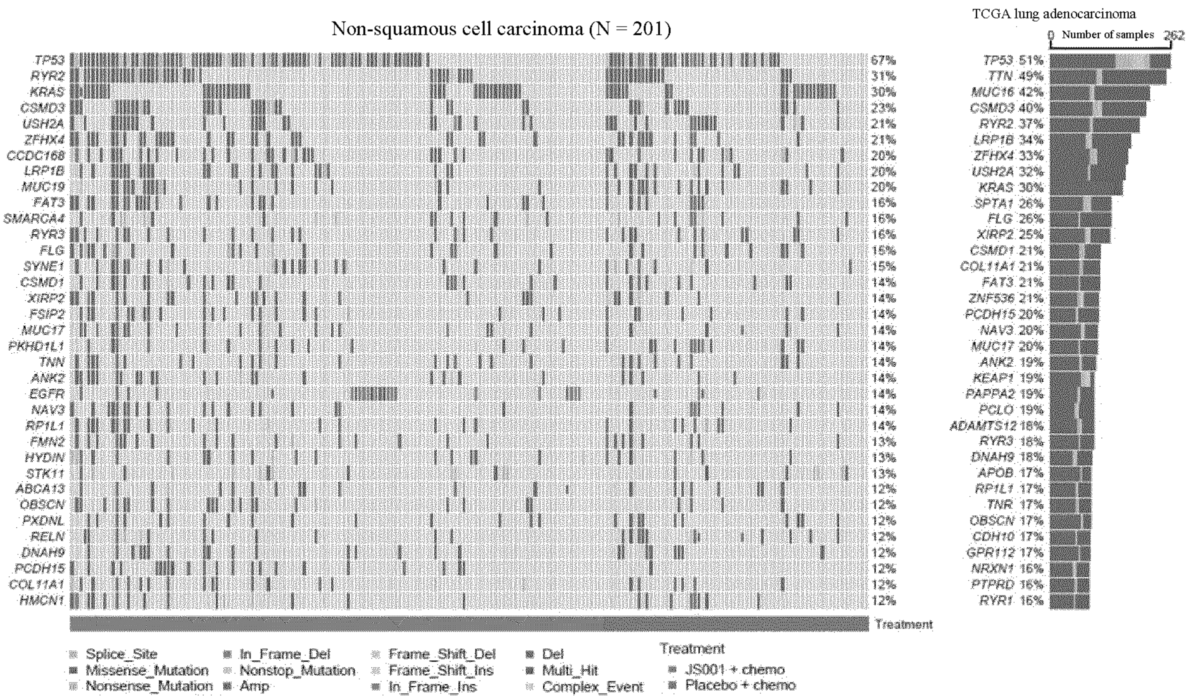
Non-squamous cell carcinoma (N = 201)
TP53 67%
RYR2 31%
KRAS 30%
CSMD3 23%
USH2A 21%
ZFHX4 21%
CCDC168 20%
LRP1B 20%
MUC19 20%
FAT3 16%
SMARCA4 16%
RYR3 16%
FLG 15%
SYNE1 15%
CSMD1 14%
XIRP2 14%
FSIP2 14%
MUC17 14%
PKHD1L1 14%
TNN 14%
ANK2 14%
EGFR 14%
NAV3 14%
RP1L1 14%
FMN2 13%
HYDIN 13%
STK11 13%
ABCA13 12%
OBSCN 12%
PXDNL 12%
RELN 12%
DNAH9 12%
PCDH15 12%
COL11A1 12%
HMCN1 12%
Treatment
TCGA lung adenocarcinoma
0 Number of samples 262
TP53 51%
TTN 49%
MUC16 42%
CSMD3 40%
RYR2 37%
LRP1B 34%
ZFHX4 33%
USH2A 32%
KRAS 30%
SPTA1 26%
FLG 26%
XIRP2 25%
CSMD1 21%
COL11A1 21%
FAT3 21%
ZNF536 21%
PCDH15 20%
NAV3 20%
MUC17 20%
ANK2 19%
KEAP1 19%
PAPPA2 19%
PCLO 19%
ADAMTS12 18%
RYR3 18%
DNAH9 18%
APOB 17%
RP1L1 17%
TNR 17%
OBSCN 17%
CDH10 17%
GPR112 17%
NRXN1 16%
PTPRD 16%
RYR1 16%
Splice_Site
Missense_Mutation
Nonsense_Mutation
In_Frame_Del
Nonstop_Mutation
Amp
Frame_Shift_Del
Frame_Shift_Ins
In_Frame_Ins
Del
Multi_Hit
Complex_Event
Treatment
JS001 + chemo
Placebo + chemo

FIG. 5a

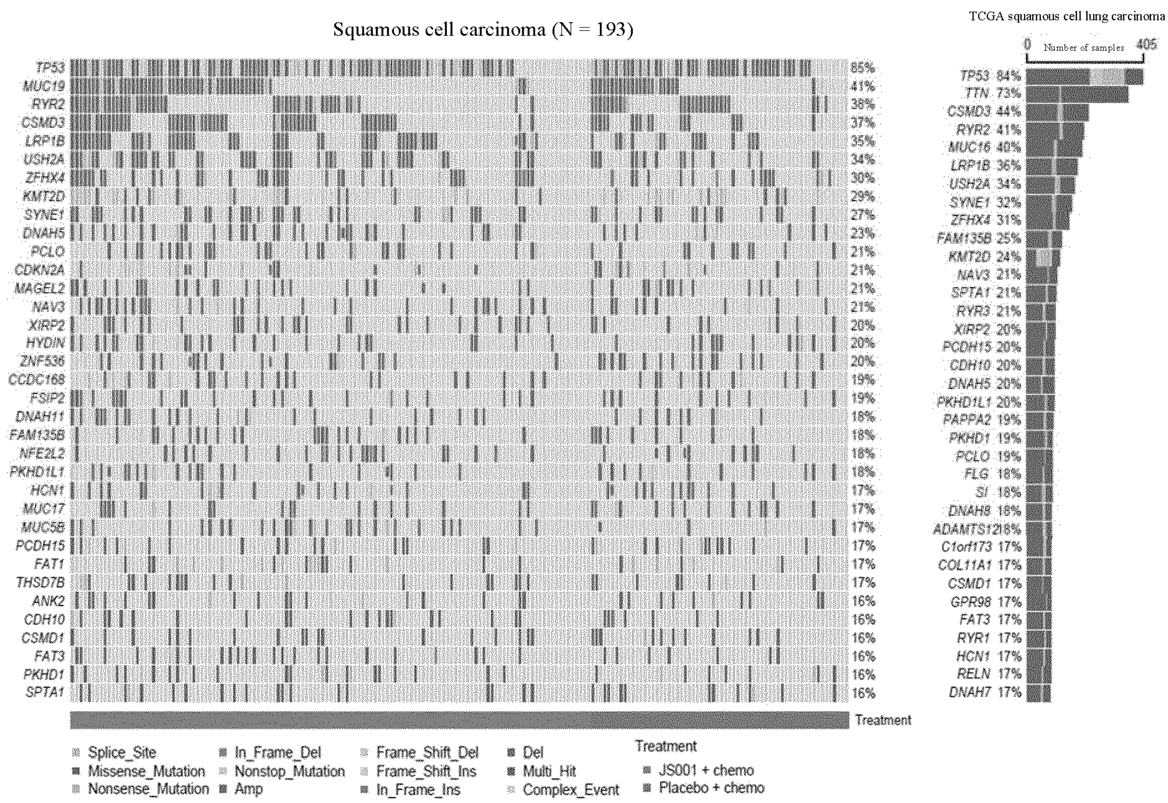
Squamous cell carcinoma (N = 193)
TP53 85%
MUC19 41%
RYR2 38%
CSMD3 37%
LRP1B 35%
USH2A 34%
ZFHX4 30%
KMT2D 29%
SYNE1 27%
DNAH5 23%
PCLO 21%
CDKN2A 21%
MAGEL2 21%
NAV3 21%
XIRP2 20%
HYDIN 20%
ZNF536 20%
CCDC168 19%
FSIP2 19%
DNAH11 18%
FAM135B 18%
NFE2L2 18%
PKHD1L1 18%
HCN1 17%
MUC17 17%
MUC5B 17%
PCDH15 17%
FAT1 17%
THSD7B 17%
ANK2 16%
CDH10 16%
CSMD1 16%
FAT3 16%
PKHD1 16%
SPTA1 16%
Treatment
TCGA squamous cell lung carcinoma
0 Number of samples 405
TP53 84%
TTN 73%
CSMD3 44%
RYR2 41%
MUC16 40%
LRP1B 36%
USH2A 34%
SYNE1 32%
ZFHX4 31%
FAM135B 25%
KMT2D 24%
NAV3 21%
SPTA1 21%
RYR3 21%
XIRP2 20%
PCDH15 20%
CDH10 20%
DNAH5 20%
PKHD1L1 20%
PAPPA2 19%
PKHD1 19%
PCLO 19%
FLG 18%
SI 18%
DNAH8 18%
ADAMTS12 18%
C1orf173 17%
COL11A1 17%
CSMD1 17%
GPR98 17%
FAT3 17%
RYR1 17%
HCN1 17%
RELN 17%
DNAH7 17%
Splice_Site
Missense_Mutation
Nonsense_Mutation
In_Frame_Del
Nonstop_Mutation
Amp
Frame_Shift_Del
Frame_Shift_Ins
In_Frame_Ins
Del
Multi_Hit
Complex_Event
Treatment
JS001 + chemo
Placebo + chemo

FIG. 5b

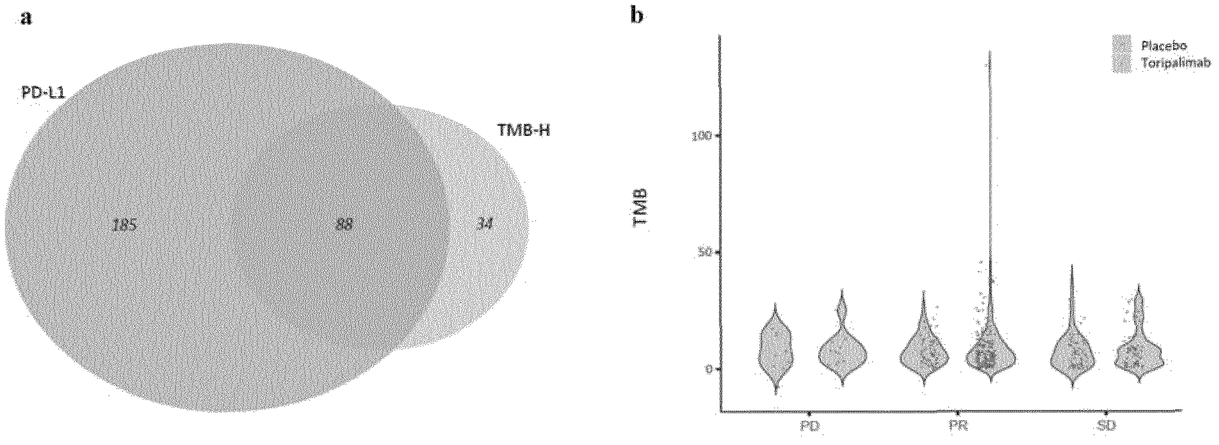
a
PD-L1
TMB-H
185
88
34
b
Placebo
Toripalimab
TMB
100
50
0
PD
PR
SD

FIG. 6

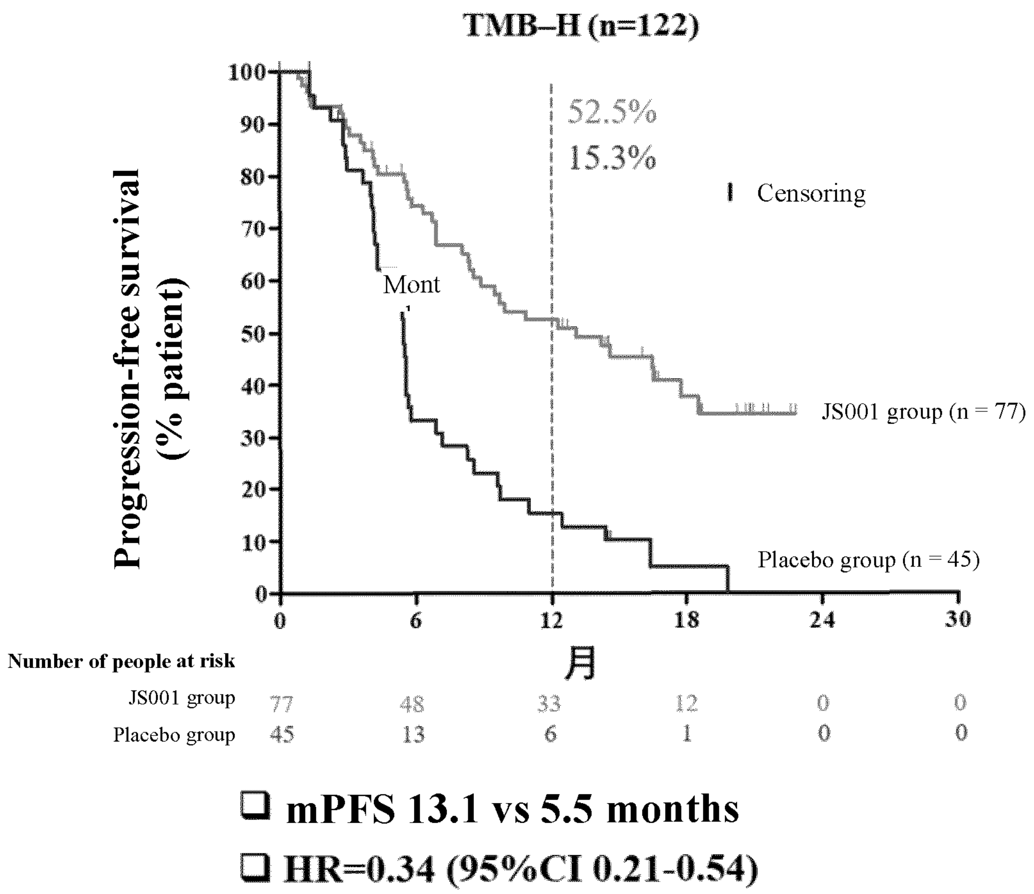
TMB–H (n=122)
Progression-free survival (% patient)
100
90
80
70
60
50
40
30
20
10
0
52.5%
15.3%
Censoring
Mont
JS001 group (n = 77)
Placebo group (n = 45)
0
6
12
18
24
30
月
Number of people at risk
JS001 group 77 48 33 12 0 0
Placebo group 45 13 6 1 0 0
❑ mPFS 13.1 vs 5.5 months
❑ HR=0.34 (95%CI 0.21-0.54)

FIG. 7a

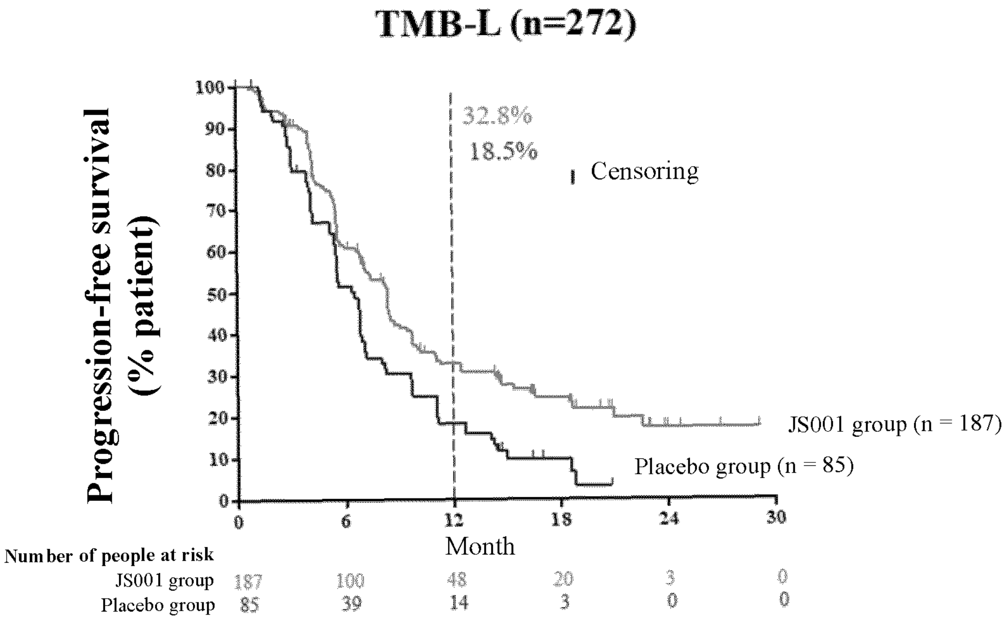


❑ **mPFS 8.3 vs 6.5 months**

❑ **HR=0.62 (95%CI 0.46-0.83)**

FIG. 7b

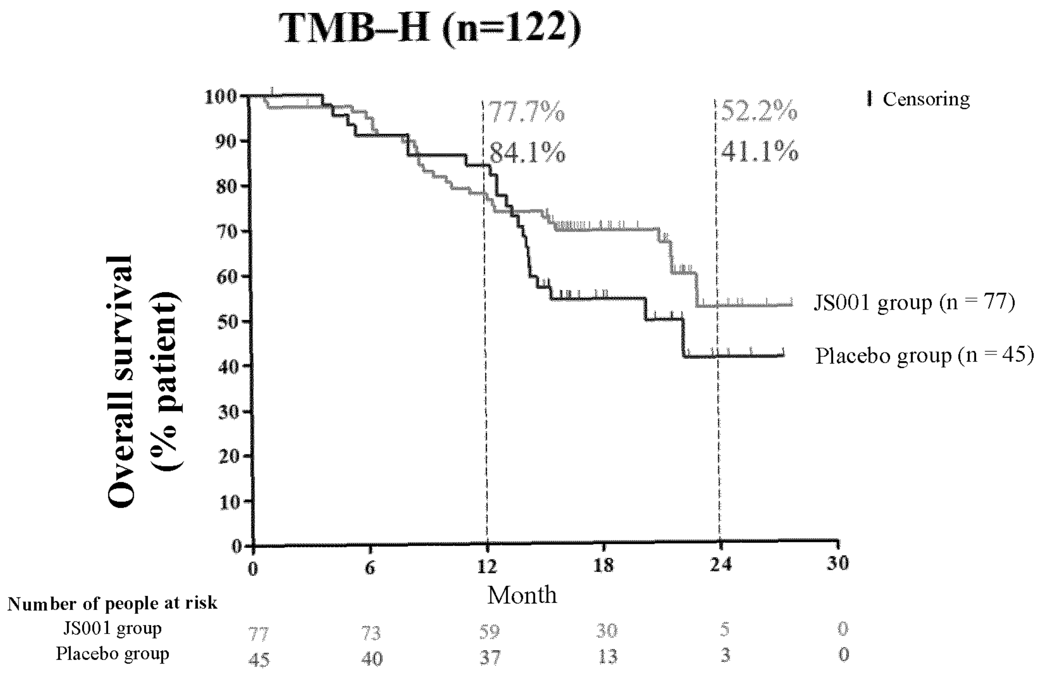


❑ **mOS NE vs 20.3 months**

❑ **HR=0.67 (95%CI 0.38-1.19)**

FIG. 8a

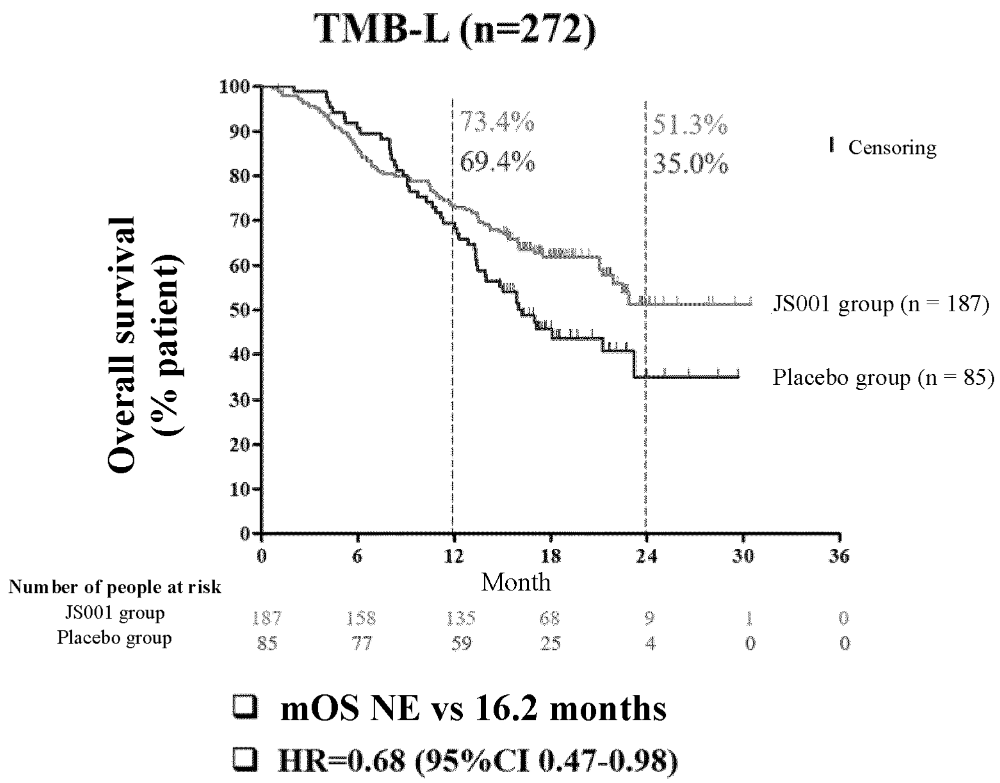


Figure 8b

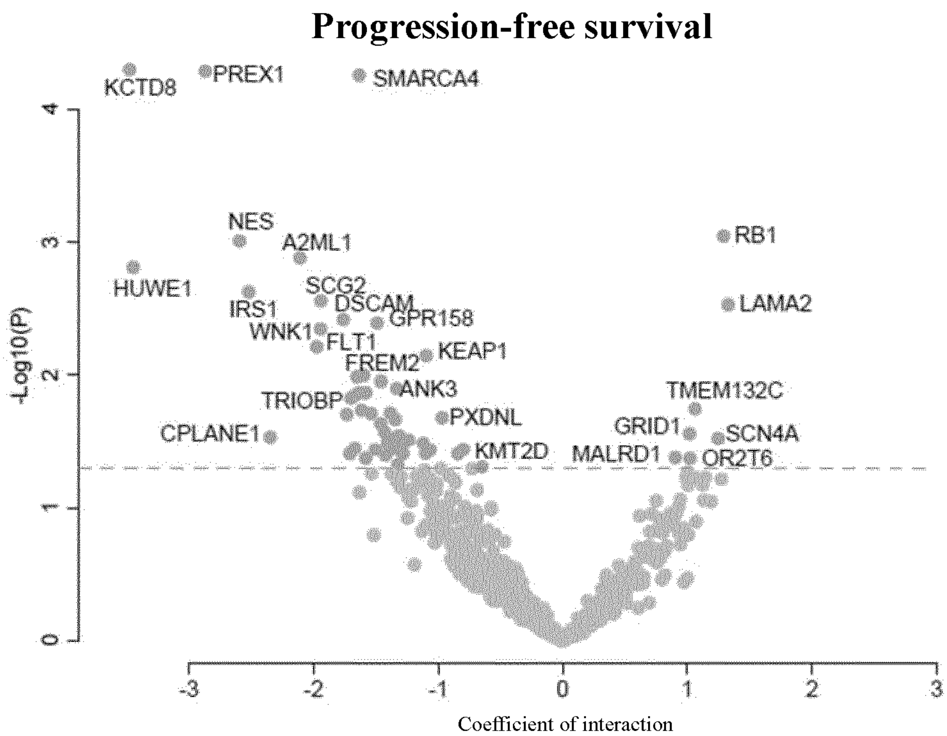


FIG. 9a

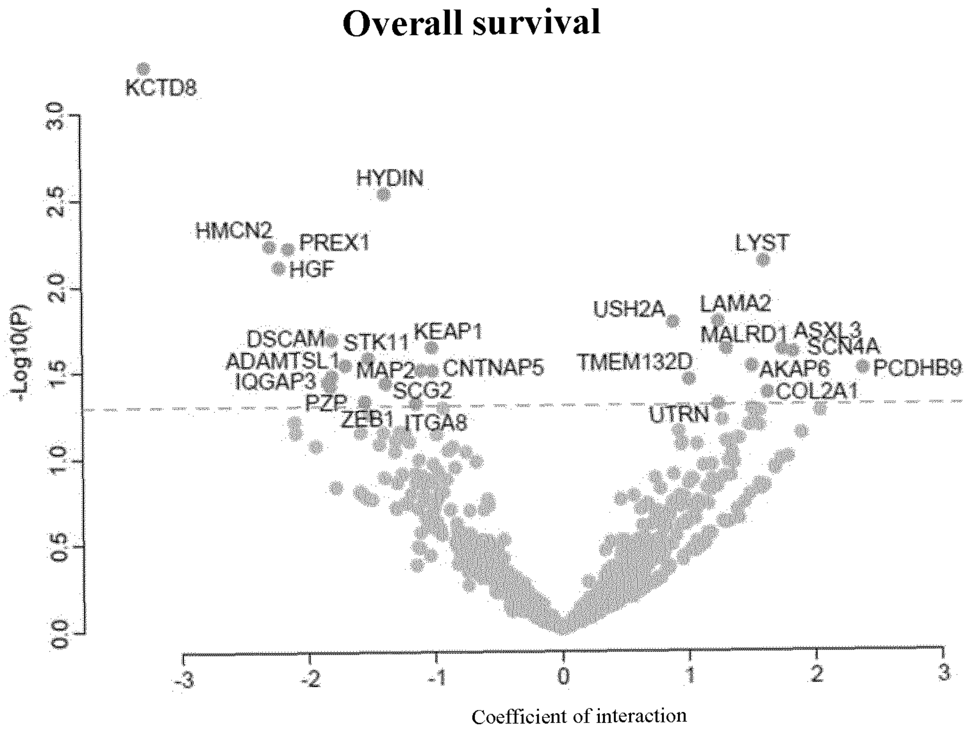
Overall survival
KCTD8
HYDIN
HMCN2
PREX1
HGF
LYST
USH2A
LAMA2
DSCAM
STK11
KEAP1
MALRD1
ASXL3
SCN4A
ADAMTSL1
MAP2
CNTNAP5
TMEM132D
AKAP6
PCDHB9
IQGAP3
SCG2
COL2A1
PZP
ZEB1
ITGA8
UTRN
-Log10(P)
0.0
0.5
1.0
1.5
2.0
2.5
3.0
-3
-2
-1
0
1
2
3
Coefficient of interaction

FIG. 9b

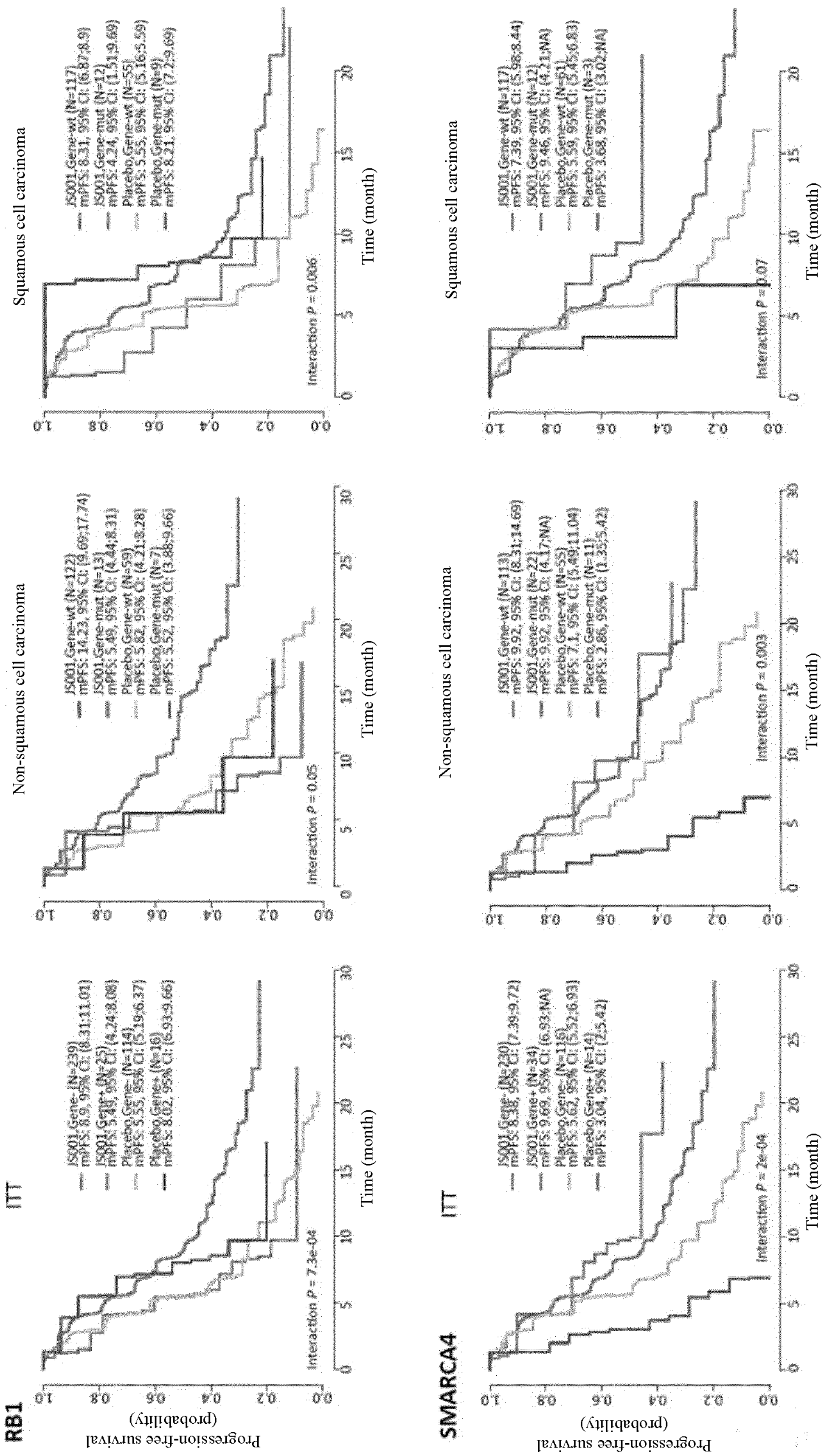
RB1
ITT
JS001,Gene- (N=239)
mPFS: 8.9, 95% CI: (8.31;11.01)
JS001,Gene+ (N=25)
mPFS: 5.49, 95% CI: (4.24;8.08)
Placebo,Gene- (N=114)
mPFS: 5.55, 95% CI: (5.19;6.37)
Placebo,Gene+ (N=16)
mPFS: 8.02, 95% CI: (6.93;9.66)
Interaction P = 7.3e-04
Non-squamous cell carcinoma
JS001,Gene-wt (N=122)
mPFS: 14.23, 95% CI: (9.69;17.74)
JS001,Gene-mut (N=13)
mPFS: 5.49, 95% CI: (4.44;8.31)
Placebo,Gene-wt (N=59)
mPFS: 5.82, 95% CI: (4.21;8.28)
Placebo,Gene-mut (N=7)
mPFS: 5.52, 95% CI: (3.88;9.66)
Interaction P = 0.05
Squamous cell carcinoma
JS001,Gene-wt (N=117)
mPFS: 8.31, 95% CI: (6.87;8.9)
JS001,Gene-mut (N=12)
mPFS: 4.24, 95% CI: (1.51;9.69)
Placebo,Gene-wt (N=55)
mPFS: 5.55, 95% CI: (5.16;5.59)
Placebo,Gene-mut (N=9)
mPFS: 8.21, 95% CI: (7.2;9.69)
Interaction P = 0.006
SMARCA4
ITT
JS001,Gene- (N=230)
mPFS: 8.38, 95% CI: (7.39;9.72)
JS001,Gene+ (N=34)
mPFS: 9.69, 95% CI: (6.93;NA)
Placebo,Gene- (N=116)
mPFS: 5.62, 95% CI: (5.52;6.93)
Placebo,Gene+ (N=14)
mPFS: 3.04, 95% CI: (2;5.42)
Interaction P = 2e-04
Non-squamous cell carcinoma
JS001,Gene-wt (N=113)
mPFS: 9.92, 95% CI: (8.31;14.69)
JS001,Gene-mut (N=22)
mPFS: 9.92, 95% CI: (4.17;NA)
Placebo,Gene-wt (N=55)
mPFS: 7.1, 95% CI: (5.49;11.04)
Placebo,Gene-mut (N=11)
mPFS: 2.86, 95% CI: (1.35;5.42)
Interaction P = 0.003
Squamous cell carcinoma
JS001,Gene-wt (N=117)
mPFS: 7.39, 95% CI: (5.98;8.44)
JS001,Gene-mut (N=12)
mPFS: 9.46, 95% CI: (4.21;NA)
Placebo,Gene-wt (N=61)
mPFS: 5.59, 95% CI: (5.45;6.83)
Placebo,Gene-mut (N=3)
mPFS: 3.68, 95% CI: (3.02;NA)
Interaction P = 0.07
Progression-free survival (probability)
Time (month)

FIG. 10

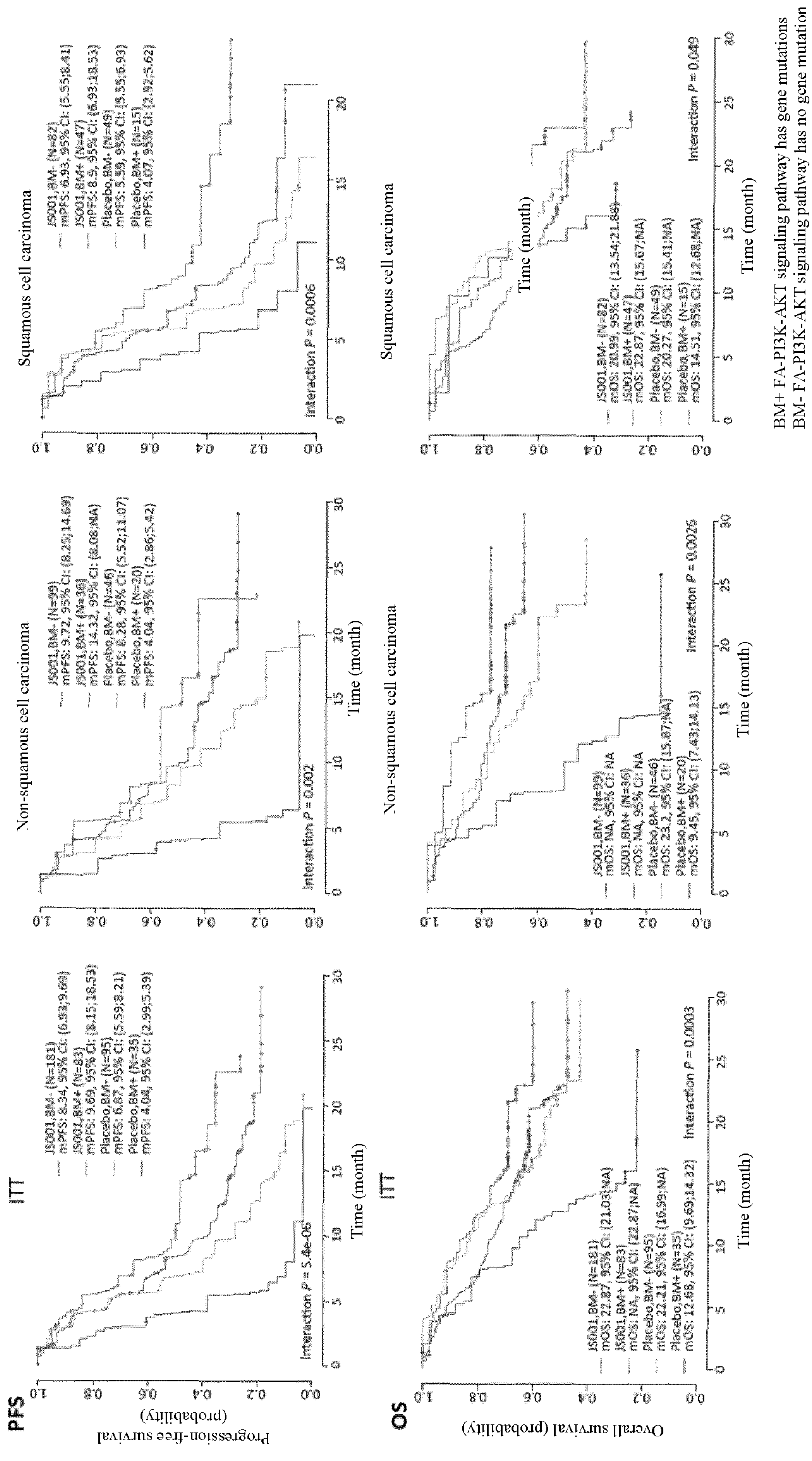
PFS
ITT
Progression-free survival (probability)
JS001,BM- (N=181) mPFS: 8.34, 95% CI: (6.93;9.69)
JS001,BM+ (N=83) mPFS: 9.69, 95% CI: (8.15;18.53)
Placebo,BM- (N=95) mPFS: 6.87, 95% CI: (5.59;8.21)
Placebo,BM+ (N=35) mPFS: 4.04, 95% CI: (2.99;5.39)
Interaction P = 5.4e-06
Time (month)
Non-squamous cell carcinoma
JS001,BM- (N=99) mPFS: 9.72, 95% CI: (8.25;14.69)
JS001,BM+ (N=36) mPFS: 14.32, 95% CI: (8.08;NA)
Placebo,BM- (N=46) mPFS: 8.28, 95% CI: (5.52;11.07)
Placebo,BM+ (N=20) mPFS: 4.04, 95% CI: (2.86;5.42)
Interaction P = 0.002
Squamous cell carcinoma
JS001,BM- (N=82) mPFS: 6.93, 95% CI: (5.55;8.41)
JS001,BM+ (N=47) mPFS: 8.9, 95% CI: (6.93;18.53)
Placebo,BM- (N=49) mPFS: 5.59, 95% CI: (5.55;6.93)
Placebo,BM+ (N=15) mPFS: 4.07, 95% CI: (2.92;5.62)
Interaction P = 0.0006
OS
ITT
Overall survival (probability)
JS001,BM- (N=181) mOS: 22.87, 95% CI: (21.03;NA)
JS001,BM+ (N=83) mOS: NA, 95% CI: (22.87;NA)
Placebo,BM- (N=95) mOS: 22.21, 95% CI: (16.99;NA)
Placebo,BM+ (N=35) mOS: 12.68, 95% CI: (9.69;14.32)
Interaction P = 0.0003
Non-squamous cell carcinoma
JS001,BM- (N=99) mOS: NA, 95% CI: NA
JS001,BM+ (N=36) mOS: NA, 95% CI: NA
Placebo,BM- (N=46) mOS: 23.2, 95% CI: (15.87;NA)
Placebo,BM+ (N=20) mOS: 9.45, 95% CI: (7.43;14.13)
Interaction P = 0.0026
Squamous cell carcinoma
JS001,BM- (N=82) mOS: 20.99, 95% CI: (13.54;21.88)
JS001,BM+ (N=47) mOS: 22.87, 95% CI: (15.67;NA)
Placebo,BM- (N=49) mOS: 20.27, 95% CI: (15.41;NA)
Placebo,BM+ (N=15) mOS: 14.51, 95% CI: (12.68;NA)
Interaction P = 0.049
BM+ FA-PI3K-AKT signaling pathway has gene mutations
BM- FA-PI3K-AKT signaling pathway has no gene mutation

FIG. 11

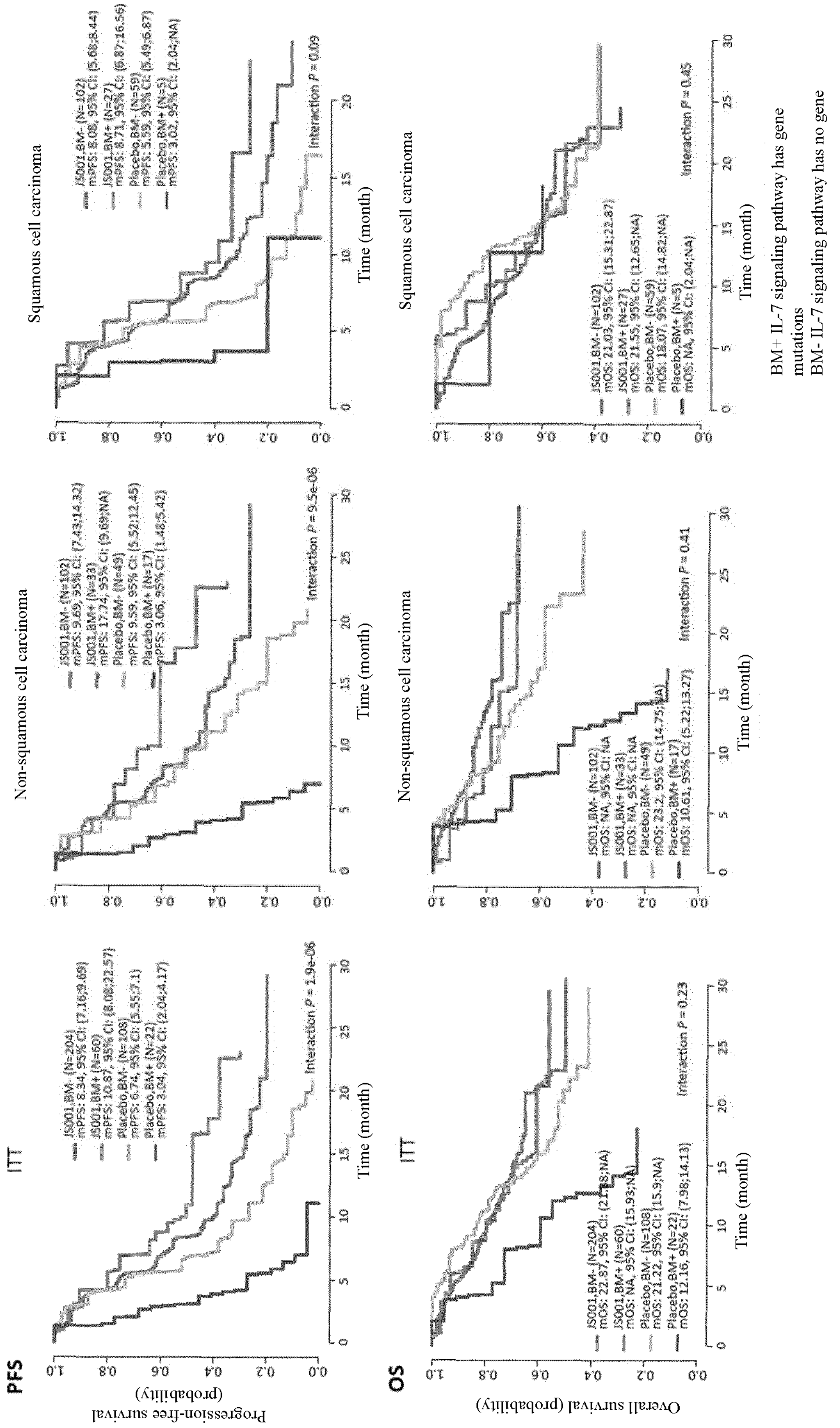
PFS
OS
ITT
Non-squamous cell carcinoma
Squamous cell carcinoma
Progression-free survival (probability)
Overall survival (probability)
Time (month)
JS001,BM- (N=204)
mPFS: 8.34, 95% CI: (7.16;9.69)
JS001,BM+ (N=60)
mPFS: 10.87, 95% CI: (8.08;22.57)
Placebo,BM- (N=108)
mPFS: 6.74, 95% CI: (5.55;7.1)
Placebo,BM+ (N=22)
mPFS: 3.04, 95% CI: (2.04;4.17)
Interaction P = 1.9e-06
JS001,BM- (N=102)
mPFS: 9.69, 95% CI: (7.43;14.32)
JS001,BM+ (N=33)
mPFS: 17.74, 95% CI: (9.69;NA)
Placebo,BM- (N=49)
mPFS: 9.59, 95% CI: (5.52;12.45)
Placebo,BM+ (N=17)
mPFS: 3.06, 95% CI: (1.48;5.42)
Interaction P = 9.5e-06
JS001,BM- (N=102)
mPFS: 8.08, 95% CI: (5.68;8.44)
JS001,BM+ (N=27)
mPFS: 8.71, 95% CI: (6.87;16.56)
Placebo,BM- (N=59)
mPFS: 5.59, 95% CI: (5.49;6.87)
Placebo,BM+ (N=5)
mPFS: 3.02, 95% CI: (2.04;NA)
Interaction P = 0.09
JS001,BM- (N=204)
mOS: 22.87, 95% CI: (21.88;NA)
JS001,BM+ (N=60)
mOS: NA, 95% CI: (15.93;NA)
Placebo,BM- (N=108)
mOS: 21.22, 95% CI: (15.9;NA)
Placebo,BM+ (N=22)
mOS: 12.16, 95% CI: (7.98;14.13)
Interaction P = 0.23
JS001,BM- (N=102)
mOS: NA, 95% CI: NA
JS001,BM+ (N=33)
mOS: NA, 95% CI: NA
Placebo,BM- (N=49)
mOS: 23.2, 95% CI: (14.75;NA)
Placebo,BM+ (N=17)
mOS: 10.61, 95% CI: (5.22;13.27)
Interaction P = 0.41
JS001,BM- (N=102)
mOS: 21.03, 95% CI: (15.31;22.87)
JS001,BM+ (N=27)
mOS: 21.55, 95% CI: (12.65;NA)
Placebo,BM- (N=59)
mOS: 18.07, 95% CI: (14.82;NA)
Placebo,BM+ (N=5)
mOS: NA, 95% CI: (2.04;NA)
Interaction P = 0.45
BM+ IL-7 signaling pathway has gene mutations
BM- IL-7 signaling pathway has no gene

FIG. 12

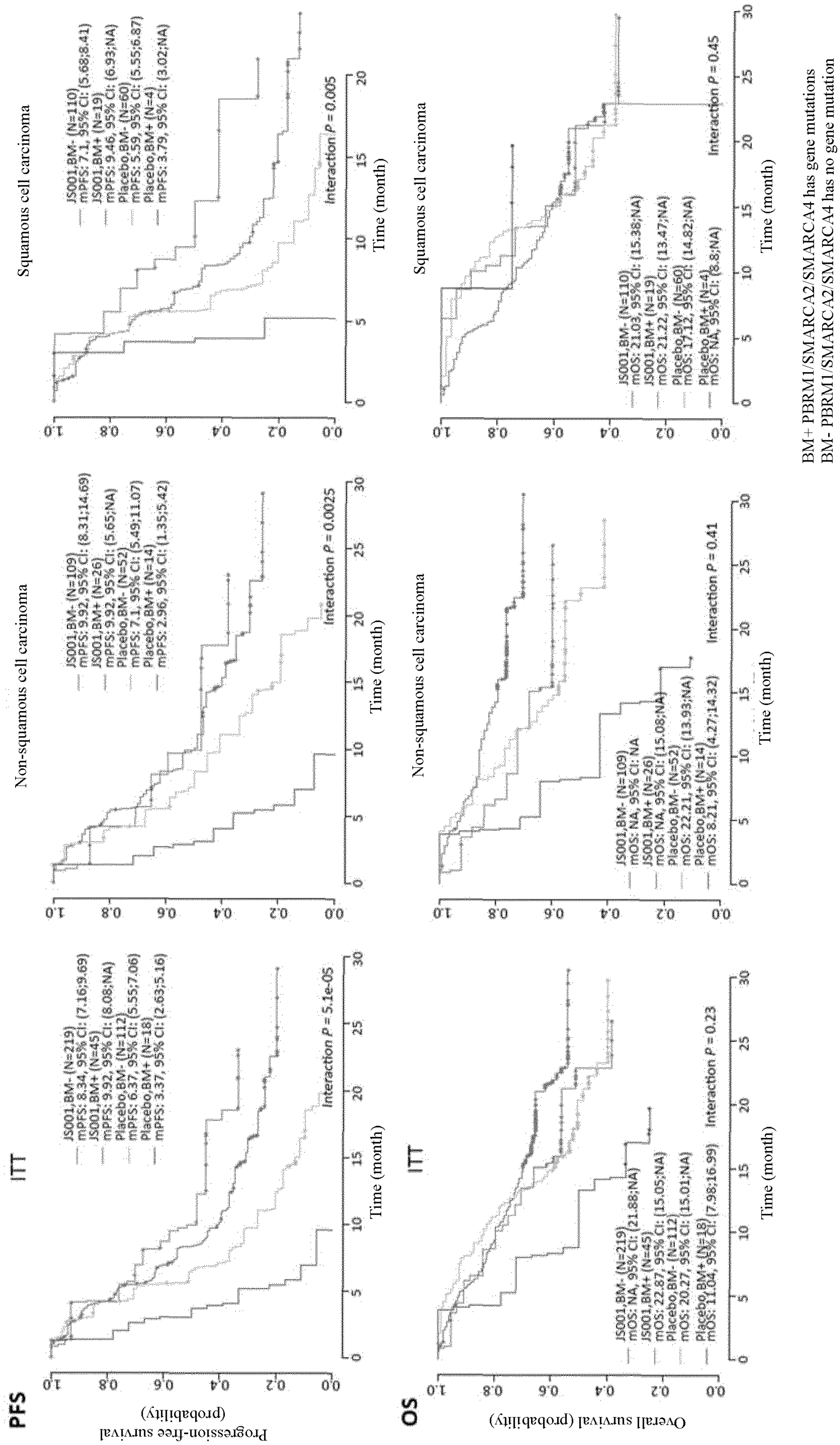
PFS
OS
ITT
Non-squamous cell carcinoma
Squamous cell carcinoma
Progression-free survival (probability)
Overall survival (probability)
Time (month)
JS001,BM- (N=219)
mPFS: 8.34, 95% CI: (7.16;9.69)
JS001,BM+ (N=45)
mPFS: 9.92, 95% CI: (8.08;NA)
Placebo,BM- (N=112)
mPFS: 6.37, 95% CI: (5.55;7.06)
Placebo,BM+ (N=18)
mPFS: 3.37, 95% CI: (2.63;5.16)
Interaction P = 5.1e-05
JS001,BM- (N=109)
mPFS: 9.92, 95% CI: (8.31;14.69)
JS001,BM+ (N=26)
mPFS: 9.92, 95% CI: (5.65;NA)
Placebo,BM- (N=52)
mPFS: 7.1, 95% CI: (5.49;11.07)
Placebo,BM+ (N=14)
mPFS: 2.96, 95% CI: (1.35;5.42)
Interaction P = 0.0025
JS001,BM- (N=110)
mPFS: 7.1, 95% CI: (5.68;8.41)
JS001,BM+ (N=19)
mPFS: 9.46, 95% CI: (6.93;NA)
Placebo,BM- (N=60)
mPFS: 5.59, 95% CI: (5.55;6.87)
Placebo,BM+ (N=4)
mPFS: 3.79, 95% CI: (3.02;NA)
Interaction P = 0.005
JS001,BM- (N=219)
mOS: NA, 95% CI: (21.88;NA)
JS001,BM+ (N=45)
mOS: 22.87, 95% CI: (15.05;NA)
Placebo,BM- (N=112)
mOS: 20.27, 95% CI: (15.01;NA)
Placebo,BM+ (N=18)
mOS: 11.04, 95% CI: (7.98;16.99)
Interaction P = 0.23
JS001,BM- (N=109)
mOS: NA, 95% CI: NA
JS001,BM+ (N=26)
mOS: NA, 95% CI: (15.08;NA)
Placebo,BM- (N=52)
mOS: 22.21, 95% CI: (13.93;NA)
Placebo,BM+ (N=14)
mOS: 8.21, 95% CI: (4.27;14.32)
Interaction P = 0.41
JS001,BM- (N=110)
mOS: 21.03, 95% CI: (15.38;NA)
JS001,BM+ (N=19)
mOS: 21.22, 95% CI: (13.47;NA)
Placebo,BM- (N=60)
mOS: 17.12, 95% CI: (14.82;NA)
Placebo,BM+ (N=4)
mOS: NA, 95% CI: (8.8;NA)
Interaction P = 0.45
BM+ PBRM1/SMARCA2/SMARCA4 has gene mutations
BM- PBRM1/SMARCA2/SMARCA4 has no gene mutation

FIG. 13

## INTERNATIONAL SEARCH REPORT

International application No.
**PCT/CN2022/088257**

**A. CLASSIFICATION OF SUBJECT MATTER**

A61K 39/395(2006.01)i； A61K 31/00(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN; CNKI; 百度学术; PUBMED; WEB OF SCIENCE; 中国专利生物序列检索系统, China Patents Biological Sequence Search System; GenBank: 抗PD-1抗体, 抗原结合片段, 化疗, 组合, 联合, 联用, 治疗, 非小细胞肺癌, 药物, 药盒, 晚期非小细胞肺癌, anti-PD-1 antibody, anti-programmed death 1 monocloncal antibody , antigen binding fragment, chemotherapy, first-line therapy, combination, NSCLC, advanced non-small-cell lung cancer, 基于SEQ ID NOs: 1-10的序列检索, sequence search based on SEQ ID NOs: 1-10

**C. DOCUMENTS CONSIDERED TO BE RELEVANT**

| Category* | Citation of document, with indication, where appropriate, of the relevant passages | Relevant to claim No. |
|---|---|---|
| X | CN 109806393 A (JIANGSU HENGRUI MEDICINE CO., LTD. et al.) 28 May 2019 (2019-05-28)<br>see claims 1-16, abstract, and description, p. 4 | 1, 2, 8-10 (in part), 17, 19 (in part) |
| Y | CN 110882385 A (SHANGHAI JUNSHI BIOSCIENCES CO., LTD. et al.) 17 March 2020 (2020-03-17)<br>see claims 1-11, and a sequence table | 5-7 (in part), 18, 20 |
| Y | CN 104250302 A (SHANGHAI JUNSHI BIOSCIENCES CO., LTD. et al.) 31 December 2014 (2014-12-31)<br>see claims 1-15, and a sequence table | 5-7 (in part), 18, 20 |
| A | WO 2016141218 A1 (MERCK SHARP & DOHME et al.) 09 September 2016 (2016-09-09)<br>see abstract, and claim 1 | 1, 2, 5-10 (in part), 17, 18, 19 (in part), 20 |
| A | CN 109893654 A (JIANGSU HENGRUI MEDICINE CO., LTD. et al.) 18 June 2019 (2019-06-18)<br>see abstract, and claims 1 and 2 | 1, 2, 5-10 (in part), 17, 18, 19 (in part), 20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2022** | **26 July 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/088257**

**C. DOCUMENTS CONSIDERED TO BE RELEVANT**

| Category* | Citation of document, with indication, where appropriate, of the relevant passages | Relevant to claim No. |
|---|---|---|
| A | CN 111617078 A (SICHUAN KELUN-BIOTECH BIOPHARMACEUTICA CO., LTD.) 04 September 2020 (2020-09-04)<br>see abstract, and claim 1 | 1, 2, 5-10 (in part), 17, 18, 19 (in part), 20 |
| A | CN 112300280 A (CTTQ AKESO SHANGHAI BIOMED TECHNOLOGY CO., LTD.) 02 February 2021 (2021-02-02)<br>see abstract | 1, 2, 5-10 (in part), 17, 18, 19 (in part), 20 |
| X | GADGEEL, S. M. et al. "Pembrolizumab and Platinum-Based Chemotherapy as First-Line Therapy for Advanced Non-Small-Cell Lung Cancer:Phase1 Cohorts From the KEYNOTE-021 Study"<br>*Lung Cancer,* Vol. 125, 30 November 2018 (2018-11-30),<br>pp. 273-281, see abstract | 1, 2, 8-10 (in part), 17, 19 (in part) |
| Y | CN 109806393 A (JIANGSU HENGRUI MEDICINE CO., LTD. et al.) 28 May 2019 (2019-05-28)<br>see claims 1-16, abstract, and description, p. 4 | 5-7 (in part), 18, 20 |
| Y | GADGEEL, S. M. et al. "Pembrolizumab and Platinum-Based Chemotherapy as First-Line Therapy for Advanced Non-Small-Cell Lung Cancer:Phase1 Cohorts From the KEYNOTE-021 Study"<br>*Lung Cancer,* Vol. 125, 30 November 2018 (2018-11-30),<br>pp. 273-281, see abstract | 5-7 (in part), 18, 20 |

Form PCT/ISA/210 (second sheet) (January 2015)

**INTERNATIONAL SEARCH REPORT**

International application No.
**PCT/CN2022/088257**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT

International application No.
PCT/CN2022/088257

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

[1] The subject matter of claim 19 is a method for preventing or treating non-small-cell lung cancer (NSCLC), and belongs to the subject matter as defined in PCT Rule 39.1(iv) for which no search is required. However, the examiner still conducted a search on the basis of an application of the pharmaceutical combination according to claim 17 or 18 in the preparation of a drug for preventing or treating NSCLC.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Claims 1-20 comprise the following six inventions:

[2] invention 1: claims 1, 2, 5-10 (in part), 17-18, 19 (in part), and 20, relating to a use of a combination of an anti-PD-1 antibody or an antigen-binding fragment thereof and a first-line chemotherapeutic drug in the preparation of a drug or kit for treating NSCLC, the NSCLC being advanced NSCLC, a pharmaceutical combination comprising an anti-PD-1 antibody or an antigen-binding fragment thereof, nab-pacilitaxel, and carboplatin, or a pharmaceutical combination comprising an anti-PD-1 antibody or an antigen-binding fragment thereof, pemetrexed, and cisplatin or carboplatin, a kit comprising said pharmaceutical combination, and a method for treating NSCLC by administering the pharmaceutical combination;

[3] invention 2: claims 1-3 (in part), 5-10 (in part), and 11-16 (in part), relating to a use of a combination of an anti-PD-1 antibody or an antigen-binding fragment thereof and a first-line chemotherapeutic drug in the preparation of a drug or kit for treating NSCLC, the NSCLC being squamous cell carcinoma;

[4] invention 3: claims 1-3 (in part), 5-10 (in part), and 11-16 (in part), relating to a use of a combination of an anti-PD-1 antibody or an antigen-binding fragment thereof and a first-line chemotherapeutic drug in the preparation of a drug or kit for treating NSCLC, the NSCLC being non-squamous cell carcinoma;

[5] invention 4: claims 1-2, 4 (in part), and 5-10 (in part), relating to a use of a combination of an anti-PD-1 antibody or an antigen-binding fragment thereof and a first-line chemotherapeutic drug in the preparation of a drug or kit for treating NSCLC, the NSCLC being NSCLC having PD-L1 expression greater than or equal to 1% in immunohistochemical staining analysis of a tumor tissue section;

[6] invention 5: claims 1-2, 4 (in part), and 5-10 (in part), relating to a use of a combination of an anti-PD-1 antibody or an antigen-binding fragment thereof and a first-line chemotherapeutic drug in the preparation of a drug or kit for treating NSCLC, the NSCLC being NSCLC having PD-L1 expression less than 1% in immunohistochemical staining analysis of a tumor tissue section; and

[7] invention 6: claim 19 (in part), relating to a method for treating NSCLC by administering the anti-PD-1 antibody or the antigen binding fragment thereof according to any one of claims 5-8. The same or corresponding technical feature among the described five inventions is treating NSCLC by using a combination of an anti-PD-1 antibody and a first-line chemotherapeutic drug. The same or corresponding technical feature between invention 6 and inventions 1-5 is treating NSCLC by using an anti-PD-1 antibody.

[8] However, the described same or corresponding technical features were disclosed in the prior art, for example, see GADGEEL S M, STEVENSON J P, LANGER C J, et al. Pembrolizumab and Platinum-Based Chemotherapy as First-Line Therapy for Advanced Non-Small-Cell Lung Cancer: Phase1 Cohorts From

INTERNATIONAL SEARCH REPORT

International application No.
**PCT/CN2022/088257**

**Box No. III Observations where unity of invention is lacking (Continuation of item 3 of first sheet)**

the KEYNOTE-021 Study[J]. Lung Cancer, 2018, 125:273-281. DOI:10.1016/j. lungcan. 19 August 2018. Therefore, the corresponding technical features among the described six inventions do not constitute special technical features. The described six inventions do not share a same or corresponding special technical feature, and thus do not comply with the requirement of unity of invention as defined in PCT Rule 13.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1, 2, 5-10 (in part), 17, 18, 19 (in part), and 20**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2022/088257**

| Patent document cited in search report | | | Publication date (day/month/year) | Patent family member(s) | | | Publication date (day/month/year) |
|---|---|---|---|---|---|---|---|
| CN | 109806393 | A | 28 May 2019 | None | | | |
| CN | 110882385 | A | 17 March 2020 | None | | | |
| CN | 104250302 | A | 31 December 2014 | EP | 3026062 | A1 | 01 June 2016 |
| | | | | JP | 2016523265 | A | 08 August 2016 |
| | | | | PL | 3026062 | T3 | 11 January 2021 |
| | | | | US | 2019023782 | A1 | 24 January 2019 |
| | | | | SI | 3026062 | T1 | 31 December 2020 |
| | | | | DK | 3026062 | T3 | 12 October 2020 |
| | | | | US | 2016272708 | A1 | 22 September 2016 |
| | | | | US | 2021009688 | A1 | 14 January 2021 |
| | | | | PT | 3026062 | T | 13 October 2020 |
| | | | | MY | 176822 | A | 24 August 2020 |
| | | | | WO | 2014206107 | A1 | 31 December 2014 |
| | | | | PH | 12015502819 | A1 | 21 March 2016 |
| | | | | RU | 2016102176 | A | 31 July 2017 |
| | | | | HR | P20201600 | T1 | 05 March 2021 |
| | | | | HU | E052487 | T2 | 28 May 2021 |
| | | | | EP | 3845562 | A1 | 07 July 2021 |
| | | | | BR | 112015031883 | A2 | 14 February 2018 |
| | | | | ES | 2822927 | T3 | 05 May 2021 |
| WO | 2016141218 | A1 | 09 September 2016 | KR | 20170122809 | A | 06 November 2017 |
| | | | | CA | 2978226 | A1 | 09 September 2016 |
| | | | | US | 2022023285 | A1 | 27 January 2022 |
| | | | | US | 2018092901 | A1 | 05 April 2018 |
| | | | | WO | 2016140717 | A1 | 09 September 2016 |
| | | | | JP | 2021035962 | A | 04 March 2021 |
| | | | | JP | 2018512391 | A | 17 May 2018 |
| | | | | AU | 2015384801 | A1 | 07 September 2017 |
| CN | 109893654 | A | 18 June 2019 | None | | | |
| CN | 111617078 | A | 04 September 2020 | None | | | |
| CN | 112300280 | A | 02 February 2021 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014206107 A


### Non-patent literature cited in the description


- **KABAT et al.** Sequences of Proteins of Immunological Interest. National Institutes of Health, 1991
- **KABAT.** *Adv. Prot. Chem.,* 1978, vol. 32, 1-75
- **KABAT et al.** *J. Biol. Chem.,* 1977, vol. 252, 6609-6616
- **CHOTHIA et al.** *J Mol. Biol.,* 1987, vol. 196, 901-917
- **CHOTHIA et al.** *Nature,* 1989, vol. 341, 878-883
- **EISENHAUVER E.A. et al.** *Eur. J Cancer,* 2009, vol. 45, 228-247
- **W. A. WEBER.** *J. Nucl. Med.,* 2009, vol. 50, 1S-10S
- *WHO Drug Information,* 2018, vol. 32, 372-373
- **THOMPSON, R. H. et al.** *PNAS,* 2004, vol. 101 (49), 17174-17179
- **TAUBE, J. M. et al.** *Sci Transl Med,* 2012, vol. 4, 127-37
- **TOPALIAN, S. L. et al.** *New Eng. J. Med.,* 2012, vol. 366 (26), 2443-2454